# EUROPEAN PATENT APPLICATION

(11) **EP 4 241 927 A1**
(43) Date of publication of application: **13.09.2023**
(21) Application number: 21889219.8
(22) Date of filing: 04.11.2021
(51) Int. Cl.: B25J 1/02, B25J 18/06, A61B 34/30

(54) **LONG MOVABLE STRUCTURE BODY AND WIRING ASSISTANCE TOOL**

(30) Priority: 05.11.2020 US 202063109986 P; 27.08.2021 JP 2021138738
(71) Applicant: National University Corporation Shiga University of Medical Science, Otsu-shi Shiga 520-2192 (JP)
(72) Inventor: YAMADA, Atsushi, Otsu-shi, Shiga 520-2192 (JP); TANI, Tohru, Otsu-shi, Shiga 520-2192 (JP)
(74) Representative: Gunzelmann, Rainer
(86) International application number: PCT/JP2021/040517
(87) International publication number: WO 2022/097666

(57) **Abstract**

[Problem] An object of the present invention is to provide a movable elongated structural body, a wiring aid used in the movable elongated structural body, a movable elongated structural instrument, and the like.

[Solution] A spacer 40 is provided with a cylindrical cylinder 42, a base end side flange 43b, and a distal end side flange 43a, wherein at the base end side flange 43b, there are provided a proximity regulation part 45b that regulates proximity in a circumferential direction of a pair of traction wires 50 led out from a pair of base end side wire lumens 32 provided in a base end side flexible tube 30 and introduced into a pair of distal end side wire lumens 22 provided in a distal end side flexible tube 20, and an arrangement recess 44 that is open on a radially outer side and arranged by the traction wires 50, and wherein at a distal end side flange 43a, there are provided a separation regulation part 45a that regulates the circumferential separation of the traction wires 50, and a placement recess 44.

## Description

### TECHNICAL FIELD

The present invention is applicable to, for example, a movable elongated structure that is inserted into a duct such as a hollow organ, a vessel, or a blood vessel, and a distal end of which is bent in a predetermined direction at a bifurcation. The present invention relates to wiring aids, movable elongated structural instruments, medical systems, tools, manipulators, robots, medical robots, insertion methods, robot operating methods, and movable elongated structure operating methods.

### BACKGROUND OF THE INVENTION

For example, elongated medical devices such as catheters and endoscopes that are inserted into body cavities are frequently used. Such an elongated medical device, for example, like the medical manipulator described in Patent Literature 1, is inserted into a bifurcation by bending the distal end.

The endoscope provided in the manipulator of Patent Document 1 is configured such that the bending portion provided in the middle portion of the distal end side bends in four directions of up, down, left, and right, so that the direction of the distal end may be changed up, down, left, and right. However, its structure is complicated, and its assembling property is low.

### PRIOR ART DOCUMENTS

[Patent Document 1] Japanese Laid-open Publication Hei 8-224247

### SUMMARY OF THE INVENTION

### [Problems to be solved by the invention]

Therefore, it is an object of the present invention to provide a movable elongated structure with high assembly properties, a wiring aid used for the movable elongated structure, a movable elongated structural instrument, a medical system, a tool, a manipulator, a robot, a medical robot, an insertion method, a robot operation method, and a movable elongated structure operation method.

### [Means for solving the problem]

The present invention provides a movable elongated structure including a distal end side tubular body, a base end side tubular body, and a traction operation body, and a wiring aid removably held by the traction operation body between the distal end side tubular body and the base end side tubular body.

Further, the present invention provides a wiring aid removably held between a distal end side tubular body and a base end side tubular body of a movable elongated structure having the distal end side tubular body, the base end side tubular body, and a traction operation body.

As an aspect of the present invention, there may be provided at least a cylindrical body arranged along the longitudinal direction between the flexible elongated distal end side tubular body and base end side tubular body arranged in series from the distal end side to the base end side along the longitudinal direction, a base end side flange protruding radially outward provided with an operation body arrangement part at the base end side end of the cylindrical body in which the interval in a circumferential direction is set wider than the interval in the circumferential direction between the pair of distal end side through holes provided inside the tube wall of the distal end side tubular body and penetrating in the longitudinal direction and a pair of traction operation bodies led out from a pair of base end side through holes provided inside the tube wall of the base end side tubular body and introduced into the distal end side through hole are arranged, and a distal end side flange projecting radially outward from the distal end of the cylindrical body provided with a placement part arranged by the traction body which is led out from the base end side through hole and introduced into the distal end side through hole.

The traction operation body may be string-shaped or band-shaped. Further, the pair of traction operation bodies may be separate traction operation bodies, or one traction operation body may be bent back to form a pair. Further, when pulling the pair of traction operation bodies, both of the traction operation bodies may be pulled, or one of the traction operation bodies may be pulled.

The cylindrical body may be a tubular body having a circular cross-section, an elliptical cross-section, or a polygonal cross-section, and the internal space may have a similar shape to the outer diameter of the cross-section, or may have a different cross-sectional shape. Moreover, the internal space in the cylindrical body may be arranged at the center of the outer shape, or may be arranged off the center of the outer shape.

The separation regulation part may be provided for each of the traction operation bodies, or may be integrally provided.

The above-mentioned interval in the circumferential direction refers to an interval on the shorter side of the interval in the circumferential direction between the pair of through holes.

Therefore, the interval in the circumferential direction is set wider than the interval in the circumferential direction between the pair of distal end side through holes provided inside the tube wall of the distal end side tubular body and penetrating in the longitudinal direction, and the pair of base end side through holes provided in the tube wall of the base end side tubular body and penetrating in the longitudinal direction means that the interval on the short side of the interval in the circumferential direction between the base end side through holes is wider than the interval on the short side of the interval in the circumferential direction between the distal end side through holes.

As another aspect of the present invention, the distal end side interval, which is the interval between the distal end side imaginary line and the distal end side center line parallel to the distal end side virtual line passing through the distal end side virtual line connecting the centers of the pair of distal end side through holes and the center of the distal end side tubular body may be set wider than the base end side interval between a base end side imaginary line connecting the centers of the pair of base end side through holes and a base end side central line passing through the center of the base end side tubular body and parallel to the base end side imaginary line.

As another aspect of the present invention, there is provided a movable elongated structure,
wherein a distal end side tubular body and a base end side tubular body are formed in an elongated shape having flexibility;
wherein a pair of flexible traction operation bodies that are inserted through the pair of longitudinal through holes provided inside the tube walls of the distal end side tubular body and the base end side tubular body are provided;
wherein a wiring aid that is disposed between the distal end side tubular body and the base end side tubular body and regulates the direction of the traction operation body is provided;
wherein the through hole provided in the base end side tubular body is defined as a base end side through hole, and the through hole provided in the distal end side tubular body is defined as a distal end side through hole;
wherein the interval between the pair of base end side through holes in the circumferential direction is set wider than the interval between the pair of distal end side through holes in the circumferential direction;
wherein the wiring aid includes a tubular body arranged along the longitudinal direction, and a base end side flange at an end of the base end side of the tubular body and protruding radially outward, and a distal end side flange provided at an end of the distal end side of the cylindrical body and protruding radially outward;
wherein a proximity regulation (or restriction) part (or portion) that regulates proximity of the traction operation body in the circumferential direction which is led out from the base end side through hole and introduced into the distal end side through hole and an operation body arrangement part that is open radially outward and in which the traction operating body is placed are provided at the base end side flange; and
wherein a separation regulation (or restriction) part (or portion) that regulates a separation of the traction operation body in the circumferential direction which is led out from the base end side through hole and introduced into the distal end side through hole and an operation body arrangement part that is open radially outward and in which the traction operating body is placed are provided at the distal end side flange.

In addition, the proximity regulation part may be provided for each of the traction operation bodies, or may be provided integrally.

Further, according to the present invention, there is provided movable elongated structural instrument including the above-mentioned movable elongated structure and a traction drive unit for pulling the pair of traction operation bodies, wherein the traction drive unit pulls the pair of traction operation bodies to bend and deform the distal end side tubular body.

Further, according to the present invention, there is provided a movable elongated structural instrument including the above-mentioned movable elongated structure and a traction drive unit for pulling the pair of traction operation bodies, wherein a plurality of the traction drive units are provided and prescribed one of the traction drive units pulls a pair of the traction operation bodies to bend and deform the distal end side tubular body in a desired direction.

According to another aspect of the present invention, there is provided a medical system including the movable elongated structural instrument described above, a drive unit for driving the traction drive unit, and a control unit connected to apply a drive signal to the drive unit.

The present invention also provides a medical system including the movable elongated structural instrument as described above, a drive unit for selectively driving at least one of the plurality of traction drive units, and a control connected to apply a drive signal to the drive unit.

In addition, the present invention provides a tool including the above-described movable elongated structure instrument, a mounting portion mounting the base end side tubular body in the movable elongated structure to a distal end of a robot arm, and a connection part connected a drive mechanism for driving the traction drive units on the robot arm side.

The present invention also provides a robot including the tool, a robot arm having the tool at its distal end, a drive unit for driving the traction drive units and the robot arm, and a controller connected to apply a drive signal to the drive unit.

Further, the present invention provides a manipulator including the movable elongated structural instrument described above, a body portion provided at a base end of the base end side tubular body in the movable elongated structure, and an operation unit operating the traction drive part in the main body part.

Further, the present invention provides a robot including an input/output unit wired and/or wirelessly connected to the above-described movable elongated structural instrument, an input unit that receives operation signals in real time, an arithmetic unit that executes a predetermined operation program based on the operation signal, and an output unit that generates a driving signal for pulling a predetermined traction operation body by the traction drive unit and bending and/or expanding and contracting (extending) at least the distal end side tubular body in a desired direction based on an output from the arithmetic unit.

Further, the present invention provides a medical robot including the robot described above, wherein the output unit provides a drive signal to an externally provided drive unit that mechanically drives the movable elongated structure.

Further, the present invention provides an insertion method including the steeps of inserting the above-described movable elongated structure into a duct, driving and controlling the traction drive unit that pulls the pair of traction operating bodies to bend and deform the distal end side tubular body, and inserting the distal end side tubular body into a branched duct.

Further, the present invention provides an operation method of the robot equipped with the above-described movable long structural instrument, including the steps of receiving an operation signal in real time by the input/output unit wired and/or wirelessly connected to the robot, executing a predetermined operation program based on the received operation signal by the arithmetic unit, and then, pulling the traction operation body by the traction drive unit based on the output from the arithmetic unit to bend-deform and/or expand-contract (extension) deform the distal end side tubular body in a desired direction.

When an operation program predetermined based on is executed, the traction drive unit pulls the traction operation body based on the output from the arithmetic unit to bend and/or stretch the distal tubular body in a desired direction (it is characterized by being a method of operating a robot that deforms (extension).

According to these inventions, as described above, by bending at least the distal end portion of the movable elongated structure toward the branched duct, the distal end portion may be easily inserted into the branched duct.

### ADVANTAGES OF THE INVENTION

Thus, this invention may provide a movable elongated structure with high ease of assembly, a wiring aid used for the movable elongated structure, a movable elongated structural instrument, a medical system, a tool, a manipulator, a robot, a medical robot, an insertion method, an operation method of a robot and an operation method of the moveable elongated structures, since the wiring aid is removably held between the distal end side tubular body and the base end side tubular body in the movable elongated structure having the distal end side tubular body, the base end side tubular body, and the traction operation body.

### BRIEF DESCRIPTION OF THE DRAWINGS

FIGS. 1A and 1B are explanatory diagrams of a movable elongated structure provided with a wiring aid that may be removed and held as an embodiment of the present invention; FIG. 1A is a perspective view of a movable elongated structure, and FIG. 1B is a perspective view of the movable elongated structure showing a distal end side flexible tube and a base end side flexible tube in a see-through state;
FIGS. 2A and 2B are explanatory diagrams of a movable elongated structure; FIG. 2A is a front view of the movable elongated structure, and FIG. 2B is a cross-sectional view taken along line A-A in FIG. 2A;
FIGS. 3A to 3C are explanatory diagrams of a movable elongated structure; FIG. 3A is a cross-sectional view taken along line B-B in FIG. 2A, FIG. 3B is a cross-sectional view taken along arrow C-C in FIG. 2A, and FIG. 3C is a front view of the movable elongated structure with the spacer removed;
FIGS. 4A to 4D are explanatory diagrams of a spacer; FIG. 4A is a perspective view showing the front, right side and top of the spacer, FIG. 4B is a perspective view showing the front, left side and bottom of the spacer, FIG. 4C is a front view of the spacer, and FIG. 4D is a sectional view taken along an arrow D-D in FIG. 4C;
FIGS. 5A to 5D are explanatory diagrams of a movable elongated structure; taken along taken along taken along FIG. 5A is an exploded perspective view seen from the base end side of the movable elongated structure, FIG. 5B is an exploded perspective view seen from the distal end side of the movable elongated structure, FIG. 5C is an enlarged view of part "a" in FIG. 5A, and FIG. 5D is an enlarged view of part "b" in FIG. 5B.
FIG. 6 is a perspective view of the movable elongated structure with the distal end side flexible tube bent;
FIGS. 7A and 7B are explanatory views of the movable elongated structure with the distal end flexible tube bent; FIG. 7A is a cross-sectional view corresponding to the cross -sectional view of the movable elongated structure in a state where the distal end side flexible tube is bent, taken along line B-B in FIG. 2A, and FIG. 7B is a sectional view corresponding to a cross-sectional view along the line C-C of FIG. 2A;
FIGS. 8A and 8B are explanatory diagrams of a movable elongated structure with a distal end flexible tube obliquely bent; FIG. 8A is a cross-sectional view corresponding to the cross-sectional view taken along the line B-B of FIG. 2A in the movable elongated structure with the distal end flexible tube obliquely bent, and FIG. 8B is a cross-sectional view corresponding to the cross-sectional view taken along line C-C of FIG. 2A;
FIGS. 9A and 9B are image diagrams of clinical use of the movable elongated structure;

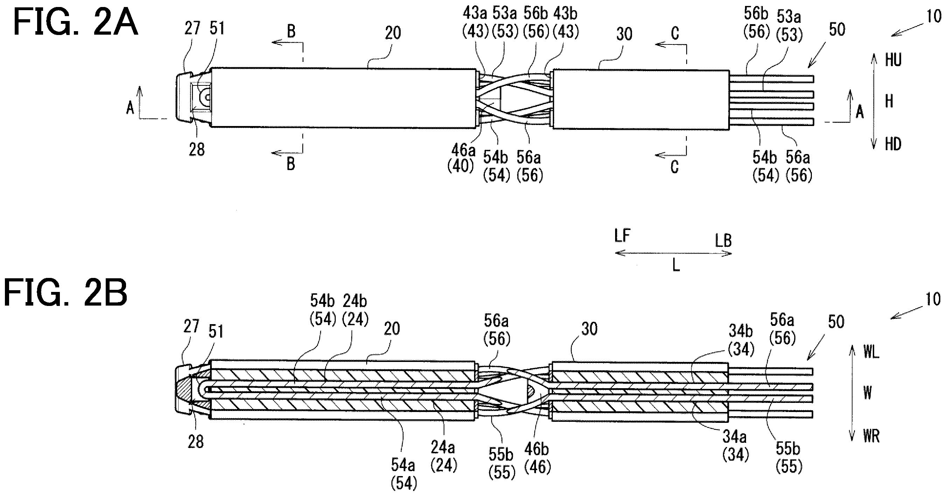

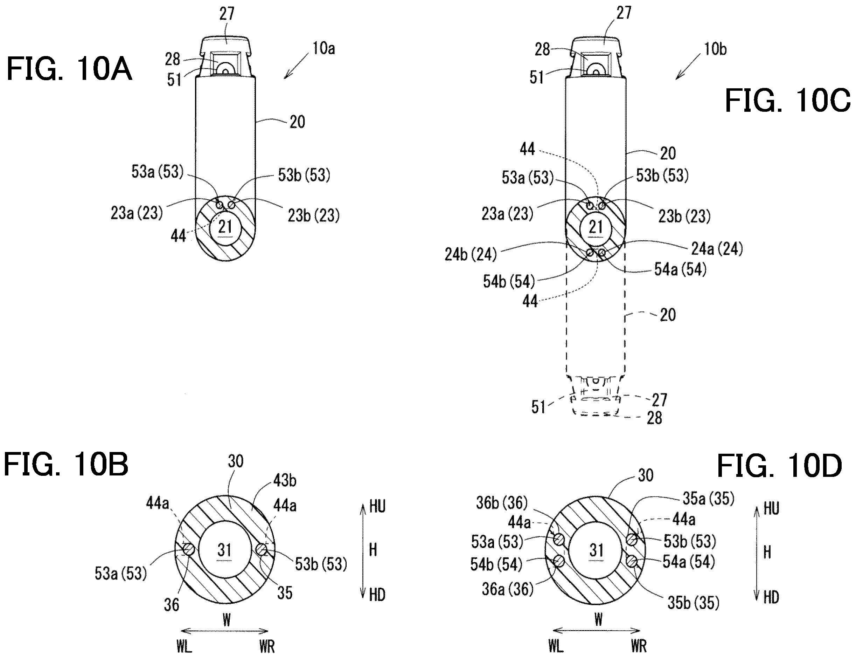

FIGS. 10A to 10D are explanatory diagrams of another example of a movable elongated structure in which the distal end flexible tube is bent; FIG. 10A is a cross-sectional view corresponding to the cross-sectional view of another example of the movable elongated structure taken along line B-B in FIG. 2A, and FIG. 10B is a cross-sectional view of the movable elongated structure taken along arrows C-C of FIG. 2A. FIG. 10C is a cross-sectional view corresponding to the cross-sectional view of another example of the movable elongated structure taken along line B-B in FIG. 2A, and FIG. 10D is a sectional view of the movable elongated structure corresponding to C-C arrow sectional drawing of FIG. 2A;
FIGS. 11A and 11B are explanatory diagrams of a movable elongated structure with another arrangement pattern; FIG. 11A is a perspective view of a movable elongated structure with another arrangement pattern, and FIG. 11B is a perspective view of the movable elongated structure showing a distal end side flexible tube and a base end side flexible tube in a see-through state;
FIGS. 12A and 12B are explanatory diagrams of a movable elongated structure with another arrangement pattern; FIG. 12A is a front view of the movable elongated structure, and FIG. 12B is a cross-sectional view taken along line A-A in FIG. 12A;
FIGS. 13A to 13C are explanatory diagrams of another example of a spacer; FIG. 13A is a front, right side, and top perspective view of another example of a spacer; FIG. 13B is a front, left side, and top perspective view of the spacer; and FIG. 13C is a plan view of the spacer;
FIGS. 14A to 14C are illustrations of another example of a movable elongated structure; FIG. 14A is a front view of the movable elongated structure, FIG. 14B is a cross-sectional view taken along line E-E in FIG. 15A, and FIG. 14C is a rear view of the movable elongated structure;
FIGS. 15A to 15C are illustrations of another example of a movable elongated structure; FIG. 15A is a cross-sectional view along line F-F in FIG. 14A, FIG. 15B is a cross-sectional view along line G-G in FIG. 14A, and FIG. 15C is a cross-sectional view along line H-H in FIG. 14A;
FIGS. 16A to 16D are explanatory diagrams of another example of a spacer; FIG. 16A is a front, right side, and top perspective view of the spacer, FIG. 16B is a back, right side, and top perspective view of the spacer, FIG. 16C is a front view of the spacer, and FIG. 16D is a top view of the spacer;
FIGS. 17A to 17D are explanatory diagrams of another example of a spacer; FIG. 17A is a front, right side, and top perspective view of the spacer; FIG. 17B is a rear, right side, and top perspective view of the spacer; FIG. 17C is a front view of the spacer; and FIG. 17D is a side view of the spacer;
FIGS. 18A to 18C are illustrations of still another example of a movable elongated structure and spacers; FIG. 18A is a perspective view of the movable elongated structure, FIG. 18B is a cross-sectional view taken along line I-I in FIG. 18A, and FIG. 18C is a cross-sectional view along arrow J-J in FIG. 18A.
FIGS. 19A to 19C are illustrations of still another example of a movable elongated structure and spacers; FIG. 19A is a perspective view of a spacer of yet another example, FIG. 19B is a side view of the spacer viewed from the distal end side, and FIG. 19C is a side view of the spacer viewed from the base end side;
FIGS. 20A to 20C are illustrations of still another example of a movable elongated structure; FIG. 20A is a perspective view of the movable elongated structure before assembly, FIG. 20B is a perspective view of the movable elongated structure in the assembled state, and FIG. 20C is a perspective view of the movable elongated structure with a traction wire routed;
FIGS. 21A to 21C are illustrations of still another example of a movable elongated structure; FIG. 21A is a front view of the movable elongated structure before assembly, FIG. 21B is a front view of the movable elongated structure in the assembled state, and FIG. 21C is a front view of the movable elongated structure with a traction wire routed;
FIGS. 22A and 22B are explanatory diagrams of an assembly spacer; FIG. 22A is an enlarged sectional view of the assembled spacer portion before assembly, and FIG. 22B is an enlarged sectional view of the assembled spacer portion in the assembled state;
FIGS. 23A and 23B are schematic explanatory diagrams of a retractor in another example; FIG. 23A is a perspective view of the retractor, and FIG. 23B is a perspective view of the retractor showing the traction wire in a see-through state;
FIGS. 24A to 24C are schematic explanatory diagrams of a retractor in another example; FIG. 24A is a top view of the retractor, FIG. 24B is a cross section through the upper wire lumen, FIG. 24C is a top view of the retractor with the elastic retractor in an open state;
FIG. 25 is a schematic diagram of a medical device in another example of this embodiment;
FIG. 26 is a schematic diagram of a telesurgery system in another example of this embodiment;
FIGS. 27A and 27B are schematic explanatory diagrams of a surgical device in a telesurgery system; FIG. 27A is a plan view of a surgical device that may be loaded onto a robot arm assembly of the telesurgery system; FIG. 27B is a diagram showing the internal configuration of the surgical device of FIG. 27A;
FIGS. 28A and 28B are explanatory diagrams of a telesurgery system; FIG. 28A is a block diagram showing the connection relationship with each unit, and FIG. 28B is an operation flow diagram of the telesurgery system;
FIGS. 29A to 29C are diagrams showing the results of a demonstration experiment of a movable elongated structure; FIG. 29A is a photograph of the result of the initial state, FIG. 29B is a photograph of the result of bending the movable elongated structure, and FIG. 29C is a photograph of the result in a bent state imitating a conventional two-way movable elongated structure; and
FIGS. 30A and 30B are diagrams showing the results of a demonstration experiment of a movable elongated structure comparing bending radii.

### DETAILED DESCRIPTION

Hereinafter, embodiments of the present invention will be described with reference to the drawings. In the following description, the same parts and components are given the same reference numerals. For example, the embodiments include the following disclosures.

### [Configuration 1]

A movable elongated structure (10, 300) includes a distal end side tubular body (20, 60), a base end side tubular body (30), and a traction operation body (50), and is provided with a wiring aid or spacer (40) removably held by a traction operation body (50) between the distal end side tubular body (20, 60) and the base end side tubular body (30).

As an example, the movable elongate structure (10, 300) is configured to include a flexible distal end side tubular body (20, 60) having a through hole, a flexible base end side tubular body (30) having a through hole, and a traction operation body (50), wherein there is provided a tubular body (42) between the distal end side tubular body (20, 60), wherein there are provided annular flanges (43) protruding radially outward from both ends of the tubular body (42), and an operation body arrangement part (44) having a recess or cutout shape in the annular flanges (43) for arranging the traction operation body (50), and wherein the traction operation body (50) is inserted through the through hole of the base end side tubular body (30), removably holds the wiring aid (40) by the operation body arrangement part (44), and is inserted through the through holes of the distal end side tubular body (20, 60).

If necessary, the distal end side tubular body (20) and the base end side tubular body (30) are formed in a flexible and elongated shape,
wherein there are provided a pair of flexible traction operation bodies (50) inserting through a pair of through holes along the longitudinal direction (L) provided inside tube walls of the distal end side tubular body (20) and the base end side tubular body (30), and the wiring aid (40) arranged between the distal end side tubular body (20) and the base end side tubular body (30) to regulate a direction of the traction operation body (50),
wherein through holes provided in the base end side tubular body (30) are defined as base end side through holes (32), and through holes provided in the distal end side tubular body (20) are defined as distal end side through holes (22),
wherein an interval (or distance) in a circumferential direction between the pair of base end side through holes (32) (an interval Rf between a base end side wire lumen 36b and the base end side wire lumen 35a, or an interval Rg between a base end side wire lumen 36a and a base end side wire lumen 35b) is set wider than an interval in a circumferential direction between the pair of distal end side through holes (22) (an interval Ra between a distal end side wire lumen 23a and a distal end side wire lumen 23b, or an interval Re between a distal end side wire lumen wire lumen 24a and a distal end side wire lumen wire lumen 24b),
wherein the wiring aid (40) is provided with a tubular body (42) arranged along the longitudinal direction (L), a base end side flange (43b) protruding radially outward provided at an end of a base end side (LB) of the tubular body (42), and a distal end side flange (43a) protruding radially outward provided at an end of a distal end side (LF) of the tubular body (42),
wherein on the base end side flange (43b), there are provided a proximity regulation part (45b) that regulates proximity in the circumferential direction of the traction operation body (50) led out from the base end side through holes (32) and introduced into the distal end side through holes (22) and an operation body arrangement part (44), which is open radially outward and in which a traction operation body (50) is arranged, and
wherein on the distal end side flange (43a), there are provided a separation regulation part (45a) that regulates the separation in the circumferential direction of the traction operation body (50) led out from the base end side through hole (32) and introduced into the distal end side through hole (22), and an operation body arrangement part (44) which is open radially outward and in which the traction operation body (50) is arranged.

If necessary, a distal end side interval (or distance) (X1) which is an interval between a distal end side virtual line (FVL) connecting the centers of the pair of distal end side through hole (22) and a distal end side central line (FCL) parallel to the distal end side virtual line (FVL) passing through a center of the distal end side tubular body (20) may be set wider than a base end side interval (X2) which is an interval between a base end side virtual line (BVL) connecting the centers of the pair of base end side through hole (32) and a base end side central line (BCL) parallel to the base end side virtual line (BVL) passing through a center of the base end side tubular body (30).

If necessary, a pair of base end side through holes (32) and a pair of distal end side through holes (22) through which a pair of traction operation bodies (50) are inserted may constitute a set of through holes (11), and a plurality of sets of through holes (11) may be arranged at different positions in the circumferential direction, and a plurality of proximity regulation parts (45b) and operation body arrangement parts (44) may be provided on the base end side flange (43b), and the distal end side flange (43a) may be provided with a plurality of separation regulation portions (45a) and operation body arrangement parts (44).

On the base end side flange (43b), proximity of circumferentially adjacent traction operation bodies (50) of the pair of traction operation bodies (50) inserted through the circumferentially adjacent through hole sets (11) may be regulated by the common proximity regulation part (45b), and, on the distal end side flange (43a), separation of the traction operation bodies (50) adjacent to each other in the circumferential direction may be regulated by the separation regulation part (45a) common to the traction operation bodies (50) inserted into the through hole sets (11) adjacent to each other on the opposite side in the circumferential direction.

One traction operation body (50) and the other traction operation body (50) inserted through the through hole sets (11) adjacent in the circumferential direction may cross each other in the circumferential direction, and a guide portion (46) may be provided to guide the one traction operation body (50) to the outside of the other traction operation body (50).

An intermediate tubular body (60) having an elongated shape and having intermediate through holes (63, 64, 65, 66) may be arranged between the distal end side tubular body (20) and the base end side tubular body (30), a plurality of pairs of traction operation bodies (50) may be provided, and at least one pair of traction operation bodies (50) among the plurality of pairs of traction operation bodies (50) has a distal end side through hole (22), an intermediate through hole (63, 64, 65, 66) and the base end side through hole (32), and at least one pair of the traction operation bodies (50) out of the plurality of pairs of traction operation bodies (50) may be inserted through the intermediate through holes (63, 64, 65, 66) and the base end side through hole (32).

The traction operation body (50) may be a flexible wire, and the distal end side tubular body (20) and the base end side tubular body (30) may be composed of flexible tubes having main lumens (21, 31), the through hole may be a wire lumen formed inside the tube wall of the tube and through which a wire may be inserted.

The wire may be bent back at the distal end side (LF) of the distal end side tubular body (20) to form a pair of traction operation bodies (50).

### [Configuration 2]

A wiring aid (40) removably held between a distal end side tubular body (20, 60) and the base end side tubular body (30) of a movable elongated structure (10, 300) having the distal end side tubular body (20, 60), the base end side tubular body (30), and a traction operation body (50).

As an example, the wiring aid (40) includes annular flanges (43) protruding radially outward from both ends of a tubular body (42), and the annular flanges (43) is provided with an operation body arrangement part (44) having a shape of a concave portion or a notch portion arranging the traction operation body (50).

In addition, there are provided;
at least a cylindrical tubular body (42) disposed along a longitudinal direction (L) between a flexible elongated distal end side tubular body (20) and a base end side tubular body (30) which is arranged in series from a distal end side (LF) to a base end the base end side (LB) along the longitudinal direction (L);
a base end side flange (43b) protruding radially outward on an end of the base end side (LB) of the tubular body (42), in which an interval in a circumferential direction is set wider than the interval in the circumferential direction between the pair of distal end side through holes (22) penetrating in the longitudinal direction (L) provided inside the tube wall of the distal end side tubular body (20), provided with an operation body placement portion (44) arranging a pair of traction operation bodies (50) which are drawn out from a pair of base end side through holes (32) provided inside the tube wall of the base end side tubular body (30) and penetrating in the longitudinal direction (L) and introduced into the distal end side through hole (22); and
a distal end side flange (43a) protruding radially outward on an end of the distal end side (LF) of the tubular body (42) provided with an operation body placement portion (44) arranging the pulling operation body (50), which is led out from the base end side through hole (32) and introduced into the distal end side through hole (22).

The base end side flange (43b) may be provided at the base end side (LB) end of the tubular body (42), and the distal end side flange (43a) may be provided at the end of the tubular body (42), wherein
a proximity regulation part (45b) that regulates proximity in the circumferential direction of the traction operation body (50) guided from the base end side through hole (32) and introduced into the distal side through hole (22) may be provided at the base end side flange (43b), and
the operation body placement portion (44) may be open radially outward.

A pair of base end side through holes (32) and a pair of distal end side through holes (22) through which a pair of traction operation bodies (50) are inserted constitute a set of through holes (11), and a plurality of sets of through holes (11) are arranged at different positions in the circumferential direction, and a plurality of proximity regulation parts (45b) and operation body arrangement parts (44) are provided on the base end side flange (43b), and the distal end side flange ( 43a) may be provided with a plurality of separation regulation portions (45a) and operation body arrangement parts (44).

The common proximity regulation part (45b) at a base end side flange (43b) may regulate circumferentially adjacent traction operation bodies (50) of the pair of traction operation bodies (50) inserted through the circumferentially adjacent through hole sets (11) close to each other, and the separation regulation part (45a) common to the traction operation bodies (50) inserted into the through hole sets (11) adjacent to each other on the opposite side in the circumferential direction at the distal end side flange (43a) may regulate a separation of the traction operation bodies (50) adjacent to each other in the circumferential direction of the pair of traction operation bodies (50) inserted through the through hole sets (11) adjacent in the circumferential direction.

A guide portion (46) may be provided to circumferentially intersect one traction operation body (50) and the other traction operation body (50) inserted through the through hole sets (11) adjacent in a circumferential direction, and to guide the one traction operation body (50) to the outside of the other traction operation body (50).

### [Configuration 3]

A movable elongated structural instrument (100, 217) including the movable elongated structure (10, 300) and a traction drive unit (102, 221) for pulling the pair of traction operation bodies (50) to bend and deform a distal end side tubular body (20) by pulling the pair of traction operation bodies (50).

### [Configuration 4]

A movable elongated structural instrument (100, 217) including the movable elongated structure (10, 300) described above and a traction drive unit (102, 221) for pulling the pair of traction operation bodies (50), wherein there are provided a plurality of traction drive units (102, 221), and a predetermined traction drive unit (102, 221) pulls a pair of traction operation bodies (50) to bend and/or stretch (extend) the distal end side tubular body (20) in a desired direction.

### [Configuration 5]

A medical system (200) including, the movable elongated structural instrument (100, 217) as described above, a drive unit for driving a traction drive unit (102, 221), and a control unit (104, 202) connected to apply a drive signal to the drive unit.

### [Configuration 6]

A medical system (200) including the movable elongated structural instrument (100, 217) as described above, a drive unit for selectively driving at least one of the plurality of traction drives (102, 221), and a control unit (104, 202) connected to apply a drive signal to the drive unit.

An operation part for selectively operating at least one of the plurality of traction drive units (102, 221) may be provided and connected to the control unit (104, 202).

### [Configuration 7]

A tool including the above mentioned movable elongated structural instrument (100, 217), a mounting part for mounting the base end side tubular body (30) in the movable elongated structure (10, 300) to a distal end of a robot arm (212), and a connection part (228) for connecting with a drive mechanism that drives a traction drive unit (102, 221) on a side of the robot arm (212).

### [Configuration 8]

A robot including the tool (217) described above, a robot arm (212) having the tool (217) at a distal end thereof, a drive unit for driving a traction drive unit (102, 221) and the robot arm (212), and a control unit (104, 202) connected to apply a drive signal to the drive unit.

### [Configuration 9]

A manipulator (100) provided with the movable elongated structural instrument (100) described above, and an operation part for operating the base end side tubular body (30) in the movable elongated structural body (10, 300) on a main body side and the traction drive unit (102, 221) on the main body side.

### [Configuration 10]

A robot including
an input/output unit (210a) wired and/or wirelessly connected to the movable elongated structural device (100, 217) described above;
an input unit that receives an operation signal in real time;
an arithmetic unit (CPU) that executes a predetermined operation program based on the operation signal; and
an output unit for generating a drive signal to bend and/or stretch (extend) at least the distal end side tubular body (20) in a desired direction by pulling a predetermined traction operation body (50) by a traction drive unit (102, 221) based on an output from the arithmetic unit (CPU).

### [Configuration 11]

A medical robot including the robot as described above, wherein the output unit provides a drive signal to an externally disposed drive unit for mechanically driving the movable elongated structure (10, 300).

### [Configuration 12]

An insertion method including the steps of inserting the above-described movable elongated structure (10, 300) into a duct, driving and controlling the traction drive unit (102, 221) to bend and deform the distal end side tubular body (20), and inserting the distal end side tubular body (20) into a branched duct.

The duct may be at least one of a hollow organ, a vessel, and a blood vessel.

### [Configuration 13]

An operating method for a robot having the movable elongated structural instrument (100, 217) as described above, including the steps of;
receiving an operation signal in real time by the input/output unit (210a) connected by wire and/or wireless; and
pulling the traction operation body (50) to bend and/or stretch (extend) the distal tubular body (20) in a desired direction by the traction drive unit (102, 221) based on the output from the arithmetic unit (CPU) when the arithmetic unit (CPU) executes a predetermined operation program based on the received operation signal.

### [Configuration 14]

An operation method of removing a wiring aid (40) from a movable elongated structure (10, 300) including a distal end side tubular body (20), a base end side tubular body (30) and a traction operation body (50) and provided with the wiring aid (40) removably held by the traction operation body (50) between the distal end side tubular body (20) and the base end side tubular body (30),

The pair of traction operation bodies (50) may be pulled to bend and deform the distal tubular body (20), a plurality of pairs of the pulling operation bodies (50) may be provided, and a predetermined pair of the pulling operation bodies (50) out of the plurality of pairs of the pulling operation bodies (50) may be pulled to bend deform the distal end side tubular body (20) in a desired direction.

Although the following examples are used for medical equipment as an example of the treatment tool of the present invention, the present invention provides a treatment tool that is not limited to medical equipment.

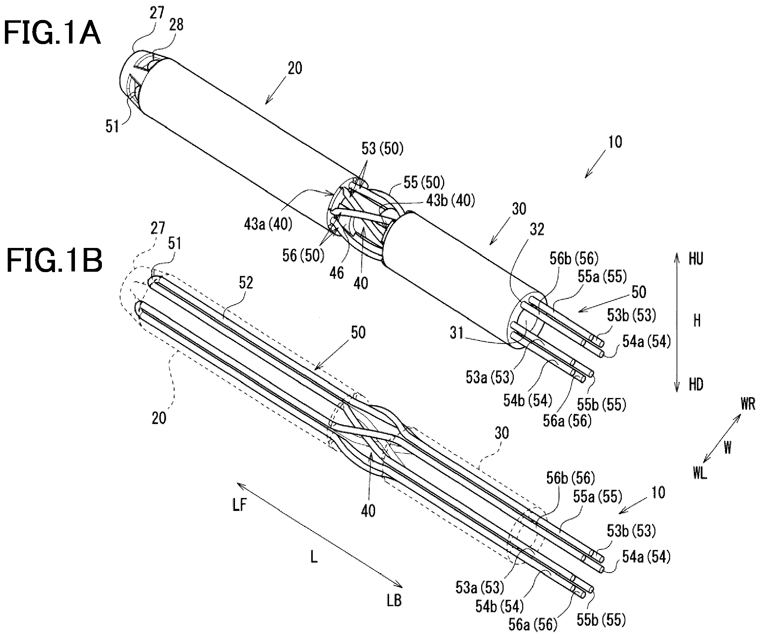

First, the drawings will be described in detail. For example, FIGS. 1A and 1B show, as an embodiment of the present invention, an explanatory view of a movable elongated structure 10 equipped with a wiring aid that may be removed and held. FIG. 1A shows a perspective view of movable elongated structure 10, and FIG. 1B shows distal end side flexible tube 20 and base end side flexible tube 30 (hereinafter flexible tubes 20, 30) in a see-through state.

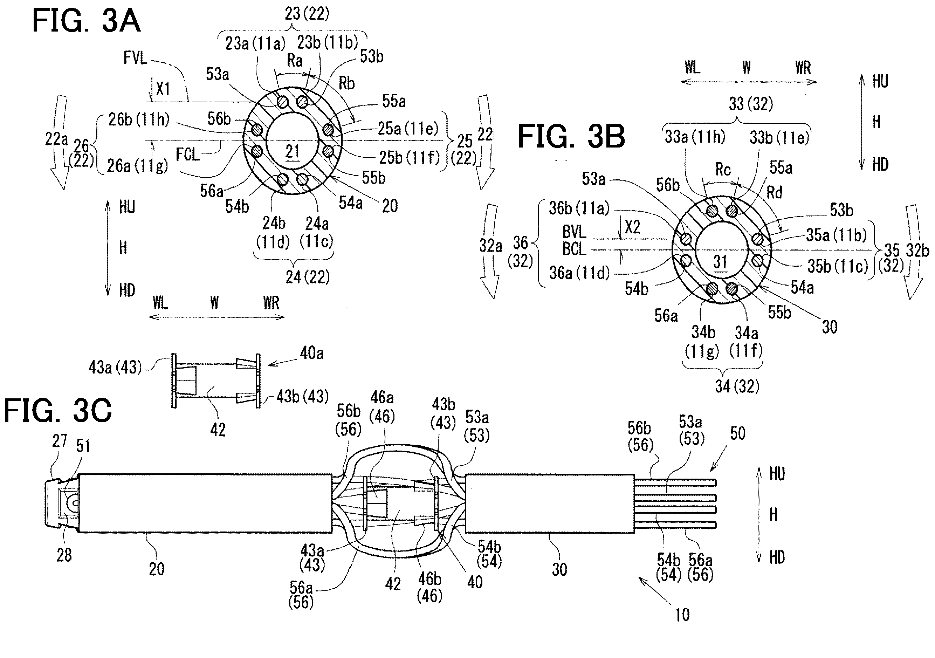

FIGS. 2 and 3 show illustrations of the movable elongated structure 10. FIG. 2A shows a front view of the movable elongated structure 10, FIG. 2B shows a cross-sectional view taken along line A-A in FIG. 2A. FIG. 3A shows a cross-sectional view taken along line B-B in FIG. 2A, FIG. 3B shows a cross-sectional view taken along line C-C in FIG. 2A, and FIG. 3C shows a front view of the movable elongated structure 10 with the spacer 40 removed.

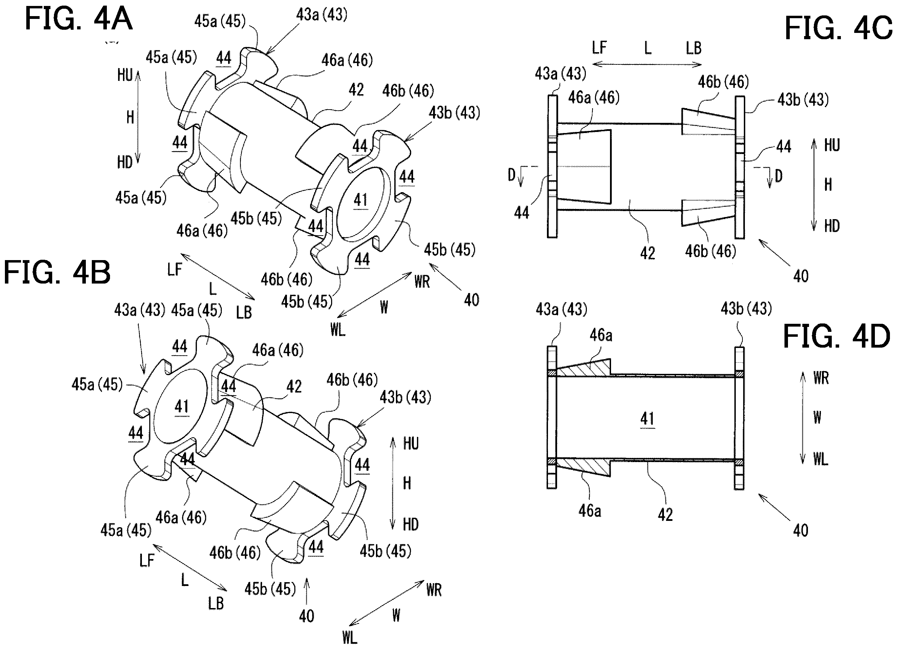

FIGS. 4A to 4D show explanatory views of a spacer 40 which is a wiring aid explained in other examples. FIG. 4A shows a front, right side and top perspective view of spacer 40, FIG. 4B shows a front, left side and bottom perspective view of spacer 40; FIG. 4C shows a front view of spacer 40, and FIG. 4D shows a cross-sectional view taken along line D-D in FIG. 4C.

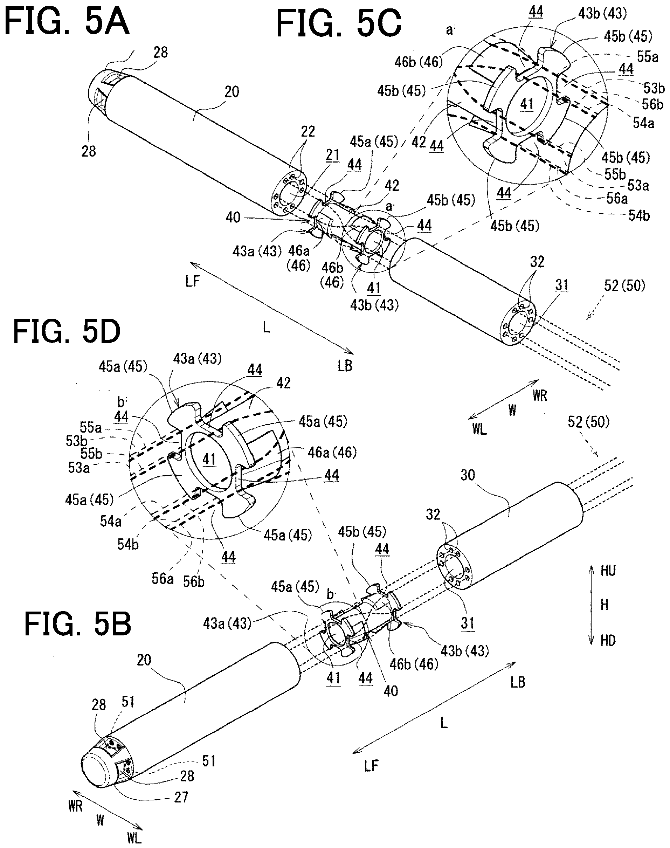

FIGS. 5A to 5D show explanatory views of the movable elongated structure 10. FIG. 5A shows an exploded perspective view of the movable elongated structure 10 viewed from the base end side LB, FIG. 5B shows an exploded perspective view of the movable elongated structure 10 viewed from the distal end side LF, FIG.5C shows an enlarged view of part "a" in FIG. 5A, and FIG. 5D shows an enlarged view of part "b" in FIG. 5B.

It should be noted that the traction wires 50 are illustrated with dashed lines in FIGS. 5A to 5D.

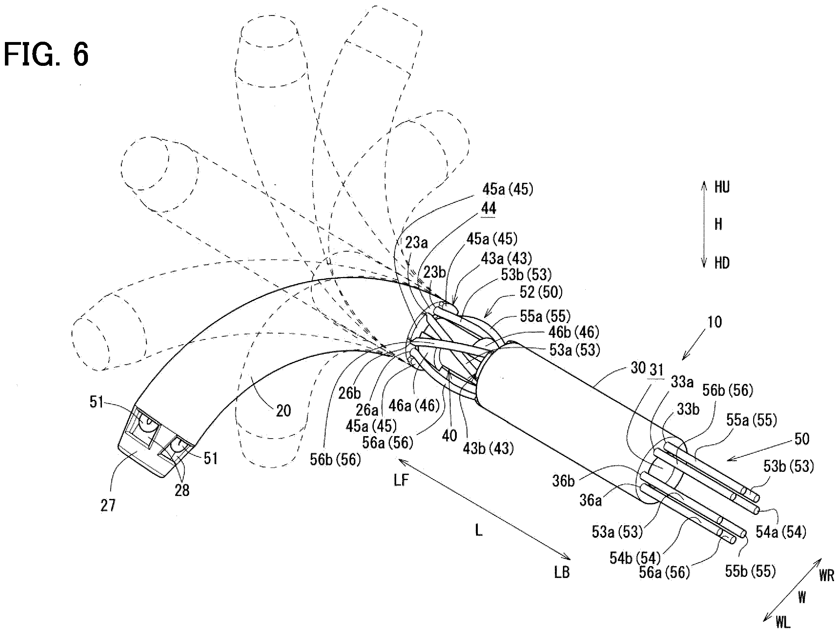

FIG. 6 shows a perspective view of the movable elongated structure 10 with the distal end side flexible tube 20 bent.

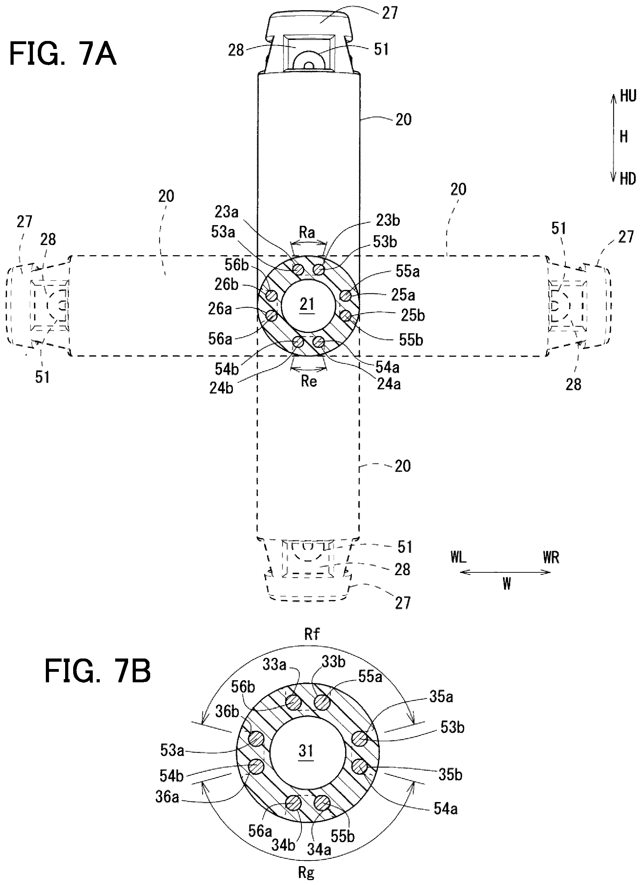

FIGS. 7A and 7B show explanatory views of the movable elongated structure 10 with the distal end side flexible tube 20 bent. FIG. 7A shows a cross-sectional view corresponding to the cross-sectional view taken along the line B-B of FIG. 2A in the movable elongated structure 10 with the distal end side flexible tube 20 bent, and FIG. 7B shows an enlarged cross-sectional view corresponding to the cross-sectional view taken along the line C-C of FIG. 2A.

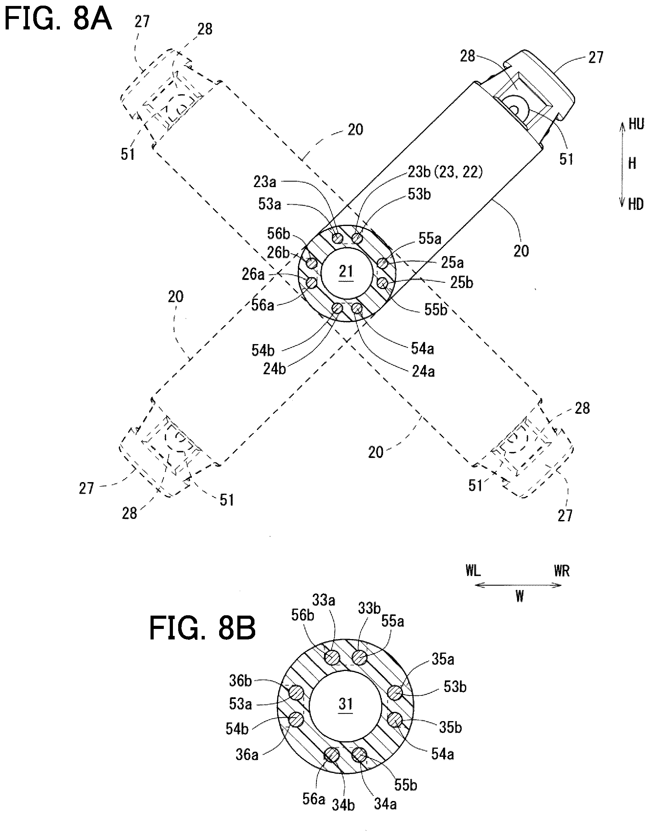

FIGS. 8A and 8B show explanatory views of the movable elongated structure 10 with the distal end flexible tube 20 obliquely bent. FIG.8A shows a cross-sectional view corresponding to the cross-sectional view taken along the line B-B in FIG. 2A in the movable elongated structure 10 with the distal end side flexible tube 20 obliquely bent, and FIG. 8B shows an enlarged cross-sectional view corresponding to the cross-sectional view taken along the line C-C of FIG. 2A.

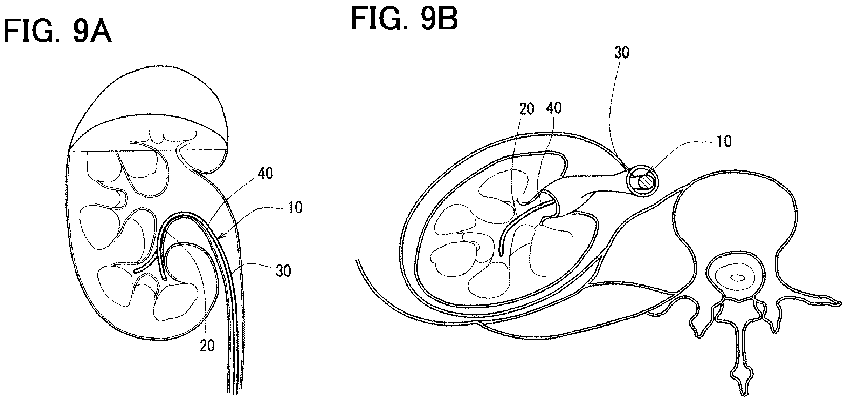

FIGS. 9A and 9B shows an image diagram of clinical use of the movable elongated structure 10, and FIGS. 10A to 10D show explanatory diagrams of the movable elongated structures 10a and 10b with the distal end side flexible tube 20 which is bent. FIG. 10A shows a cross-sectional view corresponding to the cross-sectional view of the movable elongated structure 10a in a bent state taken along the line B-B in FIG. 2A, and FIG. 10B shows an enlarged cross-sectional view corresponding to the arrow cross-sectional view taken along the line C-C in FIG. 2A in the movable elongated structures 10a. FIG. 10C shows a cross-sectional view of the movable elongated structure 10b in a bent state corresponding to the cross-sectional view of FIG. 2A taken along the line B-B, and FIG. 10D shows an enlarged cross-sectional view corresponding to the arrow cross-sectional view taken along the line C-C in FIG. 2A,

Note that the longitudinal direction of the movable elongated structure 10 is defined as a longitudinal direction L, and in the longitudinal direction L, the side of the distal end side flexible tube 20 with respect to the base end side flexible tube 30 is defined as a distal end side LF. The side of the base end side flexible tube 30 with respect to the tube 20 is defined as the base end side LB. For convenience of explanation of the movable elongated structure 10, the vertical direction in FIGS. 1A and 1B is defined as the height direction H, the upward direction as the upward direction HU, and the downward direction as the downward direction HD. Further, the direction connecting the upper right and the lower left in FIGS. 1A and 1B is defined as the width direction W, the upper right is defined as the right side WR, and the left lower side is defined as the left side WL.

Next, an embodiment of the movable elongated structure 10 will be described in detail with reference to FIGS. 1 to 5.

The movable elongated structure 10 includes a distal end side flexible tube 20 and a base end side flexible tube 30 arranged along the longitudinal direction L, a spacer 40 between the distal end side flexible tube 20 and the base end side flexible tube 30. and a traction wire 50 that passes through the inner walls of the flexible tubes 20 and 30. The movable elongated structure 10 is covered along the longitudinal direction L with an exterior cover (not shown). Also, only the outside of the spacer 40 may be covered with an exterior cover (not shown).

The distal end side flexible tube 20 is a cylindrical flexible tube elongated in a longitudinal direction L, and has a distal end side main lumen 21 inside (FIG. 3A: cross-sectional view taken along line B-B in FIG. 2A). The distal main lumen 21 is a space having a circular cross section along the longitudinal direction L.

Further, as shown in FIG. 3A, distal end side wire lumens 23a, 23b, 24a, 24b, 25a, 25b, 26a, 26b (hereinafter collectively referred to as 22) are provided inside the tube wall between the distal main lumen 21 and the outer peripheral surface in the distal flexible tube 20.

The distal end side wire lumen 22 is a space with a circular cross section extending in the longitudinal direction L inside the tube wall, and is formed with a diameter that allows a traction wire 50 to be described later to be inserted therethrough. The distal end side wire lumens 22 are provided in four directions on the tube wall having a ring-shaped cross section.

Specifically, as shown in FIG. 3A, there are provided, in a tube wall with a ring-shaped cross section, distal end side wire lumens 23a, 23b (hereinafter collectively referred to as 23) in the upward direction HU, distal end side wire lumens 24a, 24b (hereinafter collectively referred to as 24) in the downward direction HD, distal end side wire lumens 25a, 25b (hereinafter collectively referred to as 25) of the right WR, and wire lumens 26a, 26b (hereinafter collectively referred to as 26) on the distal end side of the left WL.

In addition, as described above, the distal end side wire lumens 22 provided in the four directions in the tube wall having the ring-shaped cross section are spaced apart at predetermined intervals in the circumferential direction of the circular cross section so that the pair of traction wires 50 may be inserted therethrough, with two each.

In addition, the counterclockwise side of the wire lumen 22 on the distal end side constituted by a pair of two through holes is 22a (23a, 24a, 25a, 26a), and the clockwise side is 22b (23b, 24b, 25b, 26b).

In addition, the interval Ra between two distal end wire lumens 22 (22a, 22b) provided in each of the four directions on the pipe wall having a ring-shaped cross section (Ra is illustrated between 23a and 23b in FIG. 3A) is set at an interval of about half the interval Rb between the distal end side wire lumens 22 (23b and 25a, 25b and 24a, 24b and 26a, 26b and 23a) adjacent in the circumferential direction (Rb between 23b and 25a in FIG. 3A).

It should be noted that the distal end side wire lumens 22 provided two by two in four directions may be integrally formed into an elliptical shape.

As shown in FIGS. 1, 2 and 5, a tip (or distal end) cap 27 is provided at the distal end LF of the distal end side flexible tube 20.

The distal end cap 27 has a bent recess 28 for forming a bent portion 51 of the traction wire 50, which will be described later. The bent recesses 28 are provided at four locations corresponding to the distal end side wire lumens 22 provided in four directions on the tube wall and are provided with two insertion holes through which the traction wires 50 are inserted.

In a device using the movable elongated structure 10, the distal end cap 27 may be provided with a through hole or the like that penetrates in the longitudinal direction L by means of an instrument or the like that is inserted through a main lumen formed by communicating the distal main lumen 21, an internal space 41 described later, and the base end side main lumen 31.

The distal end side flexible tube 20 configured as described above may be configured from a flexible tube such as polyamide elastomer, expanded polytetrafluoroethylene, polyurethane, or polytetrafluoroethylene.

As shown in FIGS. 1, 2 and 3, the base end side flexible tube 30 includes a cylindrical flexible tube elongated in the longitudinal direct ion L, similar to the distal end side flexible tube 20, and a base end side main lumen 31 inside. The base end side main lumen 31 is a space having a circular cross section along the longitudinal direction L.

Further, as shown in FIG. 3B (cross-sectional view taken along line C-C in FIG. 2A), base end side wire lumens 33a, 33b, 34a, 34b, 35a, 35b, 36a, 36b (hereinafter collectively referred to as 32) are provided inside a tube wall between the base end side main lumen 31 and the outer peripheral surface of the base end side flexible tube 30.

Like the distal end side wire lumen 22, the base end side wire lumen 32 is a circular cross-sectional space extending in the longitudinal direction L inside the tube wall and is formed with a diameter that allows a pulling wire 50, which will be described later, to pass through.

Like the distal end side wire lumens 22, the base end side wire lumens 32 are provided in four directions on the tube wall having a ring-shaped cross section.

Specifically, as shown in FIG. 3B, the base end side wire lumens 33a and 33b (hereinafter collectively referred to as 33) in the upward direction HU and the base end side wire lumen 34a in the downward direction HD in the pipe wall having a ring-shaped cross section 34b (hereinafter collectively referred to as 34), right WR base end side wire lumens 35a and 35b (hereinafter collectively referred to as 35), and left WL base end side wire lumens 36a and 36b (hereinafter collectively referred to as 36).

In addition, as described above, the base end side wire lumens 32 provided in the four directions in the tube wall having a ring-shaped cross section are arranged two each at predetermined intervals in the circumferential direction of the circular cross section so that the pair of traction wires 50 may be inserted through them.

Note that the base end side wire lumens 32, which are provided in four directions and are provided two by two, may be integrally formed into an elliptical shape.

In addition, the counterclockwise side of the base end side wire lumen 32 configured by a pair of two through holes is 32a (33a, 34a, 35a, 36a), and the clockwise side is 32b (33b, 34b, 35b, 36b).

In addition, the interval Rc between the base end side wire lumens 32 (32a, 32b) provided in each of the four directions on the pipe wall having a ring-shaped cross section (Rc is illustrated between 33a and 33b in FIG. 3B) is defined about half the interval Rd between base end side wire lumens 32 (33b and 35a, 35b and 34a, 34b and 36a, 36b and 33a) adjacent in the circumferential direction (Rd is example written between 33b and 35a in FIG. 3B).

Like the distal end side flexible tube 20, the base end side flexible tube 30 configured as described above may be constructed of flexible tube such as polyamide elastomer, expanded polytetrafluoroethylene, polyurethane, or polytetrafluoroethylene. The distal end side flexible tube 20 and the base end side flexible tube 30 may be made of the same material or may be made of different materials.

As shown in FIGS. 4 and 5, the spacer 40 includes a cylinder 42 having an inner space 41 having a circular cross section penetrating in the longitudinal direction L, and flanges 43 (43a and 43b) provided at both ends of the cylinder 42 in the longitudinal direction L.

The internal space 41 is formed with a space having the same diameter as the main lumens 21 and 31 described above. The cylinder 42 has a diameter that is arranged inside the diameter of the traction wire 50 that is inserted through the distal end side wire lumen 22 provided in the distal end side flexible tube 20 and the base end side wire lumen 32 provided in the base end side flexible tube 30.

The flanges 43 (43a, 43b) are formed with an outer diameter that protrudes radially outward from the outer diameter of the cylinder 42, and are referred to as a distal end side wire lumen 22 and a base end side wire lumen 33 (hereinafter referred to as wire lumens 22, 32). An arrangement recess 44 for arranging the traction wire 50 is provided at a corresponding location. The flange portion 43 is formed with an outer diameter equivalent to that of the distal end flexible tube 20 and the base end side flexible tube 30.

The arrangement recess 44 is a recess for collectively arranging two traction wires 50 to be inserted into the two wire lumens 22 and 32, and is formed with a width and depth corresponding to the two wire lumens 22 and 32.

Of the protrusions 43 provided on both sides in the longitudinal direction L of the cylinder 42, the distal end side LF is defined as a distal end side flange 43a, and the base end side LB is defined as a base end side flange 43b.

Further, a separation regulation part 45a is provided between a plurality of arrangement recesses 44 provided in the circumferential direction in the distal end side flange portion 43a. More specifically, separation regulation parts 45a provided on both sides of the placement recess (or arrangement) 44 restrict (or regulate) the traction wires 50 placed in the same placement recess 44 from being spaced apart in the circumferential direction at the distal end side flange portion 43a.

Specifically, the separation in the circumferential direction between the traction wires 50 arranged in the arrangement recesses 44 provided in the four directions in the distal end side flange 43a is regulated by the separation regulation parts 45a provided on both sides of the placement recess 44.

In addition, a proximity regulation part 45b is provided between a plurality of arrangement recesses 44 provided in the circumferential direction in the base end side flange 43b. More specifically, the proximity regulation portions 45b between the arrangement recesses 44 regulate the proximity of the traction wires 50 arranged in the arrangement recesses 44 adjacent in the circumferential direction in the base end side flange 43b to each other in the circumferential direction.

Specifically, the proximity between the traction wire 50 arranged on the right side WR in the arrangement recess 44 in the upward direction HU in the base end side flange 43b and the traction wire 50 arranged in the upward direction HU in the arrangement recess 44 on the right side WR is regulated by a separation regulation part 45a between the arrangement recess 44 in the upward direction HU and the arrangement recess 44 in the right side WR.

Similarly, the proximity between the traction wire 50 arranged in the downward direction HD in the arrangement recess 44 of the right WR and the traction wire 50 arranged in the right WR in the arrangement recess 44 in the downward direction HD is regulated by the separation regulation part 45a between the arrangement recess 44 on the right side WR and the arrangement recess 44 on the downward direction HD,
the proximity between the traction wire 50 arranged on the left side WL in the arrangement recess 44 in the downward direction HD and the pulling wire 50 arranged in the downward direction HD in the arrangement recess 44 on the left side WL is regulated by the separation regulation part 45a between the arrangement recess 44 in the downward direction HD and the arrangement recess 44 in the left side WL, and
the proximity between the traction wire 50 arranged in the upward direction HU in the arrangement recess 44 on the left side WL and the traction wire 50 arranged in the left side WL in the arrangement recess 44 in the upward direction HU is regulated by a separation regulation part 45a between the placement recess 44 on the left side WL and the placement recess 44 on the upward direction HU.

Furthermore, a guide convex part 46 for guiding the traction wire 50 radially outward is provided at the end of the cylinder 42 in the longitudinal direction L.

The guide convex part 46 is provided at a position corresponding to the placement recess 44 of the flange 43 at the end in the longitudinal direction L with a corresponding width, and formed to have a trapezoidal vertical cross-sectional shape in which the protrusion height gradually increases (see FIG. 4D) with extending from the end in the longitudinal direction L to the center of the cylinder 42 in the longitudinal direction L.

The guide convex parts 46 are provided at positions facing each other in the radial direction among the arrangement recesses 44 provided in the four directions at both ends in the longitudinal direction L, and the facing direction is the distal end side guide convex part 46a on the distal end side LF. and the base end side guide convex portion 46b of the base end side LB are provided so as to be perpendicular to each other.

Specifically, the distal end side guide convex portion 46a on the distal end side LF is provided in the upward direction HU and the downward direction HD so as to face each other in the height direction H, and the base end side guide convex portion 46b on the base end side LB is provided on the right side WR and the left side WL so as to face each other in the width direction W.

The spacer 40 configured as described above may be made of, for example, a metal material such as stainless steel, an elastic body such as polytetrafluoroethylene, an elastic body such as a spring, or a resin material.

Further, the spacer 40 may be configured by integrating the cylinder 42 and the flange 43, or may be configured separately and assembled. In addition, when the cylinder 42 and the flange 43 are configured separately, the cylinder 42 and the flange 43 may be configured with the same material, or may be configured with different materials.

The traction wire 50 is a flexible wire, and as shown in FIGS. 1 and 2, and formed with a length that is at least twice as long as the length in the longitudinal direction L of the movable elongated structure 10.

The traction wire 50 is composed of a bent portion 51 arranged in the bent concave portion 28 of the tip cap 27 and two traction portions 52 on the base end side LB from the bent portion 51.

As described above, the traction wire 50 that is bent back at the bending portion 51 and has a pair of traction portions 52 is provided with four pieces corresponding to the wire lumens 22 and 32 provided in the four directions.

Specifically, traction portions 53a and 53b (hereinafter collectively referred to as 53) arranged generally in the upward direction HU, and traction portions 54a and 54b (hereinafter collectively referred to as 54) are arranged generally in the downward direction HD. There are traction portions 55a, 55b (hereinafter collectively referred to as 55) located on the right side WR and a traction portion 56a, 56b (hereinafter collectively referred to as 56) located generally on the left side WL.

Ones of the portions corresponding to the traction portion 52 in each of the traction portions 53, 54, 55, 56 (counterclockwise side in the inserted state) are set as the traction portion 53a, 54a, 55a, 56a, and the others (clockwise side in the inserted state) are served as traction portions 53b, 54b, 55b, and 56b.

The traction wire 50 may be made of a metal material such as stainless steel, nylon, fluorocarbon, or the like.

Assembly of the movable elongated structure 10 having the elements configured as described above will now be described. First, the distal end side flexible tube 20, the spacer 40, and the base end side flexible tube 30 are arranged in series along the longitudinal direction L from the distal end side LF toward the base end side LB.

At this time, as shown in FIGS. 1, 2 and 5, the distal end side wire lumen 22 (23, 24, 25, 26), the four-direction placement recesses 44, the base end side wire lumen 32 (33, 34, 35, 36) are arranged to communicate with each other in the longitudinal direction L.

Then, the traction portion 52 (FIG. 1B) of the traction wire 50, which is bent around the center of the length to form the bent portion 51, is inserted through the distal end side wire lumen 22, the placement recess 44, and the base end side wire lumen 32.

Specifically, first, the traction portion 53a of the traction portion 53 is inserted from the distal end side LF into the distal end side wire lumen 23a (FIG. 3A) of the left side WL in the distal end side wire lumen 23, the traction portion 53b is inserted to the distal end side wire lumen 23b of the right side WR to lead out the traction portion 53 from the base end side LB of the distal end side wire lumen 23, and the bent portion 51 is arranged in the bending concave portion 28 in the upward direction HU.

The traction portion 53 led out from the base end side LB of the distal end side wire lumen 23 is arranged in the arrangement recess 44 in the upward direction HU of the distal end side flange 43a, obliquely downwards toward the base end side LB along the cylinder 42, stretched and arranged in the upward direction HU of the arrangement recess 44 in the width direction W of the base end side flange 43b.

Specifically, the traction portion 53a led out from the distal end side wire lumen 23a is arranged on the left side WL of the arrangement recess 44 in the upward direction HU in the distal end side flange 43a, arranged in the downward direction HD and the left side along the cylinder 42, extends toward WL and is arranged in the upward direction HU of the placement recess 44 on the left side WL of the base end side flange 43b. Then, it is inserted into the base end side wire lumen 36b in the upward direction HU in the base end side wire lumen 36 on the left side WL of the base end side flexible tube 30, and led out from the base end side LB.

The traction portion 53b led out from the distal end side wire lumen 23b is arranged on the right side WR in the arrangement recess 44 in the upward direction HU in the distal end side flange portion 43a, and extends downward HD and right side WR along the cylinder 42, in the upward direction HU of the arrangement recess 44 on the right side WR of the base end side flange 43b. Then, it is inserted into the base end side wire lumen 35a in the upward direction HU in the base end side wire lumen 35 on the right side WR of the base end side flexible tube 30, and led out from the base end side LB.

As shown in FIGS. 2 and 3, the distal end side wire lumen 22 of the distal end side flexible tube 20 and the base end side wire lumen 32 of the base end side flexible tube 30 through which the traction portion 52 is inserted as described above are a set of through holes 11. The distal end side wire lumen 23a of the distal end side flexible tube 20 and the base end side wire lumen 36b of the base end side flexible tube 30 are formed into a through hole set 11a through which the traction portion 53a is inserted, and the distal end side wire lumen 23b and the base end side wire lumen 36b are formed into a pair of through holes 11a. The base end side wire lumen 35a is a through hole set 11b through which the traction portion 53b is inserted.

Then, as shown in FIG. 3B, a through hole set 11a is formed, and the center of the base end side wire lumen 36b through which the traction portion 53 is inserted, and the base end through which the traction portion 53 is inserted are formed. A line passing through the center of the side wire lumen 35a is defined as a base end side virtual line BVL, and a line passing through the center of the base end side flexible tube 30 and parallel to the base end side virtual line BVL is defined as a base end side center line BCL, and the interval between the base end center line BCL and the base end virtual line BVL is defined as a base end interval X2.

As shown in FIG. 3A, the center of the distal end side wire lumen 23a that constitutes the through hole set 11a and through which the traction portion 53 is inserted, and the distal end side wire lumen 23b that constitutes the through hole set 11b and through which the traction portion 53 is inserted. A line passing through the center of the distal end side is defined as a distal end side virtual line FVL, and a line passing through the center of the distal end side flexible tube 20 and parallel to the distal end side virtual line FVL is defined as a distal end side center line FCL. Assuming that the interval from the distal end side virtual line FVL is the distal end side interval X1, the distal end side interval X1 is wider than the base end side interval X2.

Next, as shown in FIGS. 1 and 3A, the traction portion 54a of the traction portion 54 is inserted from the distal end side LF into the distal end side wire lumen 24a of the right side WR in the distal end side wire lumen 24, and the traction portion 54b is inserted into the left side WL. It is inserted into the distal end side wire lumen 24b to lead out the traction portion 54 from the base end side LB of the distal end side wire lumen 24, and the bent portion 51 is arranged in the bending concave portion 28 in the downward direction HD.

The traction portion 54 led out from the base end side LB of the distal end side wire lumen 24 is placed in the downward HD placement recess 44 of the distal end side flange 43a, and obliquely upwards toward the base end side LB along the cylinder 42. It is stretched and arranged in the downward direction HD of the arrangement recess 44 in the width direction W of the base end side flange 43b.

Specifically, the traction portion 54a led out from the distal end side wire lumen 24a is arranged on the right side WR of the arrangement recess 44 in the downward direction HD in the distal end side flange 43a, and arranged in the upward direction HU and the right side along the cylinder 42, extends toward the WR, and is arranged in the downward direction HD of the arrangement recess 44 of the right WR in the base end side flange 43b. Then, it is inserted into the base end side wire lumen 35b in the downward direction HD in the base end side wire lumen 35 of the right side WR of the base end side flexible tube 30, and led out from the base end side LB.

The traction portion 54b led out from the distal end side wire lumen 24b is arranged on the left side WL of the arrangement recess 44 in the downward direction HD of the distal end side flange 43a, and extends along the cylinder 42 in the upward direction HU and the left side WL, in the downward direction HD of the arrangement recess 44 on the left side WL of the base end side flange 43b. Then, it is inserted into the base end side wire lumen 36a in the downward direction HD in the base end side wire lumen 36 on the left side WL of the base end side flexible tube 30, and led out from the base end side LB.

The distal wire lumen 24a of the distal end side flexible tube 20 and the base end side wire lumen 35b of the base end side flexible tube 30 form a through hole set 11c through which the traction portion 54a is inserted. The base end side wire lumen 36a is a through hole set 11d through which the traction portion 54b is inserted (see FIGS. 3A and 3B).

With respect of the base end side interval (X2) between the base end side virtual line (BVL) passing through the center of the base end side wire lumen 35b that constitutes the through hole set 11c through which the traction portion 54 is inserted and the center of the base end side wire lumen 36a that constitutes the through hole set 11d through which the traction portion 54 is inserted and the base end side center line (BCL) passing through the center of the base end side flexible tube 30, the distal end side interval (X1) between a distal end side virtual line (FVL) that passes through the center of the distal wire lumen 24a that constitutes the through hole set 11c through which the traction portion 54 is inserted and the center of the distal wire lumen 24b that constitutes the through hole set 11d through which the traction portion 54 is inserted and the distal center line (FCL) passing through the center of the distal end side flexible tube 20 becomes wider.

Next, as shown in FIG. 3A, the traction portion 55a of the traction portion 55 is inserted from the distal end side LF into the distal end side wire lumen 25a of the distal end side wire lumen 25 in the upward direction HU, the traction portion 55b is inserted into the distal end side wire lumen 25b of the downward direction HD, the traction portion 55 is led out from the base end side LB of the distal end side wire lumen 25, and the bent portion 51 is arranged in the bent concave portion 28 of the right side WR.

The traction portion 55 led out from the base end side LB of the distal end side wire lumen 25 is placed in the placement recess 44 on the right side WR of the distal end side flange 43a, pulled along the cylinder 42 toward the base end side LB toward the center in the width direction, and arranged on the right side WR of the arrangement recess 44 in the height direction H of the base end side flange 43b.

Specifically, as shown in FIGS. 1, 3, and 5, the traction portion 55a led out from the distal end side wire lumen 25a is directed upward HU in the arrangement recess 44 of the right WR of the distal end side flange 43a. It extends in the left side WL and upward direction HU, and is arranged in the right side WR of the placement recess 44 in the upward direction HU in the base end side flange 43b. Then, it is inserted into the base end side wire lumen 33b of the right side WR in the base end side wire lumen 33 in the upward direction HU of the base end side flexible tube 30, and led out from the base end side LB.

In addition, the traction portion 55a that is arranged in the upward direction HU in the arrangement recess 44 on the right side WR of the distal end side flange 43a, extends toward the left side WL and upward HU, and extends toward the upward direction HU in the arrangement recess 44 in the base end side flange 43b is arranged across the distal side guide convex portion 46a of the right WR provided on the distal side LF and the base end side guide convex portion 46b of the upward direction HU provided on the base end side LB, and separated from the outer surface of the cylinder 42 between the distal end side guide convex portion 46a and the base end side guide convex portion 46b.

The traction portion 55b led out from the distal wire lumen 25b is arranged in the downward direction HD in the arrangement recess 44 of the right side WR in the distal end side flange 43a, extends toward the left side WL and downward HD, and extends toward the base end side projection. It is arranged on the right side WR of the arrangement recess 44 in the downward direction HD in the edge 43b. Then, it is inserted into the base end side wire lumen 34a on the right side WR in the base end side wire lumen 34 in the downward direction HD of the base end side flexible tube 30, and led out from the base end side LB.

In addition, it is arranged in the upward direction HU in the arrangement recess 44 of the right side WR of the distal end side flange 43a, extends toward the left side WL and downward HD, and extends downward HD of the arrangement recess 44 of the base end side flange 43b. The traction portion 55b arranged on the right WR straddles the distal end side guide convex part 46a of the right WR provided on the distal end side LF and the base end side guide convex part 46b provided on the base end side LB in the downward direction HD and is spaced apart from the outer surface of the cylinder 42 between the distal end side guide convex part 46a and the base end side guide convex part 46b.

The distal wire lumen 25a of the distal end side flexible tube 20 and the base end side wire lumen 33b of the base end side flexible tube 30 are formed into a through hole set 11e through which the traction portion 55a is inserted, and the distal wire lumen 25b and the base end side wire lumen 33b are formed into a pair of through holes 11e. The base end side wire lumen 34a is a through hole set 11f through which the traction portion 55b is inserted (see FIGS. 3A and 3B).

The center of the base end side wire lumen 33b that constitutes the through hole group 11e and through which the traction portion 55 is inserted, and the center of the base end side wire lumen 34a that constitutes the through hole group 11f and through which the traction portion 55 is inserted are separated. The through hole set 11e is formed with respect to the base end side distance (X2) between the base end side virtual line (BVL) passing through and the base end side center line (BCL) passing through the center of the base end side flexible tube 30. , a distal virtual line (FVL) passing through the center of the distal end side wire lumen 25a through which the traction portion 55 is inserted, and the center of the distal end side wire lumen 25b that constitutes the through hole set 11f and through which the traction portion 55 is inserted, and the distal center line (FCL) passing through the center of the distal flexible tube 20 (X1) becomes wider.

Next, the traction portion 56a of the traction portion 56 from the distal end side LF is inserted into the distal wire lumen 26a of the distal wire lumen 26 in the downward direction HD, and the traction portion 56b is inserted into the distal wire lumen 26b of the upward direction HU. Then, the traction portion 56 is led out from the base end side LB of the distal end side wire lumen 26, and the bent portion 51 is arranged in the bent concave portion 28 on the left side WL.

The traction portion 56 led out from the base end side LB of the distal end side wire lumen 26 is arranged in the arrangement recess 44 on the left side WL of the distal end side flange 43a, and along the cylinder 42, toward the base end side LB, the center in the width direction, and is arranged on the left side WL of the arrangement recess 44 in the height direction H of the base end side flange 43b.

Specifically, as shown in FIGS. 1 and 5A, the traction portion 56a led out from the distal wire lumen 26a is arranged in the downward direction HD in the arrangement recess 44 on the left side WL of the distal end side flange 43a, extends to the right side WR and downward HD, and is arranged on the left side WL of the arrangement recess 44 in the downward direction HD in the base end side flange 43b. Then, it is inserted into the base end side wire lumen 34b on the left side WL in the base end side wire lumen 34 in the downward direction HD of the base end side flexible tube 30, and led out from the base end side LB.

In addition, the traction portion 56a that is arranged in the downward direction HD in the arrangement recess 44 on the left side WL in the distal end side flange 43a, extends toward the right side WR and downward HD, and is arranged on the left side WL of the arrangement recess 44 in the downward direction HD in the base end side flange 43b is arranged across the distal end side guide convex part 46a of the left side WL provided on the distal end side LF and the base end side guide convex part 46b provided on the base end side LB in the downward direction HD, and is spaced apart from the outer surface of the cylinder 42 between the distal end side guide convex part 46a and the base end side guide convex part 46b.

The traction portion 56b led out from the distal wire lumen 26b is arranged in the upward direction HU in the arrangement recess 44 on the left side WL of the distal end side flange 43a, extends toward the right side WR and upward HU, and is arranged on the left side WL of the arrangement recess 44 in the upward direction HU in the edge 43b. Then, it is inserted into the base end side wire lumen 33a on the left side WL of the base end side wire lumen 33 in the upward direction HU in the base end side flexible tube 30, and led out from the base end side LB.

In addition, the traction portion 56b that is arranged in the downward direction HD in the arrangement recess 44 on the left side WL of the distal end side flange 43a, extends toward the right side WR and upward HU, and is arranged on the left side WL of the arrangement recess 44 in the upward direction HU on the base end side flange 43b is arranged across the distal end side guide convex part 46a of the left side WL provided on the distal end side LF and the base end side guide convex part 46b provided on the base end side LB in the upward direction HU, and is spaced apart from the outer surface of the cylinder 42 between the distal end side guide convex part 46a and the base end side guide convex part 46b.

The distal wire lumen 26a of the distal end side flexible tube 20 and the base end side wire lumen 34b of the base end side flexible tube 30 are formed into a through hole set 11g through which the traction portion 56a is inserted, and the distal wire lumen 26b and the base end side wire lumen 34b are formed into a pair of through holes 11g. The base end side wire lumen 33a is a through hole set 11h through which the traction portion 56b is inserted (see FIGS. 3A and 3B).

With respect to a base end side interval (X2) between a base end side imaginary line (BVL) passing the center of the base end side wire lumen 34b that constitutes a through hole set 11g and through which the traction portion 56 is inserted, and the center of the base end side wire lumen 33a that constitutes a through hole set 11h and through which the traction portion 56 is inserted, and the base end side center line (BCL) passing through the center of the base end side flexible tube 30,
a distal end side interval (X1) between a distal virtual line (FVL) passing the center of the distal wire lumen 26a that constitutes the through-hole set 11g and through which the traction portion 56 is inserted and the center of the distal end side wire lumen 26b that constitutes the through hole set 11h and through which the traction portion 56 is inserted, and the distal end side center line (FCL) passing through the center of the distal end side flexible tube 20 becomes wider.

In addition, in the spacer 40 of the movable elongated structure 10 assembled as described above, the traction portion 55 and the traction portion 56 extend obliquely across the distal end side guide convex part 46a on the distal end side LF and the base end side guide convex part 46b on the base end side LB, and arranged radially outwardly away from the cylinder 42, whereby they are arranged beyond the radially outer sides of the traction portions 53 and 54 extending obliquely along the cylinder 42 .

When the traction wires 50 are inserted through the wire lumens 22 and 32 and arranged as described above, the two traction portions 52 are respectively arranged in the arrangement recesses 44 of the spacer 40.

Specifically, as shown in FIG. 5D, the traction portions 53a and 53b are arranged in the arrangement recess 44 in the upward direction HU in the distal end side flange portion 43a, and the traction portions 54a and 54b are arranged in the arrangement recess 44 in the downward direction HD. The traction portions 55a and 55b are disposed in the placement recess 44 on the right side WR, and the traction portions 56a and 56b are placed in the placement recess 44 on the left side WL.

Then, as shown in FIG. 5C, the traction portions 56b and 55a are arranged in the arrangement recess 44 in the upward direction HU in the base end side flange portion 43b, and the traction portions 56a and 55b are arranged in the arrangement recess 44 in the downward direction HD. The traction portions 53b and 54a are arranged in the placement recess 44 on the right side WR, and the traction portions 53a and 54b are placed in the placement recess 44 on the left side WL.

In the above description, the traction portions 53, 54, 55, and 56 are inserted in the order, but the insertion order is not limited and may be inserted in any order.

Furthermore, after the traction wire 50 is inserted through the distal wire lumen 22 of the distal end side flexible tube 20 and the base end side wire lumen 32 of the base end side flexible tube 30 arranged along the longitudinal direction L, loosening the traction wire 50 between the flexible tube 20 and the base end side flexible tube 30, placing the spacer 40 between the distal end side flexible tube 20 and the base end side flexible tube 30, and placing the loosened traction wire 50 in the placement recess 44 for assembly.

The movable elongated structure 10 configured in this way has the following features compared to conventional bidirectionally movable ureteroscopes and movable bile duct catheters. The movable elongated structure 10 may be formed with an outer diameter (1.8 to 3.2 mm) having a narrowest diameter smaller than that of a ureteroscope and a larger diameter than that of a movable bile duct catheter.

In addition, flexible tubes may be used for the distal end side flexible tube 20 and the base end side flexible tube 30 that constitute the movable elongated structure 10, and the maximum bending angle of 270 degrees, which is equivalent to that of a ureteroscope, is possible. It may freely move and bend in four directions (all directions) to a certain extent.

As shown in FIGS. 6 to 8, thus constructed movable elongated structure 10 may be smoothly bent in the longitudinal direction L by pulling the traction wire 50 toward the base end side LB.

Specifically, the distal end side flexible tube 20 is bent upward HU by pulling the traction portion 53 through which the distal wire lumen 23 in the upward direction HU is inserted in the distal end side flexible tube 20 toward the base end side LB. The distal end side flexible tube 20 may be bent downward HD by pulling the traction portion 54 inserted through the distal wire lumen 24 in the downward direction HD toward the base end side LB.

By pulling the traction portion 55 of the distal end side flexible tube 20 through which the distal wire lumen 25 of the right WR is inserted to the base end side LB, the distal end side flexible tube 20 may be bent to the right WR and left WL. The distal end side flexible tube 20 may be bent to the left side WL by pulling the traction portion 56 passing through the distal wire lumen 26 toward the base end side LB.

More specifically, as shown in FIGS. 7A and 7B, the traction portion 53a of the traction portion 53 that passes through the distal wire lumen 23a (FIG. 7A) in the upward direction HU in the distal end side flexible tube 20 is inserted into the base end side wire lumen 36b (FIG. 7B) of the base end side wire lumen 36 of the left side WL (FIG. 7B) in the base end side flexible tube 30, and the traction portion 53b is inserted through the wire lumen 35a in the base end side of the upward direction HU of the base end side wire lumen 35 of the right side WR.

A circumferential interval Rf between the base end side wire lumen 35a and the base end side wire lumen 36b, which is the upward direction HU of the base end side wire lumens 35 and 36 in the width direction W, is larger than the interval Ra between the distal end side wire lumen 23 and the distal end side wire lumen 23b, and the distal end side wire lumens 23a and 23b are positioned in the upward direction HU with respect to the base end side wire lumens 35a and 36b.

Therefore, when the traction portion 53 is pulled toward the base end side LB, as shown in FIG. 7A, the base end side wire lumens 35a and 36b, which are widely spaced in the circumferential direction, exert a pulling force on the base end side flexible tube 30 in the bending direction, and the traction force acts eccentrically in the upward direction HU by the traction portion 53 inserted into the distal wire lumen 23 arranged in the upward direction HU in the distal end side flexible tube 20, and the distal end side flexible tube 20 may be bent upward HU.

Similarly, the traction portion 54a of the traction portion 54 passing through the distal wire lumen 24 in the downward direction HD in the distal end side flexible tube 20 is the base end portion WR in the base end side flexible tube 30, as shown in FIG. 7B is inserted through the base end side wire lumen 35b in the downward direction HD of the base end side wire lumen 35, and the traction portion 54b is inserted through the base end side wire lumen 36a in the downward direction HD of the base end side wire lumen 36 of the left side WL.

A circumferential interval Rg between the base end side wire lumens 35b and 36a, which is the downward direction HD of the base end side wire lumens 35 and 36 in the width direction W is wider than the circumferential interval Re in the circumferential direction between the distal wire lumen 24a and the distal wire lumen 24b of the distal end side wire lumen 24, and the distal end side wire lumens 24a and 24b on are positioned downward HD with respect to the base end side wire lumens 35b and 36a.

Therefore, when the traction portion 54 is pulled toward the base end side LB, the base end side wire lumens 35b and 36a, which are widely spaced in the circumferential direction, hardly exert a pulling force on the base end side flexible tube 30 in the bending direction, the traction portion 54 inserted into the distal wire lumen 24 arranged in the downward direction HD in the distal end side flexible tube 20 acts eccentrically in the downward direction HD to bend the distal end side flexible tube 20 in the downward direction HD.

The traction portion 55a of the traction portion 55 through which the right WR distal wire lumen 25 of the distal end side flexible tube 20 is inserted is the right WR of the base end side wire lumen 33 of the base end side flexible tube 30 in the upward direction HU, and the traction portion 55b is inserted through the base end side wire lumen 34a of the right side WR of the base end side wire lumen 34 in the downward direction HD.

The interval between the base end side wire lumens 33b and 34a, which is the right WR of the base end side wire lumens 33 and 34 in the height direction H, is the interval between the distal wire lumen 25a of the distal wire lumen 25 and the distal wire lumen 25a. 25b, and the distal wire lumens 25a, 25b are positioned on the right side WR with respect to the base end side wire lumens 33b, 34a.

Therefore, when the traction portion 55 is pulled toward the base end side LB, as shown in FIG. 7A, the base end side wire lumens 33b and 34a, which are widely spaced in the circumferential direction, exert a pulling force on the base end side flexible tube 30 in the bending direction. The traction force acts eccentrically on the right side WR due to the traction portion 55 inserted into the distal end side wire lumen 25 arranged on the right side WR of the distal end side flexible tube 20, whereby the distal end side flexible tube 20 may be bent to the right WR.

Furthermore, the traction portion 56a of the traction portion 56 inserted through the distal wire lumen 26 on the left side WL of the distal end side flexible tube 20 is the left side WL of the base end side wire lumen 34 in the downward direction HD in the base end side flexible tube 30. As shown in FIG. 7B, the traction portion 56b is inserted through the base end side wire lumen 33a on the left side WL of the base end side wire lumen 33 in the upward direction HU.

A circumferential distance or interval between the base end side wire lumens 33a and 34b, which is the left side WL of the base end side wire lumens 33 and 34 in the height direction H, is wider than the circumferential interval between distal end side wire lumens 26a and 26b of the distal wire lumen 26, and the distal wire lumens 26a and 26b are positioned on the left side WL with respect to the base end side wire lumens 33a and 34b.

Therefore, when the traction portion 56 is pulled toward the base end side LB, in the base end side wire lumens 33a and 34b, which are widely spaced in the circumferential direction, hardly act on the base end side flexible tube 30 in the bending direction, a traction force acts eccentrically on the left side WL by the traction portion 56 inserted into the distal end side wire lumen 26 arranged on the left side WL in the distal end side flexible tube 20, whereby the distal end side flexible tube 20 may be bent to the left side WL.

Also, as described in detail above and shown in FIG. 7A, the distal side flexible tube 20 is bent upward HU by pulling the traction portion 53 to the base end side LB, the distal flexible tube 20 is bent downward HD by pulling the traction portion 54, the distal flexible tube 20 is bent rightward WR by pulling the traction portion 55, the distal flexible tube 20 is bent leftward WL by pulling the traction portion 56 in the movable elongated structure 10, whereby as shown in FIGS. 8A and 8B, the distal flexible tube 20 may be bent in an oblique direction crossing the height direction H and the width direction W by pulling the pulling wire 50.

Specifically, when the traction portion 53 that bends the distal end side flexible tube 20 upward HU and the traction portion 55 that bends it rightward WR are pulled toward the base end side LB with the same pulling force (FIG. 8A), the distal end side flexible tube 20 may be bent obliquely upward to the right at 45 degrees when viewed from the longitudinal direction L with respect to the upward direction HU and the right side WR.

If the traction force of the traction portion 55 is greater than that of the traction portion 53, the force for bending to the right side WR is increased, and the vehicle is bent obliquely upward to the right toward the direction of the right side WR, the force for bending in the upward direction HU increases, and the bending occurs in an oblique upward right direction approaching the upward direction HU.

Also, when the distal end side flexible tube 20 is pulled to the base end side LB with the same traction force as the traction force which is applied to the traction portion 53 bent upward HU and the traction portion 56 bent leftward WL, as shown in FIG. 8A, the distal end side flexible tube 20 may be bent upward and left at 45 degrees from the longitudinal direction L with respect to the upward direction HU and the left side WL.

If the traction force of the traction portion 56 is greater than that of the traction portion 53, the force of bending to the left side WL increases, and the distal end side flexible tube 20 comes to be bent obliquely upward to the left toward the direction of the left side WL. If the pulling force of the traction portion 53 is made larger than the pulling force of the traction portion 56, the force of bending in the upward direction HU becomes large, and it bends in the left oblique upward direction approaching the upward direction HU direction.

Conversely, if the traction portion 54 that bends the distal end side flexible tube 20 downward HD and the traction portion 55 that bends the distal end side flexible tube 20 rightward WR are pulled toward the base end side LB with the same pulling force, the distal end side flexible tube 20 may be bent obliquely downward to the right at 45 degrees for downward HD and right WR when viewed from the longitudinal direction L.

If the traction force of the traction portion 55 is greater than that of the traction portion 54, the force for bending to the right side WR is increased, and it is bent obliquely downward to the right toward the direction of the right side WR, wherein when the traction force of the traction portion 54 is made larger than the traction force of the traction portion 55, the downward bending force HD becomes large, and the bending is made obliquely to the right toward the downward HD direction.

When the traction portion 54 bending the distal end side flexible tube 20 downward HD and the traction portion 56 bending the distal end side flexible tube 20 leftward WL are pulled toward the base end side LB with the same pulling force, the distal end side flexible tube 20 is pulled downward HD and leftward WL and may be bent obliquely downward to the left at 45 degrees when viewed from the longitudinal direction L.

If the traction force of the traction portion 56 is greater than that of the traction portion 54, the force for bending to the left side WL increases, and it is bent obliquely downward to the left toward the direction of the left side WL, wherein when the traction force of the traction portion 54 is made larger than the traction force of the traction portion 55, the downward bending force HD becomes large, and the bending is made obliquely to the right toward the downward HD direction.

Furthermore, when the traction portions 53, 54, 55, 56 are pulled with the same pulling force, the flexible tubes 20 and 30 are compressed in the longitudinal direction L, whereby the length in the longitudinal direction L of the movable elongated structure 10 may also be shortened.

As described above, the moveable elongated structure 10 may be pulled distally relative to the base end side flexible tube 30 as shown in FIG. 6, the side flexible tube 20 may be bent or shortened in any desired direction.

However, as described above, the traction wire 50 arranged in the arrangement recess 44 of the spacer 40 extends in an oblique direction that intersects with the longitudinal direction L. Although the force acts, the movement is restricted by the restriction portions 45 (45a, 45b), which are the edges of the arrangement recess 44 in the flange portion 43. Therefore, it is possible to prevent a circumferential force from acting on the distal wire lumen 22 and the base end side wire lumen 32 through which the traction wire 50 is inserted.

In addition, since the movable elongated structure 10 that may bend the distal flexible tube 20 in a desired direction by pulling the traction wire 50 is provided with the placement recess 44 in which the traction wire 50 is placed is a recess that is open radially outward in the flange portion 43 of the spacer 40, as shown in FIG. 3C, when the puller wire 50 is allowed to slack between the distal flexible tube 20 and the proximal flexible tube 30, the traction wire 50 is removed from the arrangement recess 44, and the pacer 40 may be removed from between distal end side flexible tube 20 and base end side flexible tube 30 .

Therefore, for example, spacers 40a having different lengths in the longitudinal direction L or spacers having different deformability due to materials may be easily exchanged.

As mentioned above, the movable elongated structure 10 includes:
a distal end side flexible tube 20 and a base end side flexible tube 30 arranged in series along the longitudinal direction L from the distal end side LF toward the base end side LB;
a pair of flexible traction wires 50 passing through a pair of wire lumens 22, 32 along the longitudinal direction L provided inside the tube walls of the flexible tubes 20, 30;
a spacer 40 disposed between the distal end side flexible tube 20 and the base end side flexible tube 30 to regulate the direction of the traction wire 50,
wherein the interval in the circumferential direction between the pair of base end side wire lumens 32 provided in the base end side flexible tube 30 is set wider than the interval in the circumferential direction between a pair of distal wire lumens 22 provided in the distal flexible tube 20 and through which the same traction wire 50 is inserted,
wherein the spacer 40 includes a tubular cylinder 42 arranged along the longitudinal direction L, a base end side flange 43b provided at the end portion of the base end side LB of the cylinder 42 protruding radially outward and a distal end side flange 43a provided at the end of the distal end side LF of the cylinder 42 protruding radially outward,
wherein at the base end side flange 43b, there are provided a proximity regulation portion 45b that regulates proximity in the circumferential direction of the traction wire 50 led out from the base end side wire lumen 32 and introduced into the distal side wire lumen 22, and an arrangement recess 44 in which the radially outer side is open, arranged by the traction wire 50; and
wherein at the distal end side flange 43a, there are provided a separation regulation part 45a that regulates the separation in the circumferential direction of the traction wire 50 led out from the base end side wire lumen 32 and introduced into the distal end side wire lumen 22 on the distal side flange 43a, and an arrangement recess 44 that is open radially outward and in which the traction wire 50 is arranged.

Since thus constructed movable elongated structure 10 is configured such that the spacer 40 may be removed from between the distal side flexible tube 20 and the base end side flexible tube 30,
the ease of assembly of the movable elongated structure 10 may be improved, and the usability and versatility may be improved.

More specifically, by removing the spacer 40 from between the distal end side flexible tube 20 and the base end side flexible tube 30, the ease of assembly of the movable elongated structure 10 is improved and the movable elongated structure is improved. The spacer 40 may be replaced according to the application and specifications of the body 10.

In addition, when disposing of the movable elongated structural body 10, the spacer 40 may be removed for separate disposal.

In addition, a spacer (or wiring aid) 40 positioned between a flexible elongated distal end side flexible tube 20 and a base end side flexible tube 30 arranged in series along the longitudinal direction L from the distal end side LF toward the base end side LB is provided with a cylindrical cylinder 42 arranged along the longitudinal direction L, a base end side flange 43b provided at the end of the base end side LB of the cylinder 42 and protruding radially outward, and a distal end side flange 43a provided at the end of the distal end side LF of the cylinder 42 and protruding radially outward,
wherein the base end side flange 43b is provided with a proximity regulation portion 45b for regulating proximity in the circumferential direction of the traction wire 50 led out from the base end side wire lumen 32 and introduced into the distal wire lumen 22, and an arrangement recess 44 that is open radially outward and in which the traction wire 50 is arranged,
wherein the side flange portion 43a is provided with a separation regulation part 45a that regulates the separation in the circumferential direction of the traction wire 50 led out from the base end side wire lumen 32 and introduced into the distal end side wire lumen 22, and an arrangement recess 44 that is open radially outward and in which the traction wire 50 is arranged, and
wherein since the spacer 40 regulates the extending direction of a pair of flexible traction wires 50 passing through a pair of wire lumens 22, 32 along the longitudinal direction L provided inside the tube walls of the flexible tubes 20, 30, the traction wire 50 led out from the base end side wire lumen 32 provided in the base end side flexible tube 30 may be smoothly introduced into the distal end side wire lumen 22.

In addition, the interval (Rf, Rg) in the circumferential direction between the pair of base end side wire lumens 32 is set wider than the interval (Ra, Re) in the circumferential direction between the pair of distal end side wire lumens 22 through which the traction wire 50 is inserted.

In addition, with respect to the base end side interval (X2) between a base end virtual line BVL passing through the centers of the base end side wire lumen 32 through which the traction portion 52 is inserted and the base end side center line BCL passing through the center of the base end side flexible tube 30 and parallel to the base end virtual line BVL,
a distal end side interval X1 between a line parallel to the distal end side virtual line FVL passing through the centers of the distal end side wire lumens 22 through which the traction portion 52 is inserted and the distal end side center line FCL passing through the center of the distal end side flexible tube 20 and parallel to the distal end side virtual line FVL is widened.

Therefore, by pulling the pair of traction wires 50 inserted through the base end side wire lumen 32 with a wide circumferential interval and a narrow base end side interval X2, it is possible to bend the distal flexible tube 20 having the distal wire lumen 22 with a narrow interval in the circumferential direction and a wide distal interval X1.

The wire lumens 22, 32 are provided inside the tube walls of the flexible tubes 20, 30, and in the spacer 40, they are provided at the end of a cylindrical cylinder 42 and arranged on a flange 43 protruding radially outward. Since the recess 44 is provided, the traction wire 50 does not protrude into the internal space 41 of the spacer 40, and the main lumens 21 and 31 may be effectively utilized.

Furthermore, since the arrangement recess 44 provided at the end portion of the cylindrical cylinder 42 and provided in the flange portion 43 protruding radially outward is open radially outward, the traction wire 50 may be inserted into the wire lumens 22 and 32. After insertion, the spacer 40 may be arranged between the base end side flexible tube 30 and the distal end side flexible tube 20, and the traction wire 50 may be arranged in the arrangement recess 44, so that the movable elongated structure 10 may be placed. may improve the assim ilability.

Specifically, as shown in FIGS. 5C and 5D, the separation regulation portions 45a on both sides of the arrangement recess 44 in the upward direction HU regulates separation of the traction portions 53a and 53b inserted through the distal end side wire lumens 23a and 23b at the distal end side flange portion 43a.

Similarly, the separation regulation parts 45a on both sides of the arrangement recess 44 in the downward direction HD in the distal end side flange 43a restrict the separation of the traction portions 54a and 54b inserted through the distal end side wire lumens 24a and 24b. In the flange 43a, the separation regulation portions 45a on both sides of the arrangement recess 44 of the right side WR regulate the separation of the traction portions 55a and 55b inserted through the distal end side wire lumens 25a and 25b, and the left side of the distal end side flange 43a. Separation regulation parts 45a on both sides of the placement recess 44 of the WL regulate the traction portions 56a, 56b inserted through the distal end side wire lumens 26a, 26b from being separated from each other.

Then, the proximity (or approach) at the base end side flange 43b between a traction portion 53b inserted through the base end side wire lumen 35a of the upward direction HU of the base end side wire lumen 35 of the right WR and a traction portion 53a inserted through the proximal wire lumen 36b of the upward HU of the proximal wire lumen 36 of the left WL is regulated by the proximity (or approach) regulation part 45b between the arrangement recess 44 in the upward direction HU and the arrangement recess 44 in the right side WR, and the proximity regulation part 45b between the arrangement recess 44 in the upward direction HU and the arrangement recess 44 in the left side WL.

Further, in the base end side flange 43b, the traction portion 54a inserted through the base end side wire lumen 35b in the downward direction HD of the right-hand side WR and the base end side wire lumen 36 of the left-hand side WL. The proximity of the traction portion 54b inserted in the base-side wire lumen 36a in the downward direction HD is controlled by the proximity regulation portion 45b between the arrangement recess 44 in the downward direction HD and the arrangement recess 44 in the right side WR, and the downward direction. This is regulated by a proximity regulation part 45b between the placement recess 44 of the HD and the placement recess 44 of the left side WL.

Similarly, in the base end side flange portion 43b, approach between a traction portion 55a inserted through the base end side wire lumen 33b of the right WR of the base end side wire lumen 35 in the upward direction HU, and the traction portion 55b inserted through the base end side wire lumen 34a of the right WR of the base end side wire lumen 33b in the downward direction HD is regulated by the proximity regulation part 45b between the placement recess 44 on the right side WR and the placement recess 44 on the upward direction HU and the proximity regulation part 45b between the recessed portion 44 on the right side WR and the recessed portion 44 on the downward side HD.

In addition, in the base end side flange portion 43b, a traction portion 56b inserted through the base end side wire lumen 33a on the left side WL of the base end side wire lumen 36 in the upward direction HU, and the base end side wire lumen 34 in the downward direction HD. The proximity of the traction portion 56a inserted in the base end side wire lumen 34b of the left WL of the left WL to the proximity regulation part 45b between the arrangement recess 44 of the left WL and the arrangement recess 44 of the upward direction HU and the left WL is regulated by the proximity regulation part 45b between the arrangement recess 44 in the direction HD and the arrangement recess 44 in the downward direction HD.

Further, as shown in FIGS. 3A and 3B, a pair of base end side wire lumens 32 and a pair of distal wire lumens 22 through which the pair of traction wires 50 are inserted constitute a set of through holes 11, and a plurality of sets of through holes are formed. The through hole sets 11 are arranged at different positions in the circumferential direction, and a plurality of proximity regulation parts 45b and arrangement recesses 44 are provided on the base end side flange 43b, and the separation regulation parts 45a and 44 are provided on the distal end side flange 43a. Since a plurality of arrangement recesses 44 are provided, the distal end portion of the movable elongated structure 10 may be bent in a bending direction corresponding to the number of sets.

More specifically, a plurality of through hole sets 11 are arranged at different positions in the circumferential direction, a plurality of proximity regulation parts 45b and a plurality of arrangement recesses 44 are provided in the base-side flange 43b, and a plurality of recesses 44 are provided in the distal end side flange 43a. By providing a plurality of separation regulation parts 45a and arrangement recesses 44, at least a pair of the traction wires 50 out of the plurality of traction wires 50 inserted through the wire lumens 22, 32 may be pulled. The distal end portion of the movable elongated structure 10 may be bent in a bending direction corresponding to the traction wires 50.

In addition, in the base end side flange 43b, the common proximity regulation part 45b regulates the circumferentially adjacent traction wires 50 of the pair of traction wires 50 inserted through the through hole sets 11 that are adjacent in the circumferential direction from approaching each other, and the circumferentially adjacent traction wires 50 of the pair of traction wires 50 inserted through the through hole sets 11 adjacent in the circumferential direction are separated from each other at the distal end side flange 43a. Since the separation regulation part 45a is common to the traction wire 50, the directions of the plurality of pairs of traction wires 50 may be regulated by the spacer 40 having a simple structure.

More specifically, in the base end side flange 43b, the common proximity regulation portion regulates the circumferentially adjacent traction wires 50 of the pair of traction wires 50 inserted through the circumferentially adjacent through hole sets 11 from approaching each other. 45b, that is, the traction wire 50 inserted through the through hole sets 11 adjacent in the circumferential direction may also be served as the proximity regulation portion 45b, the proximity of different pairs of traction wires 50 adjacent in the circumferential direction may be restricted.

In addition, in the distal end side flange 43a, the separation of the traction wires 50 that are adjacent in the circumferential direction among the pair of traction wires 50 that are inserted through the through hole sets 11 that are adjacent in the circumferential direction may be restricted by the separation regulation part 45a. In other words, since the separation regulation portion 45a may be shared by the traction wires 50 inserted through the through hole sets 11 adjacent to each other on the opposite side in the circumferential direction, the structure is simpler than the case where the separation regulation portions 45a are provided in the respective traction wires 50, the separation of different pairs of traction wires 50 adjacent in the circumferential direction may be regulated.

As described above, the proximity regulation part 45b and the separation regulation part 45a function as the regulation parts 45 for different pairs of traction wires 50 that are adjacent in the circumferential direction, so that the construction may be made simple. It should be noted that a simpler structure may be achieved by arranging different pairs of traction wires 50 that are adjacent in the circumferential direction in the arrangement recesses 44 in the base end side flange 43b and the distal end side flange 43a.

For a specific example, as shown in FIGS. 3A and 3B, a through hole set 11a constituted by a distal wire lumen 23a through which a traction portion 53a is inserted and a base end side wire lumen 36b, and a distal wire through which a traction portion 56b is inserted. A through hole set 11h composed of the lumen 26b and the base end side wire lumen 33a will be described below.

Common proximity regulation part that the circumferentially adjacent traction portions 53a and 56b of the pair of traction portions 53 and 56 inserted through the circumferentially adjacent through hole sets 11a and 11h of the base end side flange 43b are close to each other which may be regulated by the regulation part 45b.

That is, the circumferentially adjacent towed portions 53a and 56b of the pair of towed portions 53 and 56 inserted through the circumferentially adjacent through hole sets 11a and 11h may also serve as the proximity regulation part 45b.

Therefore, compared with the case where the proximity regulation part 45b is provided in each of the towed portions 53a and 56b, the towed portions 53a and 56b of the towed portion 53 and the towed portion 56 of the through hole sets 11a and 11h adjacent in the circumferential direction are simpler in structure, so that proximity may be regulated.

In addition, in the distal end side flange portion 43a, the right side WR of the arrangement recess 44 in the upward direction HU indicates that the traction portions 53a and 53b of the pair of traction portions 53 that are inserted through the through hole sets 11a and 11b that are adjacent in the circumferential direction are separated from each other. and the separation regulation portion 45a on the left side WL.

In addition, in the distal end side flange 43a, the upward direction HU of the arrangement recess 44 of the right side WR indicates that the traction portions 55a and 55b of the pair of traction portions 55 inserted through the through hole sets 11e and 11f that are adjacent in the circumferential direction are separated from each other, and the separation regulation portion 45a in the downward direction HD.

That is, this may be used by the traction portions 53b and 55a adjacent in the circumferential direction among the pair of traction portions 53 and 55 which are inserted through the through-hole sets 11b, 11e adjacent in the circumferential direction through the separation regulation part 45a between the arrangement recess 44 in the upward direction HU and the arrangement recess 44 in the right side WR.

Therefore, compared with the case of providing the separation regulation part 45a in each of the traction portions 53b, 55a, the traction portions 53b, 55a of the traction portion 53 and the traction portion 55 of the through hole sets 11a, 11h adjacent in the circumferential direction are simpler in structure to be regulated.

In addition, one traction wire 50 and the other traction wire 50 inserted through the through hole sets 11 adjacent in the circumferential direction intersect in the circumferential direction, and the one traction wire 50 is inserted into the cylinder 42 to the outside of the other traction wire 50. Since the guide convex portion 46 is provided to guide the traction wires 50 to each other, the predetermined traction wire 50 is pulled smoothly without interfering with each other, and the distal end portion of the movable elongated structure 10 is able to be bent.

More specifically, one traction wire 50 and the other traction wire 50 are inserted through the through hole sets 11 that intersect in the circumferential direction and are adjacent to each other in the circumferential direction. Since the guiding protrusion 46 is provided on the cylinder 42, it is possible to regulate one traction wire 50 from overpassing the other traction wire 50 and causing interference. Therefore, the predetermined traction wire 50 may be smoothly pulled without the plurality of pairs of traction wires 50 interfering with each other, and the distal end portion of the movable elongated structure 10 may be bent.

Also, the traction wire 50 is a flexible wire, and the flexible tubes 20, 30 are composed of flexible tubes having main lumens 21, 31, and the wire lumens 22, 32 are formed inside the wall of tubes. Since there is a wire lumen formed inside the wall through which a wire may be inserted, a movable elongated structure composed of a tube having main lumens 21 and 31 is pulled by pulling a traction wire 50 composed of a flexible wire. At least the distal end portion of the body 10 may be bent, and a predetermined treatment may be performed by inserting an instrument or medicine through the main lumens 21 and 31 of the movable elongated structure 10 that may bend at least the distal end portion.

Since the pair of pulling wires 50 are formed by bending back the wires at the distal end side LF of the distal end side flexible tube 20, the pair of pulling wires 50 may be formed from one wire, thus a movable elongated structure 10 having a simple structure may be constructed.

In addition, even if the duct is at least one of hollow organs, vessels, and blood vessels that have a narrow and complicated branch structure, it may be easily inserted to a predetermined position. Specifically, a two-way movable bendable nephoscope is used clinically. As shown in FIGS. 9A and 9B, the kidney has a three-dimensional shape, and it has been found that there are parts that may not be reached by bidirectional bending. For example, in the three-dimensional shape of the upper small renal cup of the kidney, various inner surfaces may be accessed through a combination of bidirectional bending and axial rotation, but in the middle and lower small renal cups, some inner surfaces are inaccessible. On the other hand, as shown in FIG. 9A, a movable elongated structure 10 that may move in four directions (omnidirectionally) has two vertical directions (two directions on the drawing of FIG. 9A) and two horizontal directions (It may be bent in all directions by two directions on the drawing of FIG. 9B) and combinations thereof.

In addition, since the plurality of traction wires 50 are routed crosswise using the spacer 40, even if the base end side flexible tube 30 is bent along the path as shown in FIGS. 9A and 9B. Deterioration of the movable bending performance of the tube 20 is small. Therefore, all inner surfaces of the upper, middle and lower calyces are freely accessible.

In this way, since the movable elongated structure 10 is movable in four directions (all directions), it is possible to make the distal end reach a desired position in three-dimensional tubular organs such as kidneys, vessels, and blood vessels.

The movable elongated structure 10 described above is provided with four traction wires 50 (53, 54, 55, 56) and configured to bend the distal end side flexible tube 20 in a desired direction. The configuration is not limited, and the traction wire 50 may be arranged according to the bending direction.

Specifically, as shown in FIGS. 10A and 10B, for example, in the case of a movable elongated structure 10a that bends the distal end-side flexible tube 20 in one upward direction HU, only traction portions 53 of the above movable elongated structure 10 are provided, and the base end side flange portion 43b is provided with placement recesses 44a in which only one traction portion 53a, 53b may be placed on the right side WR and the left side WL.

Then, the traction portion 53 is inserted into the distal wire lumen 23 (23a, 23b) of the distal end side flexible tube 20, and the traction portion 53 is arranged in the arrangement recess 44 in the upward direction HU of the distal end side flange 43a, The traction portions 53a and 53b are respectively arranged in the arrangement recesses 44a of the base end side flange 43b.

Also, the traction portion 53 is inserted through the base end side wire lumen 35 and the base end side wire lumen 36 of the base end side flexible tube 30. The distal wire lumen 22 other than the distal wire lumen 23 in the distal end side flexible tube 20 and the base end side wire lumen 32 other than the base end side wire lumens 35 and 36 in the base end side flexible tube 30 may be provided or may not be provided.

In the movable elongated structure 10a configured in this manner, the distal wire lumen 23 is closer in the circumferential direction than the base end side wire lumens 35 and 36 through which the traction portion 53 is inserted into the wire lumen 36 in the upward direction HU, the traction portion 53 is inserted through the wire lumen 23 on the distal end side HU may be bent in the upward direction HU.

Further, as shown in FIGS. 10C and10D, for example, in the case of a movable elongated structure 10b that bends the distal end side flexible tube 20 in two directions, the upward direction HU and the downward direction HD, the above movable elongated structure A traction portion 53 and a traction portion 54 of the body 10 are provided.

Then, the traction portion 53 is inserted through the distal wire lumen 23 of the distal end side flexible tube 20, and the traction portion 53 is disposed in the arrangement recess 44 in the upward direction HU of the distal end side flange 43a. The traction portions 53a and 53b are arranged in the upward direction HU in the recesses 44 arranged in the width direction W of the portion 43b.

Also, the traction portion 53 is inserted through the base end side wire lumen 35a and the base end side wire lumen 36b of the base end side flexible tube 30.

Furthermore, the pulling part 54 is inserted through the distal wire lumen 24 of the distal end side flexible tube 20, and the pulling part 54 is arranged in the arrangement recess 44 in the downward direction HD of the base end side wire lumen 34a. Traction portions 54a and 54b are arranged downward HD in each recess 44 arranged in the width direction W of the lumen 34b. Then, the traction portion 54 is inserted through the base end side wire lumen 35 b and the base end side wire lumen 36 a of the base end side flexible tube 30.

In the movable elongated structure 10b configured in this way, as shown in FIGS.10C and 10D, the distal wire lumen 24 is positioned in the circumferential direction from the base end side wire lumens 35a and 36b through which the traction portion 53 is inserted. The traction portion 53 is inserted through the distal end side wire lumen 23 which is narrowly spaced and arranged in the upward direction HU from the base end side wire lumens 35a and 36b, and the base end side wire lumens 35b and 36a through which the traction portion 54 is inserted is extended in the circumferential direction is narrow, and the traction portion 54 is inserted through the distal end side wire lumen 24 arranged in the downward direction HD from the base end side wire lumens 35b and 36a. As shown in FIG.10C, the distal end side flexible tube 20 may be bent upward HU, and when the traction portion 53 is pulled toward base end side LB, the distal end side flexible tube 20 may be bent downward HD.

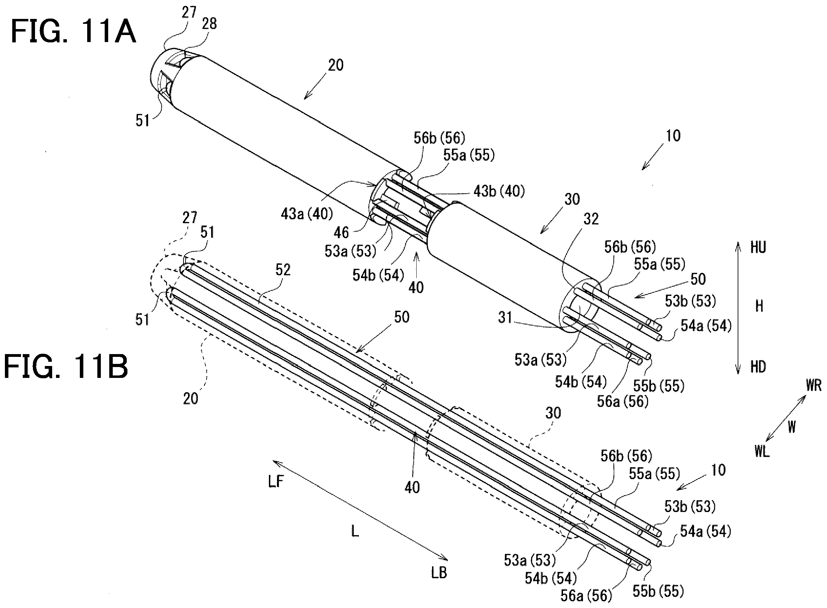

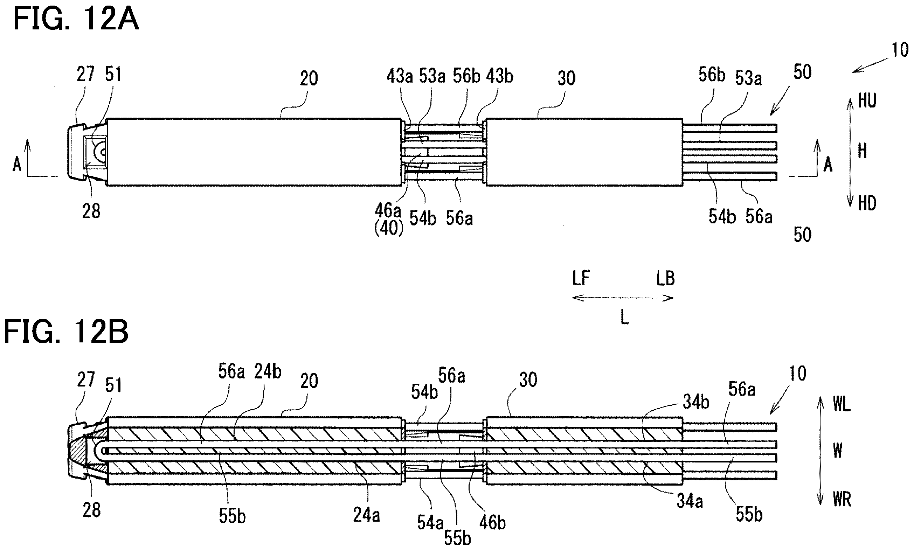

Regarding the movable elongated structure 10 using the spacer 40, in the above description, the traction wire 50 is obliquely extended in the spacer 40 and inserted through the wire lumens 23, 33. However, as shown in FIGS. 11 and 12, the traction wire 50may be arranged straight in the spacer 40.

FIGS. 11A and 11B show explanatory views of the movable elongated structure 10 with another arrangement pattern. FIG.11A shows a perspective view of the movable elongated structure 10 in another arrangement pattern, and FIG. 11B shows the movable elongated structure showing the distal end side flexible tube 20 and the base end side flexible tube 30 in a see-through state.

FIGS. 12A and 12B show explanatory views of a movable elongated structure 10 with another arrangement pattern. FIG. 12A shows a front view of the movable elongated structure 10, and FIG. 12B shows a cross-sectional view taken along line A-A in FIG.12A.

The movable elongated structure 10 with an arrangement pattern is a distal end side wire in which the traction portions 52 (53 to 56) of the traction wire 50, which are bent back near the center of the length to form a bent portion 51, are arranged in four directions. It is inserted along the longitudinal direction L through the lumen 22, the placement recess 44, and the base end side wire lumen 32.

For example, the traction portion 53a of the traction portion 53 from the distal end side LF is inserted into the distal end side wire lumen 23a of the left side WL in the distal end side wire lumen 23, the traction portion 53b is inserted through the distal end side wire lumen 23b of the right side WR. The traction portion 53 is led out from the base end side LB of the side wire lumen 23, and the bending portion 51 is arranged in the bending concave portion 28 in the upward direction HU. The other traction sections 54, 55, 56 are routed similarly.

The traction portion 53 led out from the base end side LB of the distal end side wire lumen 23 is arranged in the upward direction HU of the distal end side flange 43a and the base end side flange 43b, and is inserted through the base end side wire lumen 33 of the upward direction HU of the base end side flexible tube 30 and led out from the base end side LB.

In this way, the movable elongated structure 10 in which the straight traction portion 52 is inserted through the wire lumens 23 and 33 along the longitudinal direction L, although the effect of suppressing the bending effect that occurs in the proximal-side flexible tube 30 when the pulling wire 50 is pulled is reduced in comparison with the movable elongated structure 10 in which the traction wire 50 is arranged by extending it in the diagonal direction in the spacer 40 as described above, may bend the distal end side flexible tube 20 in a desired direction with respect to the proximal flexible tube 30, and pull all four sets of pulling wires 50. Alternatively, by pulling two pairs of pulling wires 50 that face each other in the radial direction, the movable elongated structure 10 may be shortened (compressed) without being bent.

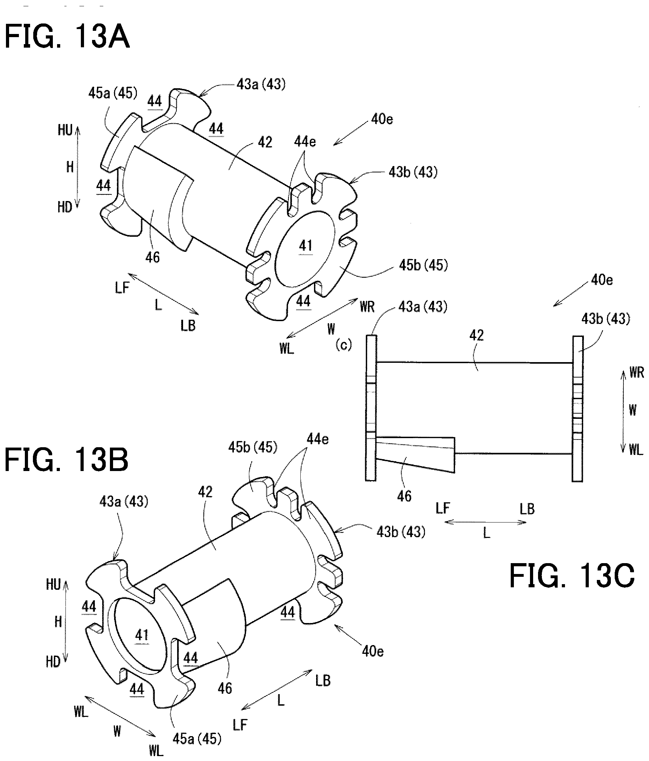

Further, the spacer 40 in the movable elongated structural body 10 described above is provided with the guide convex portion 46 on each of the distal end side LF and the base end side LB, but as shown in FIGS. 13A to 13C, only one side of the longitudinal direction L may be provided with the guiding convex portion 46.

Note that FIGS. 13A to 13C show explanatory diagrams of the spacer 40e. FIG. 13A shows a front, right side, and top perspective view of spacer 40e, FIG. 13B shows a front, left side, and top perspective view of spacer 40e, and FIG. 13C shows a top view of spacer 40e.

As shown in FIGS. 13A to 13C, the spacer 40e has a guide convex part 46 only on the distal end side LF. Further, in the spacer 40 described above, the guide convex parts 46 are provided so as to face each other in the circumferential direction.

In addition, among the arrangement recesses 44 of the base end side flange 43b in which the guide convex part 46 is not provided, the arrangement recesses 44e arranged in the upward direction HU, the right side WR and the left side WL are arranged with two traction wires 50, respectively, and are formed in a substantially W shape with an open radial outer side when viewed from the longitudinal direction L. That is, the placement recess 44e is provided corresponding to each traction wire 50, and the side walls of the placement recess 44 e function as the regulation parts 45a and 45b for each traction wire 50.

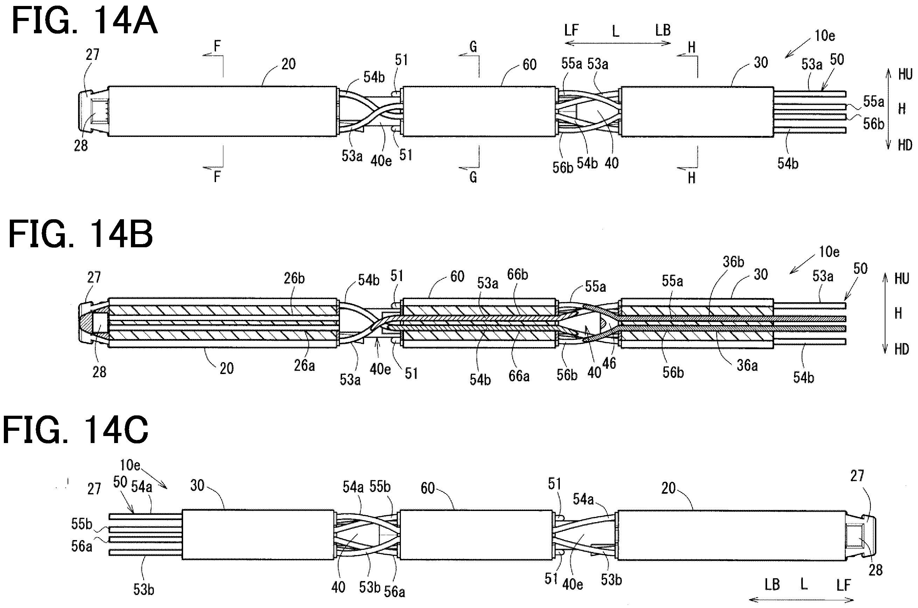

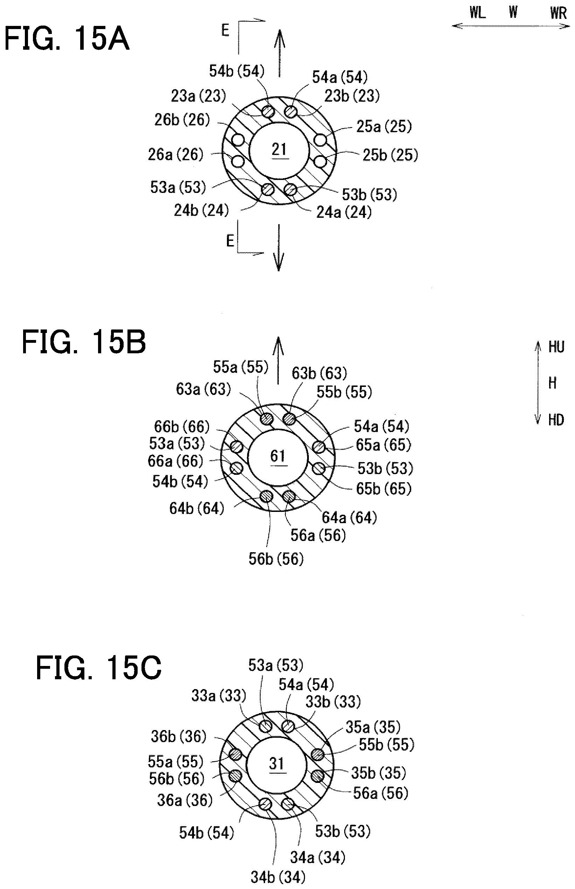

A movable elongated structure 10e using the spacer 40e configured in this way will be described with reference to FIGS.14 and 15.

FIGS.14 and 15 are explanatory diagrams of the movable elongated structure 10e. FIG. 14A shows a front view of the movable elongated structure 10e, FIG. 14B shows a cross-sectional view of FIG. 15A along line E-E, and FIG. 14C shows a rear view of the movable elongated structure 10e, FIG. 15A shows a cross-sectional view along the F-F arrow in FIG. 14A, FIG. 15B shows a cross-sectional view along the G-G arrow in FIG. 14A, and FIG. 15C shows a cross-sectional view along the H-H arrow in FIG. 14A.

The movable elongated structure 10e shown in FIGS. 14 and 15 differs from the movable elongated structure 10 in which the spacer 40 is arranged between the distal end side flexible tube 20 and the base end side flexible tube 30, an intermediate tube 60 is arranged between the distal end side flexible tube 20 and the base end side flexible tube 30, a spacer 40 is arranged between the base end side flexible tube 30 and the intermediate tube 60, and a distal end side flexible tube is arranged. Between the flexible tube 20 and the intermediate tube 60, the spacer 40e described above is arranged.

The intermediate tube 60 is a cylindrical flexible tube elongated in the longitudinal direction L, similarly to the distal end side flexible tube 20 and the base end side flexible tube 30, and has a main lumen 61 therein. Also, the intermediate tube 60 is provided with two intermediate wire lumens 63, 64, 65, 66 in each of the four directions of the upward direction HU, the downward direction HD, the right side WR and the left side WL inside the tube wall.

The intermediate tube 60 is a flexible tube made of polyamide elastomer, expanded polytetrafluoroethylene, polyurethane, or polytetrafluoroethylene, like the distal end side flexible tube 20 and the base end side flexible tube 30 may be configured. The intermediate tube 60 may be made of the same material as the distal end side flexible tube 20 and the base end side flexible tube 30, or may be made of different materials.

The traction portion 54b that passes through the left WL distal wire lumen 23a of the distal wire lumen 23 in the upward direction HU of the distal end side flexible tube 20 inserts the intermediate wire lumen 66a in the downward direction HD of the intermediate wire lumen 66 provided on the left side WL of the intermediate tube 60, and the base end side wire lumen 34b on the left side WL of the base end side wire lumen 34 provided in the downward direction HD in the base end side flexible tube 30.

Then, the traction portion 54a passing through the right WR distal end side wire lumen 23b in the upward direction HU distal end side wire lumen 23 in the distal end side flexible tube 20 inserts the intermediate wire lumen 65a in the upward direction HU of the intermediate wire lumen 65 provided on the right side WR of the intermediate tube 60, and the base end side wire lumen 33b on the right side WR of the base end side wire lumen 33 provided in the upward direction HU in the base end side flexible tube 30.

In addition, the traction portion 53b passing through the right WR distal end side wire lumen 23b in the downward direction HD distal end side wire lumen 24 in the distal end side flexible tube 20 inserts the intermediate wire lumen 65b in the downward direction HD of the intermediate wire lumen 65 provided on the right side WR of the intermediate tube 60, and the base end side wire lumen 33b on the right side WR of the base end side wire lumen 34 provided in the downward direction HD in the base end side flexible tube 30.

The traction portion 53a that passes through which the left WL distal wire lumen 24b of the downward HD distal wire lumen 24 of the distal end side flexible tube 20 inserts the intermediate wire lumen 66b in the upward direction HU of the intermediate wire lumen 66 provided on the left side WL of the intermediate tube 60, and is inserted into the proximal wire lumen 33a on the left side WL of the base end side wire lumen 34 provided in the base end side flexible tube 30 in the upward direction HU.

On the other hand, the traction portions 55 and 56 are not inserted into the distal wire lumens 25 and 26 in the width direction W of the distal end side flexible tube 20, and the left side of the intermediate wire lumen 63 provided in the upward direction HU in the intermediate tube 60. The traction portion 55a is inserted through the intermediate wire lumen 63a of the WL, and is inserted through the upward HU base end side wire lumen 36b of the base end side wire lumen 36 provided on the left WL of the base end side flexible tube 30.

Then, the traction portion 55b is inserted into the intermediate wire lumen 63b of the right WR of the intermediate wire lumen 63 provided in the upward direction HU in the intermediate tube 60, and into the base end side wire lumen 35a in the upward direction HU provided in the right WR of the base end side flexible tube 30.

In addition, the traction portion 56a is inserted into the intermediate wire lumen 64a of the right WR of the intermediate wire lumen 64 provided in the downward direction HD of the intermediate tube 60, and the base end side wire lumen 35b in the downward direction HD of the base end side wire lumen 35 provided on the right side WR of the base end side flexible tube 30.

Then, the traction portion 56b is inserted into the intermediate wire lumen 64b on the left side WL of the intermediate wire lumen 64 provided in the downward HD of the intermediate tube 60, and the base end side wire lumen 36a in the downward direction HD of the base end side wire lumen 36 provided on the left side WL of the base end side flexible tube 30.

The traction portions 55 and 56 are not inserted through the distal wire lumen 23 of the distal end side flexible tube 20 but are inserted through the intermediate wire lumen 63 of the intermediate tube 60 and the base end side wire lumen 33 of the base end side flexible tube 30, a bent portion 51 is formed at the arrangement recess 44e of the flange 43 of the spacer 40e arranged between the distal end side flexible tube 20 and the intermediate tube 60, and is bent back.

In addition, in the spacer 40e arranged between the distal end side flexible tube 20 and the intermediate tube 60 in the movable elongated structure 10e, the traction portion 53a and the traction portion 54b intersect on the front side shown in FIG. 14A, but on the back side shown in FIG. 14C, the traction portion 53b and the traction portion 54a are routed so as not to cross each other.

In the movable elongated structure 10e configured in this manner, the traction portion 53 and the traction portion 54 are inserted through the distal end side flexible tube 20, the intermediate tube 60, and the base end side flexible tube 30, and the traction portion 55 and the traction portion 56 are inserted through the intermediate tube 60 and base end side flexible tube 30.

Therefore, the spacer 40 positioned between the base end side flexible tube 30 and the intermediate tube 60 has the four traction sections 53, 54, 55, and 56 positioned thereon, while the distal end side flexible tube 20 and the intermediate tube 60 are spaced apart. Two traction portions 53 and 54 are arranged on the spacer 40e arranged between the tube 60 and the spacer 40e.

Therefore, a spacer 40e is used in which there are few intersections between the traction wires 50 and the guide convex part 46 are provided only on the distal end side LF.

As described above, traction portion 53 and traction portion 54 extend through distal end side flexible tube 20, intermediate tube 60 and base end side flexible tube 30, and traction portion 55 and traction portion 56 extend through intermediate tube 60 and base end tube 60, respectively. The movable elongated structure 10e that is inserted through the flexible tube 30 is based on one of the traction portion 53 and the traction portion 54 that are inserted through the distal end side flexible tube 20, the intermediate tube 60 and the base end side flexible tube 30. The distal end side flexible tube 20 may be bent upward HU or downward HD with respect to the intermediate tube 60 by pulling toward the end LB.

On the other hand, by pulling either the traction portion 55 or the traction portion 56 inserted through the intermediate tube 60 and the base end side flexible tube 30 to the base end side LB, the intermediate tube 60 may be bent either upward HU or downward HD direction relative to proximal flexible tube 30. At this time, if the traction portion 53 and the traction portion 54 are not pulled, the distal end side flexible tube 20 does not bend with respect to the intermediate tube 60 and bends following the intermediate tube 60 that bends upward HU or downward HD with respect to the proximal side flexible tube 30. That is, the movable elongated structure 10e may be bent with the distal end side flexible tube 20 and the intermediate tube 60 substantially not interfering with each other.

As described above, an intermediate tube 60 having an elongated intermediate wire lumen 63, 64, 65, and 66 is disposed between the distal end side flexible tube 20 and the base end side flexible tube 30, and a plurality of a pair of traction wires 50 are provided, and at least one pair of traction wires 50 out of the plurality of pairs of traction wires 50 are inserted through the distal wire lumen 22, the intermediate wire lumens 63, 64, 65, and 66 and the base end side wire lumen 32. At least one pair of traction wires 50 out of the plurality of pairs of traction wires 50 is inserted through the intermediate wire lumens 63, 64, 65, and 66 and the base end side wire lumen 32. Therefore, it is possible to configure the movable elongated structure 10e that may bend the intermediate tube 60 and the distal end side flexible tube 20, respectively.

Specifically, among the plurality of pairs of traction wires 50, the traction wires 55 and 56 are inserted through the intermediate wire lumens 63 and 64 and the base end side wire lumens 35 and 36, and the other traction wire 53 of the plurality of pairs of traction wires 50 is inserted, traction wires 53 and 54 are inserted through the distal wire lumens 23 and 24, the intermediate wire lumens 65 and 66, and the base end side wire lumens 33 and 34, the intermediate wire lumens 63, 64 and the base end side wire lumens 35, 36 are by pulling the inserted traction wires 55 and 56, the intermediate tube 60 in the intermediate portion of the movable elongated structure 10e may be bent, and the distal end side wire lumens 23 and 24, the intermediate wire lumens 65 and 66, and the base end side wire lumens 23 and 24 may be bent. By pulling the traction wires 53, 54 inserted through the wire lumens 33, 34, the distal end side flexible tube 20, which is the distal end portion of the movable elongated structure 10e, may be bent.

In this way, with the above-described configuration, the intermediate tube 60 at the intermediate portion of the movable elongated structure 10e and the distal end side flexible tube 20, which is the distal end portion of the movable elongated structure 10e, may be bent without substantial interference with each other.

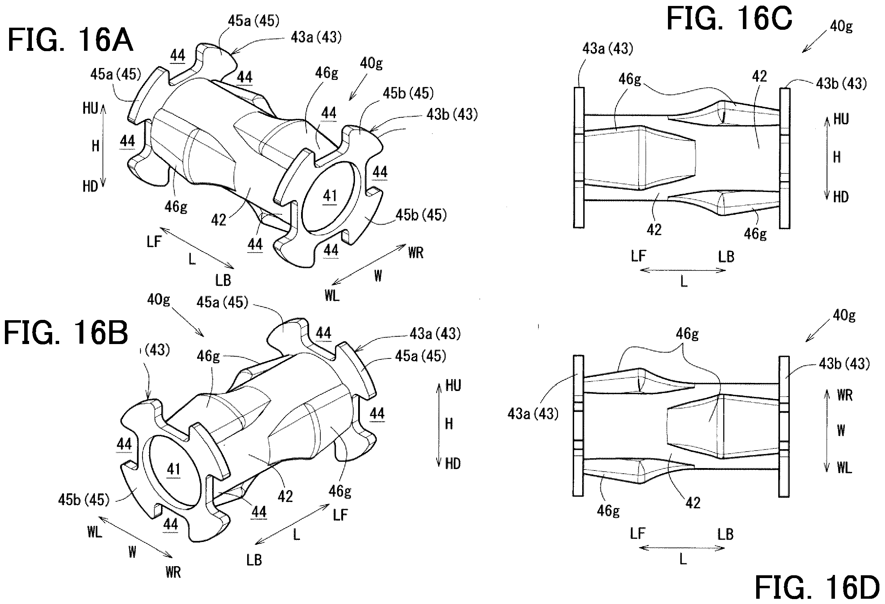

The guide convex part 46 provided on the spacers 40 and 40e described above are formed in a trapezoidal vertical cross section that gradually protrudes from the ends in the longitudinal direction L toward the center, but a guide convex part 46g may be formed in a smooth shape as shown in FIGS. 16A to 16D.

FIGS. 16A to 16D show explanatory diagrams of a spacer 40g having a guide convex part 46g. FIG. 16A shows a front, right side, and top perspective view of spacer 40g, FIG. 16B shows a rear, right side, and top perspective view of spacer 40g, FIG. 16C shows a front view of spacer 40g, and FIG. 16D shows a plan view of the spacer 40g.

The guide convex part 46g provided on the spacer 40g has a height that gradually increases from the end in the longitudinal direction L toward the center in the longitudinal direction L, and the width thereof also gradually widens. Then, it is formed in a substantially hexagonal shape in a front view in which the height gradually decreases from the widest point and the width gradually narrows.

The guide convex part 46g formed in the shape described above has a gentler corner than the guide convex part 46 provided on the spacer 40 or the spacer 40e, and stress concentration on the pulling wire 50 straddling the guiding projection 46 may be reduced.

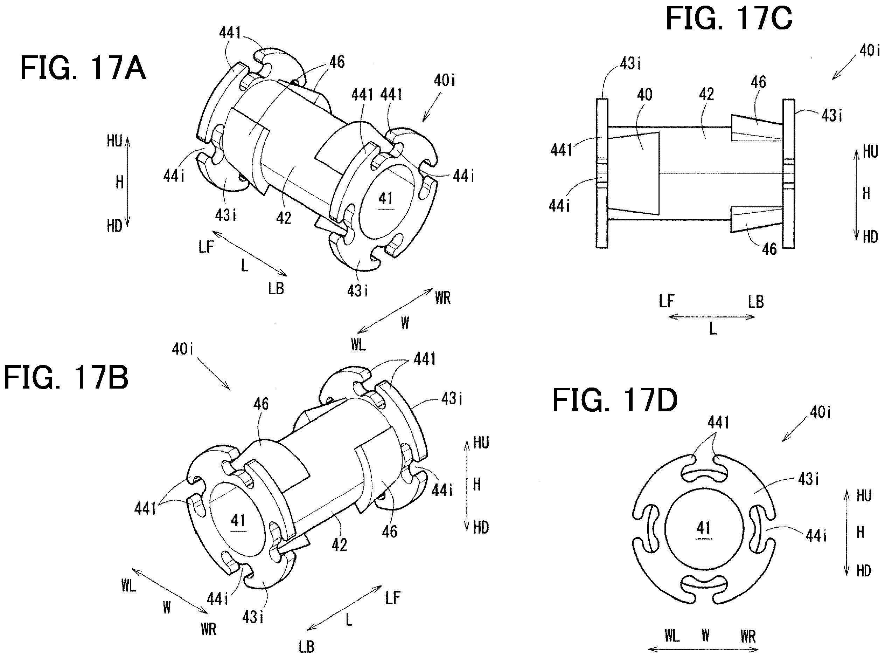

Further, the above-described spacer 40 is provided with a recessed placement recess 44 whose radial outer side is open in the flange portion 43. However, a spacer 40i having a placement recess 44i having a compression support portion 441 may be employed as shown in FIGS. 17A to 17D.

FIGS. 17A to 17D show illustrations of a spacer 40i having compression support portions 441 in the placement recesses 44i. FIG. 17A shows a front, right side and top perspective view of spacer 40i, FIG. 17B shows a back, right side and top perspective view of spacer 40i, FIG. 17C shows a front view of spacer 40i, and FIG. 17D indicates the side surface of the spacer 40i.

The flange 43i of the spacer 40i is provided with arrangement recesses 44i in four directions at equal intervals in the circumferential direction, and a compression support portion 441 is arranged in each arrangement recess 44i.

The arrangement recess 44i has circular arc recesses in which the traction wire 50 is arranged, which are arranged at predetermined intervals in the circumferential direction.

The compression support portions 441 are formed to protrude in the circumferential direction from both sides of the arrangement recess 44i on the radially outer side of the approximately bean-shaped arrangement recess 44i as described above, with a gap that allows the traction wire 50 to pass through in the radial direction.

The moveable elongated structure 10 in which the spacer 40i having the arrangement recess 44i configured in this manner is arranged between the distal end side flexible tube 20 and the base end side flexible tube 30 is configured such that the spacer 40 is arranged as described above. The same effect as the arranged movable type elongated structure 10 may be obtained, and the following effect may be obtained.

When the traction wire 50 is pulled to bend the flexible tubes 20,30, a compressive force is applied to the flexible tubes 20,30. The compressive force also acts on the spacer 40i arranged between the flexible tubes 20,30.

If the radially outer open portion of the arrangement recess 44i in the spacer 40i becomes large, the flexible tubes 20 and 30 to which the compressive force is applied may bite into the radially outer open portion of the arrangement recess 44i, regulating desired bending deformation. On the other hand, since the spacer 40i is provided with the compression support portion 441 that protrudes radially outward from the arrangement recess 44i, the compression force described above may be supported, and the desired bending deformation may be achieved.

In addition, the spacer 40i functions as a pull-out regulation part in which the compression support portion 441 regulates the traction wire 50 from slipping out of the placement recess 44i. whereby the traction wire 50 arranged in the arrangement recess 44i may be prevented from moving radially outward from the arrangement recess 44i by the compression support part 441 and unintentionally coming off.

In the spacer 40i having the arrangement recesses 44i with the compression support portions 441, although not shown, the arrangement recesses 44i may be provided at three locations in the circumferential direction of the flange portion 43i.

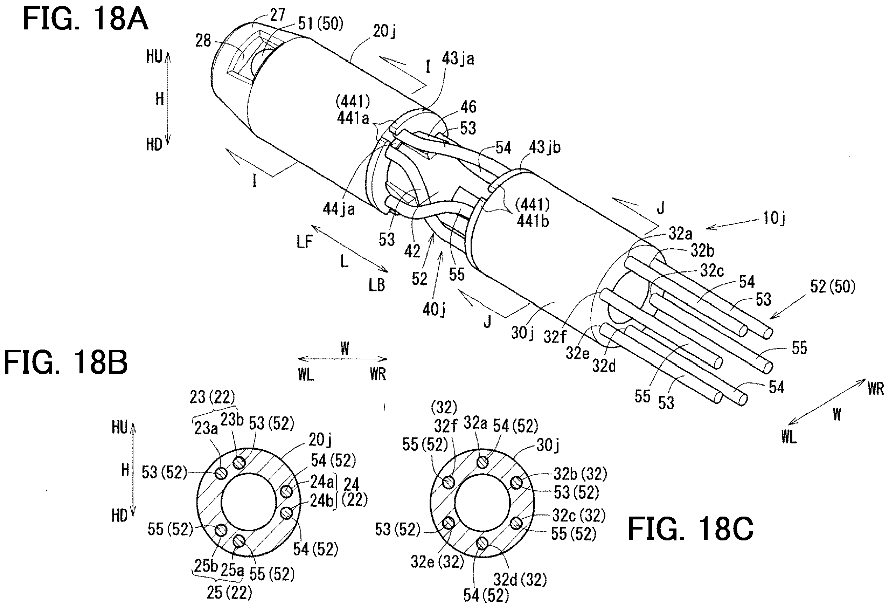

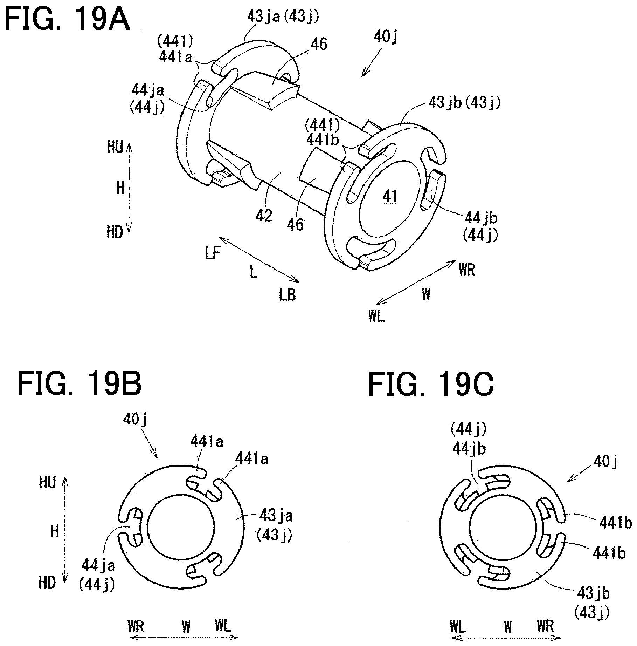

Specifically, as shown in FIGS. 18 and 19, a movable elongated structure 10j using a spacer 40j having three arrangement recesses 44j provided on a flange 43j will be described with reference to FIGS.18 and 19.

FIGS.18 and 19 are explanatory diagrams of the movable elongated structure 10j and the spacer 40j. FIG.18A shows a perspective view of the movable elongated structure 10j, FIG. 18B shows a cross-sectional view taken along line I-I in FIG. 18A, FIG. 18C shows a cross-sectional view taken along arrow J-J in FIG. 18A, and FIG. 19A shows a perspective view of the spacer 40j, FIG. 19B shows a side view of the spacer 40j viewed from the distal end side LF, and FIG. 19C shows a side view of the spacer 40j viewed from the base end side LB.

Similar to the movable elongated structure 10 described above, the movable elongated structure 10j shown in FIGS. 18 and 19, the spacer 40j and the base end side flexible tube 30j are arranged, and the traction wire 50 is routed. In addition, the same reference numerals are given to the same configurations as those of the above-described movable elongated structure 10 and the like, and the description thereof will be omitted.

The spacer 40j used in the movable elongated structure 10j has three bean-shaped placement recesses 44j similar to the placement recesses 44i of the spacer 40i shown in FIGS. 17A to 17D. A support portion 441 is provided to ethe ach of placement recesses 44i. Therefore, three traction wires 50 (53, 54, 55) are routed in the movable elongated structure 10j.

However, in the arrangement recess 44i described above, the protrusion 43i on the distal end side LF and the protrusion 43i on the base end side LB are formed in the same shape, but the placement recess 44j has the distal end side flange on the distal end side LF. The placement recess 44ja and the compression support portion 441a provided in the portion 43ja are different in shape from the placement recess 44jb and the compression support portion 441b provided in the base end side flange portion 43jb of the base end side LB.

More specifically, although the circumferential position of the arrangement recess 44ja in the distal end side flange 43ja coincides with the circumferential position of the arrangement recess 44jb in the base end side flange 43jb, the base end side flange 43jb is located at the same position. The arrangement recess 44jb provided is formed to be longer in the circumferential direction than the arrangement recess 44ja provided in the distal end side flange 43ja. Therefore, the compression support portion 441b is also formed to protrude longer than the compression support portion 441a.

Specifically, while the arrangement recess 44ja provided in the distal end side flange 43ja has the length of the two traction wires 50 arranged at an appropriate interval, the arrangement recess 44jb provided in the base end side flange 43jb is formed such that the distance between the traction wires 50 arranged on both sides of the arrangement recess 44jb is formed to have the same length in the circumferential direction as the distance between the traction wires 50 arranged in the adjacent arrangement recess 44jb. Therefore, the traction portions 52 of the traction wires 50 arranged in the arrangement recesses 44jb arranged in three directions in the circumferential direction are arranged at intervals of 60 degrees in the cross-sectional circumferential direction.

The distal end side wire lumen 22 of the distal end side flexible tube 20j and the base end side wire lumen 32 of the base end side flexible tube 30j that constitute the movable elongated structure 10j using the spacer 40j configured in this way are different arrangement. Specifically, as shown in FIG.18B, two distal wire lumens 22 (23, 24, 25) provided in the distal end side flexible tube 20j are arranged in three directions in the circumferential direction of the cross section.

Among the distal end side wire lumens 22 arranged in three directions, the upward direction HU is the distal end side wire lumen 23, the counterclockwise side of the two distal end side wire lumens 23 is the distal end side wire lumen 23a, and the clockwise side is the distal end side wire lumen 23b.

Similarly, the distal wire lumen 22 arranged on the right WR is referred to as a distal wire lumen 24, the counterclockwise side of the two distal wire lumens 24 is referred to as a distal wire lumen 24a, and the clockwise side is referred to as a distal wire lumen 24a. The distal end side wire lumen 22 arranged on the left side WL and downward HD is the distal end side wire lumen 25, while the counterclockwise side of the two distal end side wire lumens 25 is the distal end side wire lumen 25a and the clockwise side is the distal end side wire lumen 25b.

On the other hand, the base end side wire lumens 32 provided in the base end side flexible tube 30j are arranged in six directions at equal intervals in the circumferential direction of the cross section, as shown in FIG. 18C. Therefore, among the six base end side wire lumens 32, the base end side wire lumen 32 in the upward direction HU is designated as the base end side wire lumen 32a, and clockwise from the base end side wire lumen 32b to the base end side wire lumen 32f in order.

The movable elongated structure 10j using the spacer 40j constructed in this manner is assembled as follows:
A traction wire 50 having a bent portion 51 formed by bending from a bending recess 28 of a distal end cap 27 provided at the tip of the distal end side flexible tube 20j is attached, and a traction portion 52 is inserted through the distal end side wire lumen 22.

Then, the traction portion 52 led out to the base end side LB from the distal end side wire lumen 22 is arranged in the placement recess 44ja of the distal end side flange 43ja of the spacer 40j, and is extended obliquely to extend to the base end side flange 43jb is inserted into the base end side wire lumen 32 of the base end side flexible tube 30j, and led out from the base end side LB.

Specifically, the traction portion 53 is inserted from the distal end side LF into the distal end side wire lumen 23 (23a, 23b) of the distal end side flexible tube 20j, is led out from the base end side LB, and is pulled out from the distal end side flange 43ja to be arranged in the arrangement recess 44ja in the upward direction HU.

Then, the traction portion 53 is extended obliquely along the cylinder 42 toward the base end side LB and arranged in the arrangement recess 44jb in the base end side flange 43jb to be passed through base end side wire lumens 32e and 32b of the base end side flexible tube 30j from the distal side LF and led out from the base end side LB.

In addition, the traction portion 54 is inserted from the distal end side LF into the distal end side wire lumen 24 (24a, 24b) of the distal end side flexible tube 20j and is led out from the base end side LB to be placed in the placement recess 44ja of the right side WR in the distal end side flange 43ja.

Then, along the cylinder 42, the traction portion 54 is extended obliquely toward the base end side LB and placed in the placement recess 44jb of the base end side flange 43jb, and is inserted through the base end side wire lumen 32a and 32d of the base end side flexible tube 30j from the distal end side LF and led out from the base end side LB.

Similarly, the traction portion 55 is inserted through the distal wire lumen 25 (25a, 25b) of the distal end side flexible tube 20j from the distal end side LF, led out from the base end side LB, and is pulled out from the distal end side LB, and arranged in the placement recess 44ja in the downward direction HD the left side WL of the distal end side flange 43ja.

Then, along the cylinder 42, the traction portion 55 is extended obliquely toward the base end side LB and placed in the placement recess 44jb of the base end side flange 43jb, inserted through the base end side wire lumen 32c and 32f of the base end side flexible tube 30 from the distal end side LF, and led out from the base end side LB.

That is, in the base end side flexible tube 30j, the respective traction portions 52 are arranged in the base end side wire lumens 32 at opposing positions.

The movable elongated structure 10j configured in this way has the same effects as the movable elongated structure 10 described above, and pulls all of the three traction wires 50 (53, 54, 55). Thus, the movable elongated structure 10j may be shortened (compressed) without being bent.

Subsequently, a movable elongated structure 10k and spacers 40k of further different embodiments will be described below.

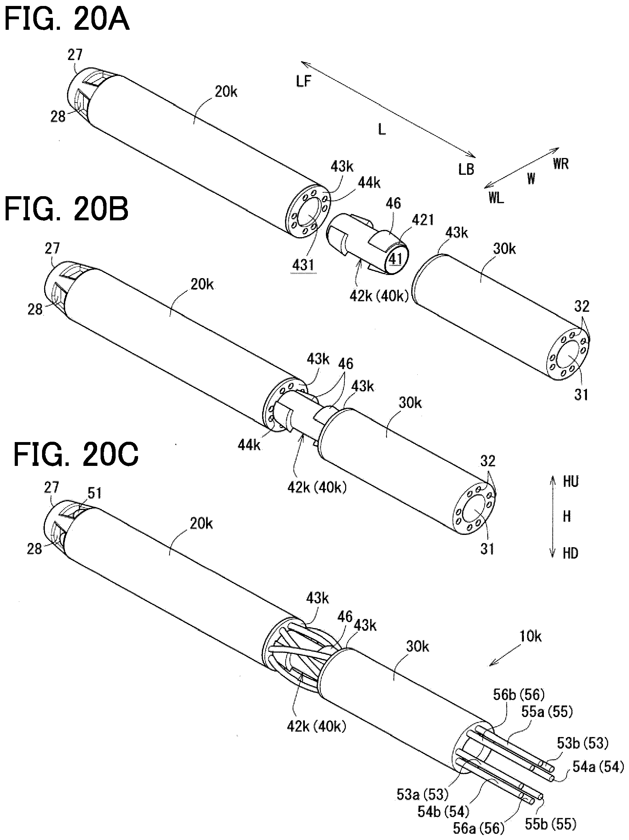

In the spacer 40 described above, the flanges 43 are provided on both sides in the longitudinal direction L of the cylinder 42 having the internal space 41, and the assembly spacer 40k may be configured only by the cylinder 42k as shown in FIGS. 20 to 22.

FIGS. 20A to 20C show explanatory views of the movable elongated structure 10k. FIG. 20A shows a perspective view of the movable elongated structure 10k before assembly, FIG. 20B shows a perspective view of the movable elongated structure 10k in an assembled state, and FIG. 20C shows a perspective view of a moveable elongated structure 10k.

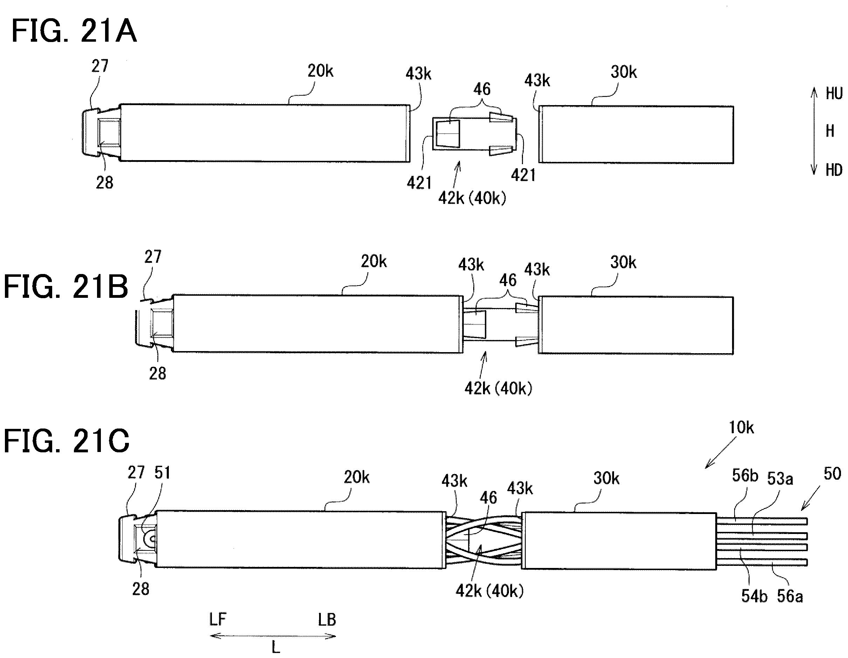

FIGS. 21A to 21C show explanatory views of a movable elongated structure 10k. FIG. 21A shows a front view of the movable elongated structure 10k before assembly, FIG. 21B shows a front view of the movable elongated structure 10k in an assembled state, and FIG. 21C shows a front view of the movable elongated structure 10k with the traction wire 50 routed.

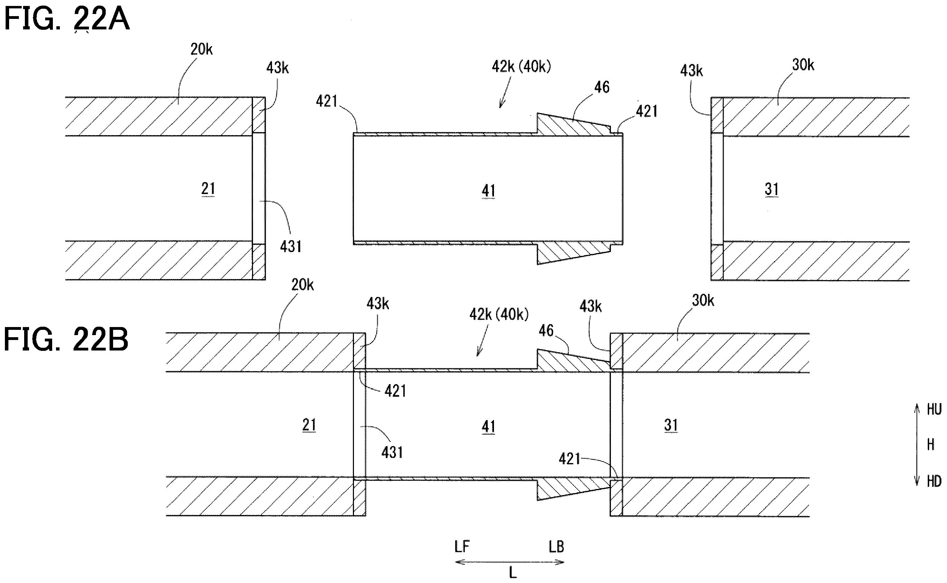

FIGS. 22A and 22B show explanatory views of the assembled spacer 40k. FIG. 22A shows an enlarged cross-sectional view of a portion including the assembly spacer 40k before assembly, and FIG. 22B shows an enlarged cross-sectional view of a portion including the assembly spacer 40k in an assembled state.

In the following description of the movable elongated structure 10k and the assembly spacer 40k, the same components as in the description of the movable elongated structure 10 and the spacer 40 are denoted by the same reference numerals, and the explanation thereof is omitted.

The assembly spacer 40k is composed of a cylindrical cylinder 42k having a guide convex part 46 at the same position as the guide convex part 46 provided on the cylinder 42 of the spacer 40.

Mounting portions 421 for mounting flanges 43k, which will be described later, are provided on both end side sides in the longitudinal direction L of the guide projection portion 46 of the cylinder 42k.

When using the cylinder 42k configured in this manner, the distal end side flexible tube 20k and the base end side flexible tube 30k having the flange portions 43k at the opposing ends are used.

Specifically, the flexible tubes 20k and 30k having main lumens 21 and 31 inside are cylindrical flexible tubes long in the longitudinal direction L, and their ends facing each other in the longitudinal direction L are provided with projecting edges 43k.

As shown in FIG. 20A, the flange portion 43k is a disc made of hard resin having a main through hole 431 provided in the main lumens 21 and 31 at its center, and corresponds to the wire lumens 22 and 32 and is composed of through holes. and a wire placement hole 44k.

The main through hole 431 is formed with a diameter that allows the attachment portion 421 of the cylinder 42k to be fitted therein.

Thus configured distal end side flexible tube 20k, base end side I flexible tube 30k and an assembly spacer 40k composed of a cylinder 42k are assembled to configure the movable elongated structure 10k.

Specifically, as shown in FIGS. 20A, 21A, and 22A, from the distal end side LF in the longitudinal direction L to the base end side LB, the distal end side flexible tube 20k, the assembly spacer 40k, and the base end side flexible tubes 30k are arranged in this order. At this time, the distal end side flexible tube 20k is arranged so that the flange 43k is on the base end side LB, and the base end side flexible tube 30k is arranged so that the flange 43k is on the distal end side LF.

Also, the assembly spacer 40k is arranged so that the guide convex part 46 is oriented in a desired direction in the circumferential direction.

Then, as shown in FIGS. 20B, 21B, and 22B, the mounting portion 421 of the cylinder 42k constituting the assembly spacer 40k is inserted into the main through hole 431 of the flange 43k and fitted to the distal end side flexible portion to assemble tube 20k, assembly spacer 40k and base end side flexible tube 30k.

As shown in FIG. 20C and FIG. 21C, the movable elongated structure 10k is configured by inserting the traction wire 50 through the wire lumens 22, 32 of the assembled distal end side flexible tube 20k and base end side flexible tube 30k.

At this time, the traction wire 50 is passed through the wire arrangement holes 44k provided in the flange portions 43k provided at the ends of the distal end side flexible tube 20k and the base end side flexible tube 30k.

The traction wire 50 may be routed in the assembly spacer 40k so as to extend in an oblique direction in the same manner as in the movable elongated structure 10 described above, or as shown in FIGS. 11 and 12, it may route straight along the longitudinal direction L.

The assembly spacer 40k and the movable elongated structure 10k using the assembly spacer 40k configured in this manner are provided with flanges provided at the ends of the distal end side flexible tube 20k and the base end side flexible tube 30k. Since it is composed of the cylinder 42k assembled with the portion 43k, the same effect as that of the movable elongated structural body 10 provided with the spacer 40 integrated above and the spacer 40 may be obtained.

In addition, compared to the movable elongated structure 10 in which the distal end side flexible tube 20, the spacer 40 and the base end side flexible tube 30 are arranged along the longitudinal direction L, and the traction wire 50 is routed and assembled, by fitting the mounting portion 421 of the cylinder 42k into the main through hole 431 of the flange portion 43k provided at the ends of the tubes 20k, 30k, the distal end side flexible tube 20k, the assembly spacer 40k, and the base end side flexible tube 30k may be assembled, whereby assimilability is improved as compared with the movable elongated structure 10 described above.

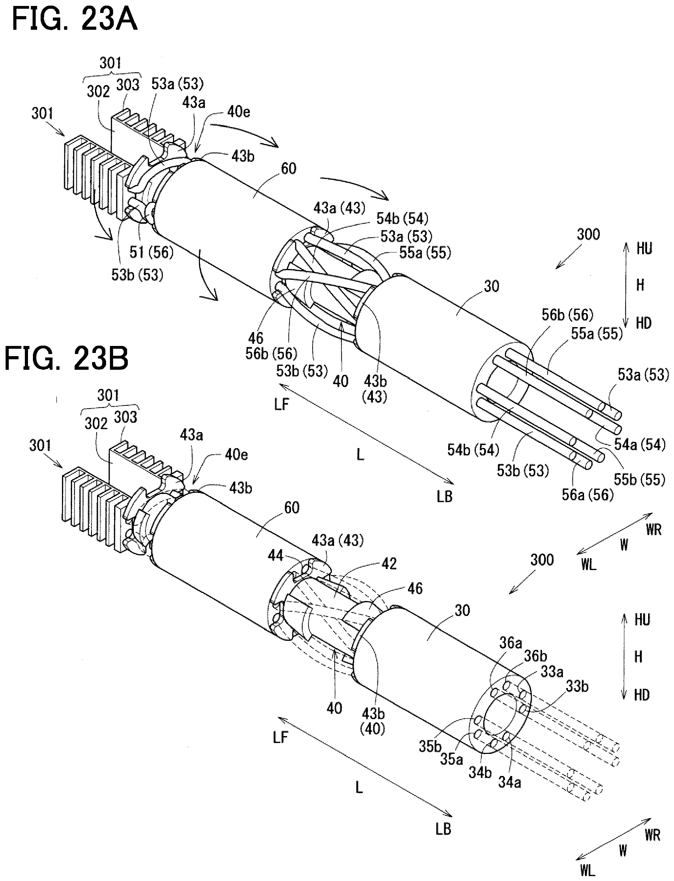

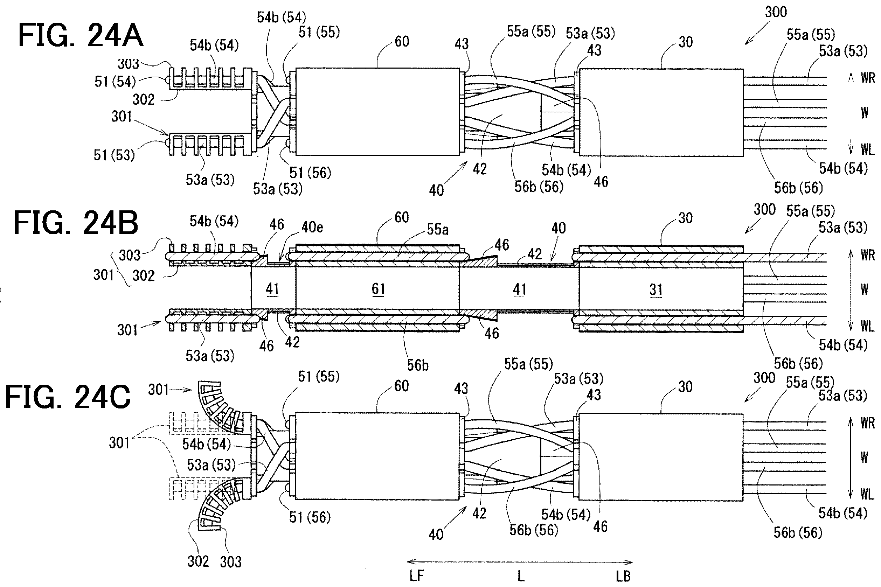

Also, as shown in FIGS. 23 and 24, the retractor 300 may be configured using the movable elongated structure 10e.

FIGS. 23A and 23B show schematic illustrations of the retractor 300. As shown in FIG. 23A shows a perspective view of retractor 300, and FIG. 23B shows a perspective view of retractor 300 with traction wire 50 shown in transparent.

FIGS. 24A to 24C show schematic illustrations of the retractor 300. As shown in FIG. 24A shows a plan view of retractor 300, FIG. 24B shows a cross-section through the upper wire lumen, and FIG. 24C shows a plan view of retractor 300 with elastic retractor 301 in the open state.

The retractor 300 utilizes the movable elongated structure 10e described above, and has an elastic retractor 301 on the distal end side LF of the spacer 40e instead of the distal end side flexible tube 20 in the movable elongated structure 10e. Specifically, the movable elongated structure 10e shown in FIGS. 14 and 15 is used with the width direction W being the height direction H.

The length of the spacer 40e in the retractor 300 in the longitudinal direction L of the cylinder 42 is shorter than that of the spacer 40e on the front end side LF in the movable elongated structure 10e, but other configurations are the same.

Also, since the wiring of the traction wire 50 is the same as that of the movable elongated structure 10e, the description thereof is omitted.

Two of the elastic retractors 301 are arranged facing each other in the width direction W and are fixed to the front end side LF of the spacer 40e.

The elastic retractor 301 is composed of an elastic body 302 extending toward the distal end side LF and a plurality of plate portions 303 projecting outward in the width direction W from the elastic body 302.

The elastic body 302 is a rectangular plate having a predetermined thickness that is longer in the longitudinal direction L than in the height direction H, and the plate portion 303 is a rectangular plate having a predetermined thickness that is longer in the height direction H than in the width direction W.

A plurality of plate portions 303 are arranged at predetermined intervals in the longitudinal direction L, integrally configured with the elastic body 302, and are formed to be longer in the height direction H than in the width direction W, longer in the longitudinal direction L than in the height direction H, and in a rectangular parallelepiped shape.

In a plurality of plate portions 303 arranged at predetermined intervals in the longitudinal direction L, an insertion hole 304 is formed through which the pulling wire 50 (54, 53) is inserted, corresponding to the distal wire lumen 22 (23, 24) of the distal flexible tube 20 in the movable elongated structure 10e.

The elastic retractor 301 configured in this way faces the front end side LF of the spacer 40e at a predetermined interval in the width direction W, that is, the projecting direction of the plate portion 303 with respect to the elastic body 302 is directed outward in the width direction W.

The traction portion 53 is inserted through the insertion hole 304 of the elastic retractor 301 on the left side WL, and the traction portion 54 is inserted through the insertion hole 304 of the elastic retractor 301 on the right side WR.

The retractor 300 configured in this manner pulls the traction portions 55 and 56 arranged in the height direction H on the base end side LB to the base end side LB, so that the intermediate tube 60 may be bent in the width direction W as indicated by the arrow in FIG. 23A.

Specifically, the intermediate tube 60 may be bent to the left side WL by pulling the traction portion 56 inserted through the base end side wire lumens 34b, 36a of the left side WL in the end side wire lumens 34, 36 to the base end side LB.

Conversely, the intermediate tube 60 may be bent to the right side WR by pulling to the base end side LB the traction portion 55 inserted through the base end side wire lumens 34a, 36b of the right side WR in the base end side wire lumens 34, 36.

Then, by canceling the traction of the traction portions 55 and 56, the elastic force of the intermediate tube 60 cancels the bending of the traction portions 55 and 56 due to the traction.

The elastic retractor 301 may be opened by pulling the traction portions 53 and 54 inserted through the base end side wire lumens 33 and 35 in the width direction W on the base end side LB.

Specifically, by pulling the traction portion 53 inserted through the insertion hole 304 of the elastic retractor 301 of the left WL, the elastic retractor 301 of the left WL may be bent outward in the width direction W as indicated by the arrow in FIG. 23A without bending the intermediate tube 60.

Conversely, by pulling the traction portion 54 inserted through the insertion hole 304 of the elastic retractor 301 of the right WR,
the elastic retractor 301 of the right WR may be bent outward in the width direction W as indicated by the arrow in FIG. 23A without bending the intermediate tube 60.

Therefore, when the traction portion 53 and the traction portion 54 are pulled at the same time, the elastic retractors 301 on both sides in the width direction W are bent outward in the width direction W as shown in FIG. 24C. which may be served as a retractor to open the site.

By canceling the traction of the traction portion 53 and/or the traction portion 54, the bending due to the traction of the traction portion 53 and/or the traction portion 54 is canceled by the elastic force of the elastic retractor 301 (302).

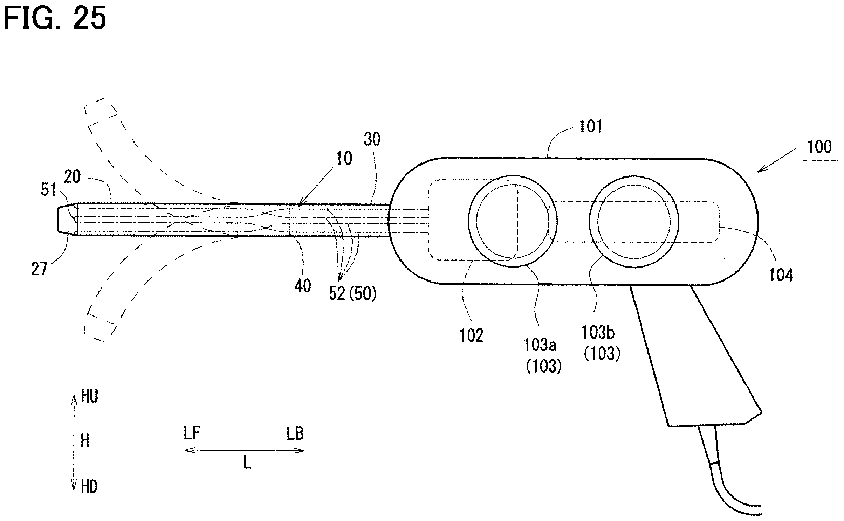

As another example of this embodiment shown in FIG. 25, a manipulator 100, which is a medical device using the movable elongated structure 10 described above, will be described below. Note that FIG. 25 shows a schematic diagram of a manipulator 100, which is a medical device in the other example.

The manipulator 100 is a medical device that is inserted into a duct such as a blood vessel that has a branched path, and performs a predetermined treatment after the tip reaches a predetermined location. A movable elongated structure 10 extending from the tip of the manipulator main body 101 to the distal end side LF is provided.

The manipulator main body 101 includes a traction drive unit 102 for pulling a traction portion 52 (not shown) of a traction wire 50 (not shown) extending from the base end side LB of the movable elongated structure 10, and a traction drive unit 102, the operation handle 103 (103a, 103b) for manually operating the bending direction of the movable elongated structure 10 by pulling the traction portion 52, and a control unit 104 for controlling how the eight traction portions 52 are pulled by the driving unit 102 with the manual operation of the operating handle 103.whether is towed.

The traction driving section 102 and the control unit 104 are arranged inside the manipulator main body 101, and the operating handle 103 is arranged outside the manipulator main body 101.

The operating handle 103 includes a vertical operating handle 103a for bending the movable elongated structure 10 in the height direction H, and a width direction operating handle 103b for bending it in the width direction.

The operator may bend the distal end side LF of the movable elongated structure 10 in the upward direction HU or the downward direction HD in the height direction H by manually operating the vertical direction operation handle 103a. It may be bent by a desired bending amount by the operation amount of 103a.

In addition, the operator may bend the movable elongated structure 10 to the right or left in the width direction by manually operating the width direction operation handle 103b, and the desired bending may be achieved by the operation amount of the width direction operation handle 103b. It may be bent in quantity.

Then, the operator manually operates both the vertical operation handle 103a and the width direction operation handle 103b at the same time or sequentially, whereby the distal end side LF of the movable elongated structure 10 may be bent in an oblique direction intersecting a vertical direction and a width direction and bent in a desired direction by the amount of operation of the vertical direction operating handle 103a and the width direction operating handle 103b.

In the description of the manipulator 100 described above, the movable elongated structure 10 is used, but any of the movable elongated structures 10a to 10e, 10j, and 10k described above may be used instead of the movable elongated structure 10. In this case, it is preferable to provide a traction driving unit 102 corresponding to the bending direction, that is, the number of pulling wires 50 (traction portions 52), and an operation handle 103 corresponding to the bending direction.

Further, if necessary, the operation handles 103a and 103b may be provided in separate units and connected to the manipulator main body 101 by wire or wirelessly.

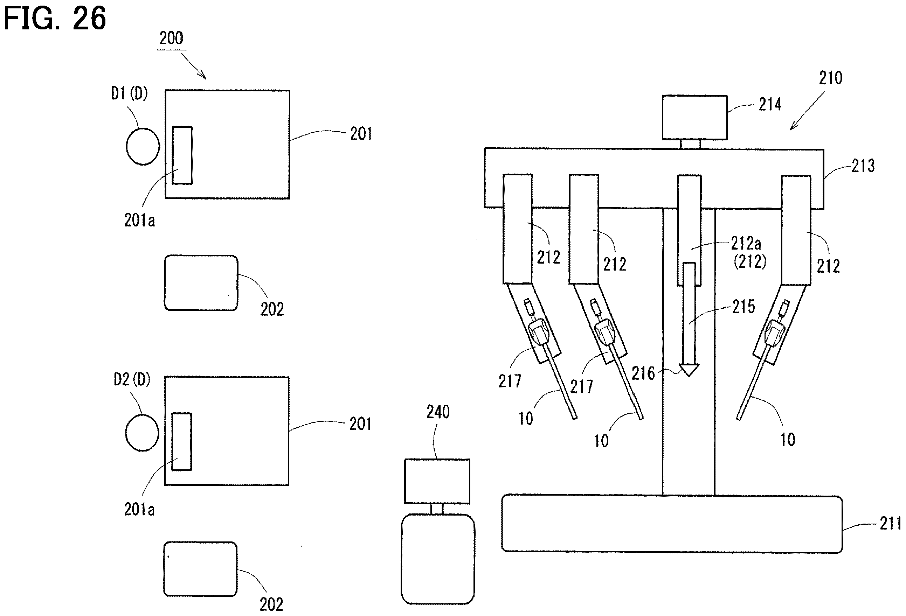

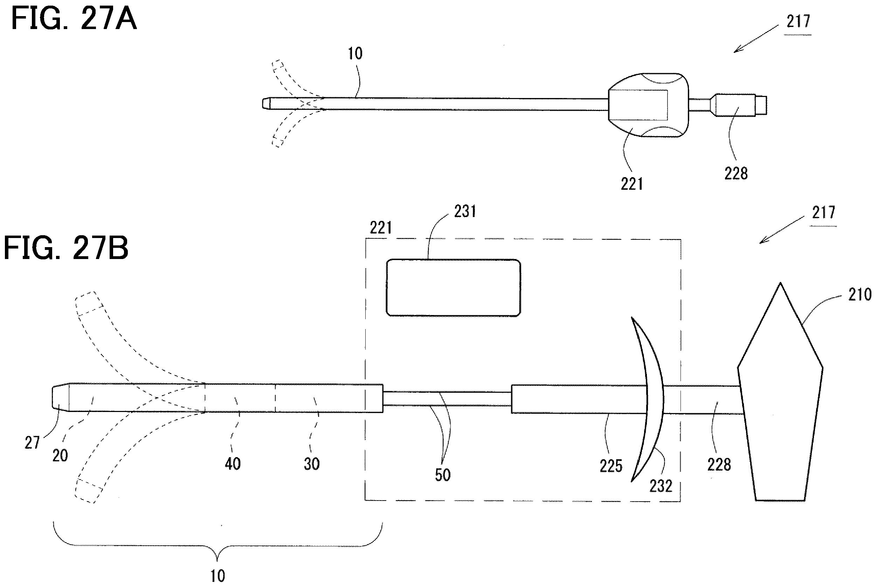

Next, as another example of this embodiment, a telesurgery system 200 will be described with reference to FIGS. 26 and 27.

FIG. 26 shows a schematic diagram of the telesurgery system 200 in the other example, and FIGS. 27A and 27B show a schematic explanatory diagram of the tool 217 in the telesurgery system 200. Specifically, FIG. 27A shows a plan view of a tool 217 that may be loaded onto the robotic arm assembly of tele surgical system 200, and FIG. 27B shows the internal configuration of tool 217.

The telesurgery system 200 includes a surgeon console 201 serving as a station for each of two operators D (D1, D2), a master control unit 202 operated by operator D, a viewing and core cart 240, and a patient side cart 210 having a robot.

The surgeon's console 201 includes a viewer 201a on which an image of the surgical site is displayed to the operator D. When using the surgeon's console 201, the operator D1 and/or D2 typically sits in the chair of the surgeon's console, has their eyes in front of the viewer 201a, and holds the master control unit 202 in one hand.

Although the telesurgery system 200 may be operated by two operators simultaneously, it may also be operated by one operator. When two operators perform operations simultaneously, cooperative operations by the two operators are possible, and there is an advantage that the operation time for the entire patient may be shortened. The surgeon's console 201 and the master control unit 202 may be configured in a system in which three or more units each are provided, if necessary.

The robot at the patient-side cart 210 is placed adjacent to the patient. In use, the patient side cart 210 is placed near the patient requiring surgery. The patient-side cart 210 robot has casters on the pedestal 211 so that it is fixed but movable during the surgical procedure. Surgeon console 201 is used in the same operating room as the patient-side cart, but may be located remotely from patient-side cart 210.

The patient-side cart 210 includes four robotic arm assemblies 212, although the number of robotic arm assemblies 212 is arbitrary. Each robot arm assembly 212 is connected to a driving device 213 that enables three-dimensional movement and is driven and controlled.

A display 214 displays image data associated with the surgery. A drive device 213 is controlled by master control unit 202 of surgeon console 201. Movement of tool 217 of robot arm assembly 212 is controlled by the operation of master control unit 202.

One robot arm assembly 212a of the four robot arm assemblies 212 is provided with an image capture device 215 such as an endoscope. A viewing camera 216 is included at the remote end of the image capture device 215. An elongated shaft-like image capture device 215 allows a viewing camera 216 to be inserted through a surgical entry port of a patient (not shown).

The image capture device 215 is operatively connected to viewer 201a of surgeon console 201 for displaying images captured by viewing camera 216 thereof. Each of the other robotic arm assemblies 212 is a linkage that respectively supports and includes a removable surgical instrument, tool 217.

The tool 217 includes a movable elongated structure 10 to allow insertion through a patient's surgical entry port. Movement of the movable elongated structure 10 is controlled by the master control unit 202 of surgeon console 201. The movable elongated structure 10 utilizes the movable elongated structure 10 of the previous embodiment.

FIGS. 27A and 27B show configurations of the tool 217 that may be loaded onto the robotic arm assembly 212 of the telesurgery system 200 of FIG. 26 as a representative example of a surgical device. The tools 217 attached to other robotic arm assemblies 212 may be of similar construction or may be surgical devices of other constructions.

The tool 217 shown in FIG. 27A has a movable elongated structure 10 having a traction wire (50), a surgical device 221 for driving control and monitoring of the tool 217, and a connector 228 for coupling to a robot. The surgical device 221 constitutes a traction driver that drives the traction wire 50 within the movable elongated structure 10.

As shown in FIG. 27B which illustrates the internal configuration of the tool 217, a surgical device 221 that drives the tool 217 directly connected to the robotic arm assembly 212 of FIG. 26, and a medical system consisting of 210 robots are shown.

The movable elongated structure 10 has a base end side tubular body 30 connected to a shaft 225, a wiring aid 40, and a distal end side tubular body 20 which is an end effector. Since the movable elongated structure 10 has a bent structure as in the above-described embodiment, the degree of freedom of the operation angle of the distal end side tubular body 20 is increased, and the accuracy of robot control is improved.

The surgical device 221 of the tool 217 has control circuitry 231 that controls signals within the surgical device and a signal interface 232 with the robot of the patient-side cart 210.

The control circuit 231 is configured to control a driving mechanism (not shown) that drives a predetermined traction wire of the movable elongated structure 10 based on a control signal from the robot 210.

As an operation is illustrated in FIGS. 26 to 28, the robot of the patient-side cart 210 is routed and/or wirelessly connected to the surgical device 221, the signal interface 232, and the connector 228, and the robot of the patient-side cart 210 Internally, there are an input unit that receives an operation signal from the master control unit 202, an arithmetic unit CPU that executes a predetermined operation program based on the operation signal, and a surgical device 221 based on the output from the arithmetic unit. and an output unit for generating a drive signal for driving the movable elongated structure 10 of the tool 217. The input unit and the output unit are composed of an input/output unit 210a (I/O).

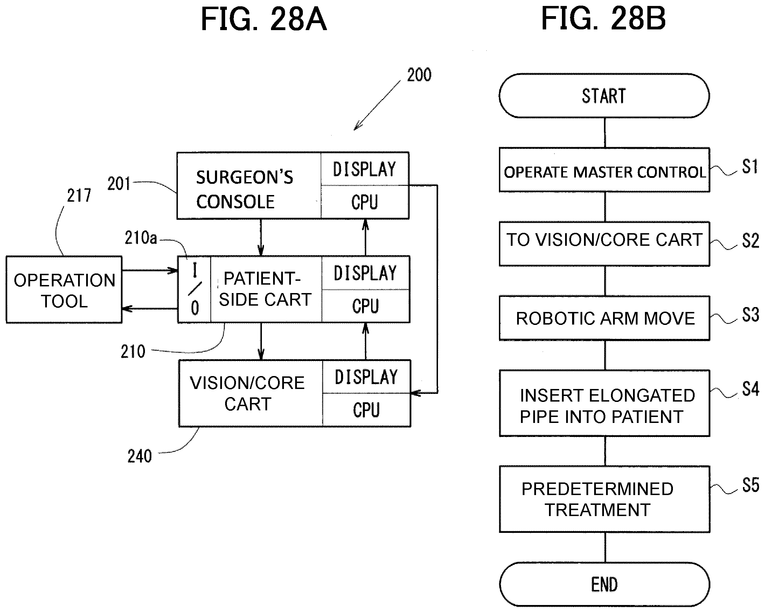

FIGS. 28A and 28B are explanatory diagrams of the telesurgery system 200, FIG. 28A is a block diagram showing the connection relationship with each unit, and FIG. 28B is an operation flow diagram of the telesurgery system 200.

A vision and core cart 240 has functions associated with image capture equipment. When the telesurgery system 200 is activated for surgery, the surgeon operates the master control unit 202 of the surgeon's console 201, and if there are two surgeons, also operates the master control unit 202 of the surgeon's console 201 (step S1), the command generated by the operation is sent to the vision/core cart 240 (step S2).

The vision and core cart 240 then interprets the signals and causes movement of the desired robotic arm assembly 212 to the patient's surgical area (step S3).

Next, the movable elongated structure 10 of the tool 217 attached to the selected robot arm assembly 212 is inserted into the patient through an elongated pipe (step S4), and after reaching a predetermined position by bending the distal end side tubular body 20, a predetermined treatment is performed (step S5), and the surgery on the living tissue is completed.

Although the movable elongated structure 10 is used in the description of the telesurgery system 200 and the tool 217 described above, instead of the movable elongated structure 10, the movable elongated structures 10a to 10e, 10j, 10k may be used. In this case, it is preferable to provide a traction drive unit 102 corresponding to the bending direction, that is, the number of traction wires 50 (traction portions 52), and an operation handle 103 corresponding to the bending direction.

Since the telesurgery system 200 including the manipulator 100, the retractor 300, the tool 217, and the tool 217 described above includes the movable elongated structure 10, in addition to the effects of the movable elongated structure 10 described above, The effect obtained by each structure may be produced.

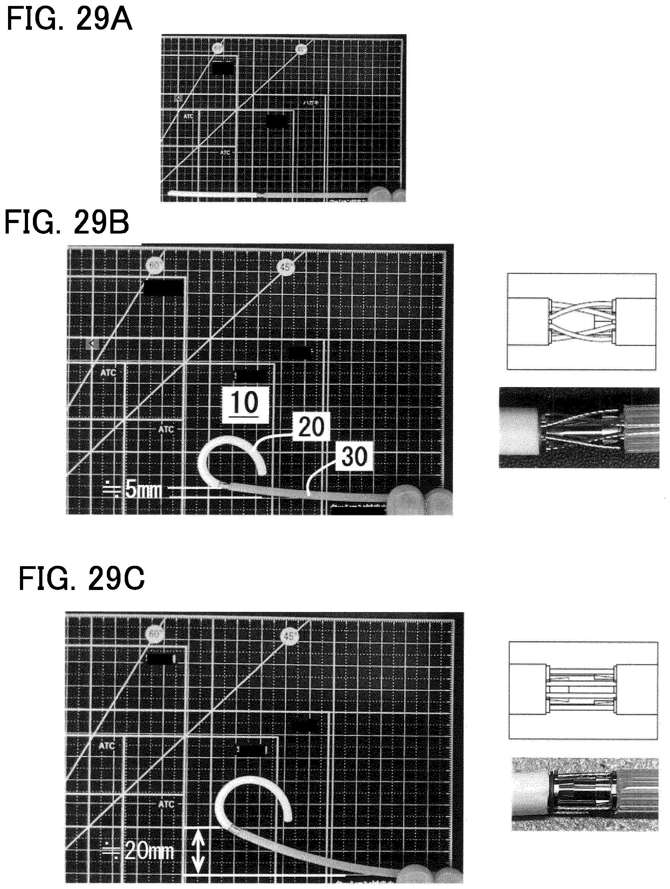

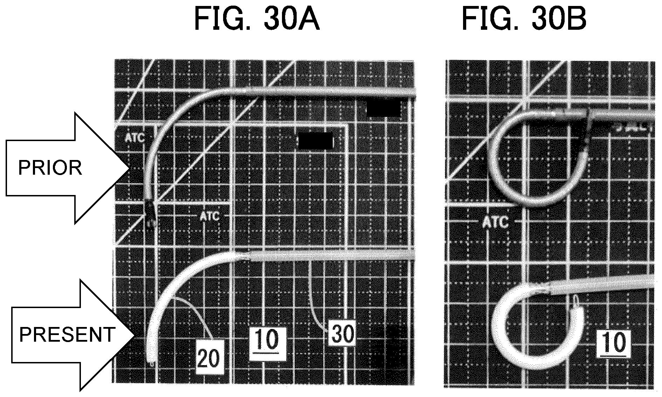

Demonstration experiments conducted on the movable elongated structure 10 (10a, 10b, 10e, 10j, 10k) configured as described above and used for the manipulator 100, the retractor 300, etc. will be described below with reference to FIGS. 29 and 30.

First, as shown in FIGS. 29A to 29C, the bending performance of a four-direction (omnidirectional) movable elongated structure 10 was confirmed as compared with a conventional two-direction movable elongated structure. 29A shows the initial state, FIG. 29B shows the state in which the movable elongated structure 10 is bent, and FIG. 29C shows the movable elongated structure simulating a conventional two-way movable elongated structure bent. 29B and 29C show the routed state of the traction portion 52 of the spacer 40 portion of the movable elongated structure 10.

In this demonstration experiment, in order to imitate a conventional two-way movable elongated structure, a two-way movable type is configured by linearly arranging the traction part 52 in the spacer 40 of the movable elongated structure 10, then, a bending shape with that of the movable elongated structure 10 is compared.

The movable elongated structure 10 shown in FIG. 29B is designed such that the outer diameter of the distal end side flexible tube 20 and the base end side flexible tube 30 is 3.2 mm, the distal end side spacing X1 is 1.157 mm, and the base end side spacing X2 is 0.32 mm. When the pair of traction wires 50 are pulled, the movable elongated structure 10 is moved to the base end side flexible tube 30 in the movable elongated structure modeled after the conventional two-way movable elongated structure (see FIG. 29C) is displaced by about 20 mm, but only about 5 mm is displaced in FIG. 29B. It has been confirmed that the deformation of the flexible tube 30 may reduce the interval of deviation from the horizontal direction by 75%.

As a result, the pulling of the traction wire 50 has little effect on the deformation of the base end side flexible tube 30 in the movable elongated structure 10, and even if the base end side flexible tube 30 is deformed due to a complicated path or external force, the deterioration of the movable bending performance of the distal end side flexible tube 20 is reduced. From this result, it is confirmed that the deterioration of the bending performance of the distal end side flexible tube 20 may be reduced by arranging the traction wire 50.

Next, as shown in FIGS. 30A and 30B, the bending radius of the movable elongated structure 10 was compared with the conventional two-way movable elongated structure.

In addition, FIG. 30A shows a diagram when bent by 90 degrees, and FIG. 30B shows a diagram when bent by 275 degrees. The upper part "PRIOR" of FIG. 30A shows a conventional two-way movable elongated structure, and the lower part "PRESENT" shows the movable elongated structure 10 of the present invention.

Although the movable elongated structure 10 shown in the lower part of FIGS. 30A and 30B may be bent in four directions (all directions), compared to the conventional two-way movable elongated structure shown in the upper part of FIGS. 30A and 30B. It has been confirmed that the bending radius is reduced by about 13% in a bending state of about 10%.

In addition, in the conventional two-way movable elongated structure, one traction wire 50 is used for bending in one direction, whereas the loop-shaped traction wire 50 is used for bending in one direction. is equivalent (synonymous) to pulling two traction wires 50, the pulling force acting on the traction wires 50 is about half that of the conventional two-way movable elongated structure. Therefore, it is possible to use a thin traction wire 50 that is easily broken compared to a conventional two-way movable elongated structure. In addition, since the pulling force acting on the traction wire 50 is approximately halved, when the traction wire 50 is bent and deformed, the inner surface of the distal wire lumen 22 of the distal end side flexible tube 20 and the base end side flexible tube 30 may be moved. Since the force of contact with the inner surface of the base end side wire lumen 32 is also approximately halved, it was confirmed that a softer tube than the distal end side flexible tube 20 or the base end side flexible tube 30 may be used.

As described above, in the correspondence between the configuration of the present invention and the above-described embodiments, the longitudinal direction of the present invention corresponds to the longitudinal direction L, and so on.
the distal end side corresponds to the distal end side LF,
the base end side corresponds to the base end side LB,
the distal end side tubular body corresponds to the distal end side flexible tubes 20, 20j, 20k,
the base end side tubular body correspond to the base end side flexible tubes 30, 30j, 30k;
the cylindrical bodies correspond to the cylinders 42, 42k,
wiring aids correspond to spacers 40, 40a, 40e, 40g, 40i, 40j and assembly spacer 40k,
base end side flanges correspond to the base end side flanges 43b and 43jb,
the distal end side flanges correspond to the distal end side flanges 43a and 43ja,
the distal end side through hole corresponds to the distal end side wire lumen 22,
the base end side through hole corresponds to the base end side wire lumen 32,
the traction operation body corresponds to the traction wire 50,
the distal end side virtual line corresponds to the distal end side virtual line FVL,
the distal end side center line corresponds to the distal end side center line FCL,
the distal end side interval corresponds to the distal end side interval X1,
the base end virtual line corresponds to the base end virtual line BVL,
the base end center line corresponds to the base end center line BCL,
the base end spacing corresponds to the base end spacing X2,
the proximity regulation portion corresponds to the proximity
the traction operation body arrangement parts correspond to the placement recesses 44, 44a, 44e, 44i, 44j, 44ja, and 44jb,
the separation regulation portion corresponds to the separation regulation portion 45a,
the through hole sets correspond to through hole sets 11, 11a, 11b, 11c, 11d, 11e, 11f, 11g, and 11h,
the guiding portion corresponds to the guiding convex portions 46, 46g,
the intermediate tubular body corresponds to the intermediate tube 60,
   the intermediate through holes correspond to the intermediate wire lumens 63, 64, 65, 66;
the main lumen corresponds to the main lumens 21 and 31,
the movable elongated structures correspond to movable elongated structures 10, 10a, 10b, 10e, 10j, and 10k,
the traction drive unit corresponds to the traction drive unit 102 and the drive device 213,
the movable elongated structural instrument corresponds to the manipulator 100 and the retractor 300,
the control unit corresponds to the control unit 104, the master control unit 202,
the medical system corresponds to the telesurgery system 200,
the robot arm corresponds to the robot arm assembly 212,
the connecting part corresponds to the connector 228,
the tool corresponds to tool 217,
the manipulator corresponds to manipulator 100,
the input/output unit corresponds to the input/output unit 210a,
the arithmetic unit corresponds to the arithmetic unit CPU, and
the robot and the medical robot correspond to the patient-side cart 210 which are not limited to the above embodiments.

Although the traction wire 50 is served as the traction operation body, it may be belt-shaped. Moreover, although the single traction wire 50 is bent at the bent portion 51 to form a pair of traction portions 53, a pair of separate traction wires 50 may be used. Further, when pulling a pair of traction wires 50 configured separately, one traction wire 50 may be pulled, or both traction wires 50 may be pulled. Also, although both traction wires 50 are pulled, each traction wire 50 may be pulled with a different pulling force.

The flexible tubes 20 and 30 are circular tubes having a ring-shaped cross section and have the main lumens 21 and 31 inside, but they may be tubular bodies having an elliptical ring-shaped cross section or a polygonal ring-shaped cross section. 31 may have a similar shape to the outer diameter of the cross-sectional shape as described above, or may have a different cross-sectional shape. Also, the main lumens 21 and 31 in the flexible tubes 20 and 30 may be arranged at positions shifted from the centers of the flexible tubes 20 and 30.

Also, the flexible tubes 20 and 30 may be configured with a spine structure in which rigid bodies having main lumens therein are connected via rotary joints or elastic bodies.

Also, tele surgical system 200 may be configured using retractor 300 instead of the moveable elongated structure 10 in the tool 217.

### [Description of symbols]

10, 10a, 10b, 10e, 10j, 10k ... movable elongated structure
11, 11a, 11b, 11c, 11d, 11e, 11f, 11g, 11h ... through hole set
20, 20j, 20k ... distal end side flexible tube
21 ... distal end side main lumen
22 ... distal end side wire lumen
30, 30j, 30k ... base end side flexible tube
31 ... base end side main lumen
32 ... base end side wire lumen
40, 40a, 40e, 40g, 40i, 40j ... spacer
40k...assembly spacer
42, 42k ... cylinder
43, 43i, 43j, 43k ... flange
43a, 43ja ... distal end side flange
43b, 43jb... base end side flange
44, 44a, 44e, 44i, 44j, 44ja, 44jb ... placement recess
45a... separation regulation part
45b... proximity regulation part
46, 46g ... guidance convex part
50 ... traction wire
60... intermediate tube
62 ... middle wire lumen
100... manipulator
102 ... traction drive unit
200... telesurgery system
202 ... master control unit
210 ... patient side cart
210a ... input/output unit
212 ... robot arm assembly
213 ... drive device
228 ... connector
217 ... tool
300 ... retractor
302... elastic body
CPU... arithmetic unit
X1... distal end side interval
X2... base end side interval
BCL: base end side center line
BVL: base end side virtual line
FCL...central line on distal end side
FVL: virtual line on distal end side
L...longitudinal direction
LF... distal end side
LB...base end side

## Claims

1. A movable elongated structure comprising a distal end side tubular body, a base end side tubular body, and a traction operation body, further comprising a wiring aid removably held between the distal end side tubular body and the base end side tubular body by the traction operation body.

2. The movable elongated structure according to claim 1,
wherein the distal end side tubular body and the base end side tubular body are formed in an elongated shape having flexibility;
wherein a pair of flexible traction operation bodies that are inserted through the pair of longitudinal through-holes provided inside the tube walls of the distal end side tubular body and the base end side tubular body are provided;
wherein the wiring aid that is disposed between the distal end side tubular body and the base end side tubular body and regulates the direction of the traction operation body is provided;
wherein the through hole provided in the base end side tubular body is defined as a base end side through hole, and the through-hole provided in the distal end side tubular body is defined as a distal end side through hole;
wherein the interval between the pair of base end side through holes in the circumferential direction is set wider than the interval between the pair of distal end side through holes in the circumferential direction;
wherein the wiring aid includes at least a tubular body arranged along the longitudinal direction, and a base end side flange at an end of the base end side of the tubular body and protruding radially outward, and a distal end side flange provided at an end of the distal end side of the cylindrical body and protruding radially outward;
wherein on the base end side flange, there is provided an operation body arrangement part for arranging the operation body that is led out from the base end side through hole and introduced into the distal end side through hole, and
wherein on the distal end side flange, there is provided an operation body arrangement part for arranging the operation body that is led out from the base end side through hole and introduced into the distal end side through hole.

3. The movable elongated structure according to claim 2,
wherein a distal end side interval which is an interval between a distal end side virtual line connecting the centers of the pair of distal end side through holes and a distal end side central line passing through the center of the distal end side tubular body and parallel to the distal end side virtual line is set wider than a base end side interval which is an interval between a base end side virtual line connecting the centers of the pair of base end side through holes and a base end side central line passing through a center of the base end side tubular body and parallel to the base end side virtual line.

4. The movable elongated structure according to claim 2 or 3,
wherein the base end side flange is provided at a base end side end of the cylindrical body,
wherein the distal end side flange is provided at a distal end side end of the cylindrical body,
wherein the base end side flange is provided with a proximity regulation part that regulates proximity in a circumferential direction of the traction operation body led out from the base end side through hole and introduced into the distal end side through hole,
wherein the distal end side flange is provided with a separation regulation part that regulates separation in a circumferential direction of the traction operation body led out from the base end side through hole and introduced into the distal end side through hole, and
wherein the operation body arrangement part is open on a radial outer side.

5. The movable elongated structure according to claim 4, wherein the base end side flange and the distal end side flange are configured integrally with the cylindrical body.

6. The movable elongated structure according to claim 4, wherein at least one of the base end side flange and the distal end side flange is configured separately from the cylindrical body and configured to be assembled.

7. The movable elongated structure according to one of claims 4 to 6,
wherein the pair of base end side through holes and the pair of distal end side through holes through which the pair of traction operation bodies are inserted form a pair of through holes,
wherein a plurality of sets of the through hole sets are arranged at different positions in a circumferential direction,
wherein a plurality of the proximity regulation parts and the traction operation body arrangement parts are provided on the base end side flange, and
wherein a plurality of the separation regulation parts and the traction operation body arrangement parts are provided on the distal end side flange.

8. The movable elongated structure according to claim 7,
wherein in the base end side flange, common the proximity regulation part regulates circumferentially adjacent traction operation bodies of a pair of the traction operation bodies inserted through the circumferentially adjacent through hole sets to come close to each other,
wherein in the distal end side flange, the separation of the traction operation bodies that are adjacent in the circumferential direction of the pair of traction operation bodies that are inserted through the through hole sets that are adjacent in the circumferential direction is regulated by the traction operation body inserted through a pair of through holes adjacent to each other on the opposite side in the circumferential direction and the common separation regulation part.

9. The movable elongated structure according to claim 7 or 8,
wherein one of the traction operation bodies and the other of the traction operation bodies inserted through the through hole sets that are adjacent in the circumferential direction intersect in the circumferential direction, and
wherein the cylindrical body is provided with a guide portion for guiding one of the traction operation bodies to the outside of the other traction operation body.

10. The movable elongated structure according to any one of claims 7 to 9,
wherein an elongated intermediate tubular body having an intermediate through hole is arranged between the distal end side tubular body and the end side tubular body,
wherein a plurality of pairs of the traction operation bodies are provided,
wherein at least one pair of the traction operation bodies out of the plurality of pairs of the traction operation bodies is inserted through the distal end side through hole, the intermediate through hole, and the base end side through hole, and
wherein at least one pair of the traction operation bodies out of the plurality of pairs of the traction operation bodies is inserted through the intermediate through hole and the base end through hole.

11. The movable elongated structure according to any one of claims 2 to 10,
wherein the traction operation body is a flexible wire,
wherein the distal end side tubular body and the end side tubular body are composed of flexible tubes having a main lumen, and
wherein the through hole is a wire lumen formed inside the tube wall of the tube and through which the wire is available to be inserted.

12. The movable elongated structure of claim 11, wherein the wire is bent back at the distal end side of the distal end side tubular body to form a pair of traction operation bodies.

13. A movable elongated structural instrument comprising the movable elongated structure according to any one of claims 1 to 12, and a traction drive for pulling the pair of traction operation bodies, wherein the traction drive pulls the pair of traction operation bodies to bend and deform the distal end side tubular body.

14. A movable elongated structural instrument comprising the movable elongated structure according to any one of claims 7 to 10, and a traction drive for pulling the pair of traction operation bodies, wherein a plurality of traction drives are provided, and the traction drives are constructed to pull the pair of traction operation bodies to bend and/or stretch (extend) the distal end side tubular body in a desired direction.

15. A medical system comprising the movable elongated structural instrument according to claim 13 or 14, a drive unit for driving the traction drive, and a control unit connected to apply a drive signal to the drive unit.

16. A medical system comprising the movable elongated structural instrument according to claim 14, a drive unit selectively driving at least one of the plurality of traction drives, and a control unit connected to apply a drive signal to the drive unit.

17. The medical system according to claim 16, wherein an operation unit selectively operating at least one of the plurality of traction drive units is provided, and is connected to the control unit.

18. A tool comprising:
the movable elongated structural instrument according to claim13 or 14;
a mounting portion mounting the end side tubular body of the movable elongated structure to a distal end of a robot arm; and
a connecting portion connected to a driving mechanism for driving the traction driving portion on the robot arm side.

19. A robot comprising:
the tool according to claim 18;
a robot arm having the tool at its distal end;
a drive unit that drives the traction drive and the robot arm; and
a control unit connected to apply a drive signal to the drive unit.

20. A manipulator comprising:
the movable elongated structural instrument according to claim 13 or 14;
a body portion provided at a base end of the base end side tubular body in the movable elongated structure; and
an operation unit operating the traction drive part in the main body part.

21. A robot comprising:
an input/output unit routed and/or wirelessly connected to the movable elongated structural instrument according to claim 13 or 14;
an input unit that receives operation signals in real time;
an arithmetic unit that executes a predetermined operation program based on the operation signal; and
an output unit that generates a driving signal for pulling a predetermined traction operation body by the traction drive unit and bending and/or expanding and contracting (extending) at least the distal end side tubular body in a desired direction based on an output from the arithmetic unit.

22. A medical robot comprising the robot according to claim 21, wherein the output unit provides drive signals to an externally mounted drive unit that mechanically drives the movable elongated structure.

23. An insertion method comprising:
inserting the movable elongated structure according to any one of claims 1 to 12 into a duct,
driving and controlling the traction drive unit that pulls the pair of traction operating bodies to bend and deform the distal end side tubular body, and
inserting the distal end side tubular body into a branched duct.

24. An insertion method comprising:
inserting the movable elongated structure including a distal end side tubular body, a base end side tubular body and a traction operation body, which includes the wiring aid removably held by the traction operation body between the distal end side tubular body and the base end side tubular body, into a duct,
driving and controlling the traction drive unit to bend and deform the distal end side tubular body, and
inserting the distal end side tubular body into a branched duct.

25. The insertion method according to claim 23 or 24, wherein the duct is at least one of a hollow organ, a vessel and a blood vessel

26. An operation method of the robot equipped with the movable long structural instrument according to claim 13 or 14, comprising:
receiving an operation signal in real time by the input/output unit routed and/or wirelessly connected to the robot;
executing a predetermined operation program based on the received operation signal, by the arithmetic unit; and then,
pulling the traction operation body by the traction drive unit based on the output from the arithmetic unit to bend-deform and/or expand-contract (extension) deform the distal end side tubular body in a desired direction.

27. A method of operating a movable elongated structure of removing a wiring aid from a movable elongated structure provided with a distal end side tubular body, a base end side tubular body and a traction operation body which includes the wiring aid removably held by the traction operation body between the distal end side tubular body and the base end side tubular body.

28. The method of operating a movable elongated structure according to claim 27 comprising;
pulling the pair of traction operation bodies to bend and deform the distal end side tubular body.

29. The method of operating the movable elongated structure,
wherein a plurality of pairs of the traction operation bodies are provided, and
wherein a predetermined pair of the pulling operation bodies out of the plurality of pairs of the pulling operation bodies are pulled to bend and deform the distal end side tubular body in a desired direction.

30. A wiring aid which is removably held between a distal end side tubular body and a base end side tubular body of a movable elongated structure having the distal end side tubular body, the base end side tubular body, and a traction operation body.

31. The wiring aid according to claim 30,
wherein there is at least provided a cylindrical body arranged along a longitudinal direction between the flexible elongated distal end side tubular body and base end side tubular body arranged in series from the distal end side to the base end side along the longitudinal direction,
wherein there is provided a base end side flange at a base end of the cylindrical body which protrudes radially outward, in which an interval in a circumferential direction is set wider than the interval in the circumferential direction between the pair of distal end side through holes provided inside the tube wall of the distal end-side tubular body and penetrating in the longitudinal direction, and is provided with an operation body arrangement part arranged by a pair of traction operation bodies led out from a pair of base end side through holes provided inside a tube wall of the base end side tubular body and extending through the longitudinal direction and introduced into the distal end side through holes, and
wherein there is provided a distal end side flange at a distal end of the cylindrical body which protrudes radially outward, and is provided with an operation body arrangement part arranged by the traction operation body that protrudes radially outward from the distal end of the cylindrical body, is led out from the base end side through hole and introduced into the distal side through hole.
